# EUROPEAN PATENT SPECIFICATION

(11) **EP 2 272 868 B1**
(45) Date of publication and mention of the grant of the patent: **04.03.2015**
(21) Application number: 10009416.8
(22) Date of filing: 04.06.2004
(51) Int. Cl.: C07K 16/00, A61K 39/395

(54) **Combination therapy for B cell disorders**
Kombinationstherapie für B-Zell-Erkrankungen
Thérapie de combinaison pour des désordres de cellules B

(30) Priority: 05.06.2003 US 476481 P; 05.06.2003 US 476414 P; 06.06.2003 US 476531 P
(43) Date of publication of application: 12.01.2011
(62) Divisional of application: 04754321.0
(73) Proprietor: Genentech, Inc., South San Francisco CA 94080-4990 (US)
(72) Inventor: Chan, Andrew, Menlo Park, California 94025 (US); Gong, Qian, Foster City, California 94404 (US); Martin, Flavius, Hayward, California 94545 (US)
(74) Representative: Walton, Seán Malcolm

(56) References cited:
- WO-A-00/43032
- WO-A-01/77342
- US-A1- 2003 095 967
- REFF M E ET AL: "Depletion of B cells in vivo by a chimeric mouse human monoclonal antibody to CD20", BLOOD, AMERICAN SOCIETY OF HEMATOLOGY, US, vol. 83, no. 2, 15 January 1994 (1994-01-15), pages 435-445, XP002112549, ISSN: 0006-4971
- CRAXTON A ET AL: "A ROLE FOR ZTNF4/APRIL IN THE CO-STIMULATION OF B CELL PROLIFERATION BY MACROPHAGES", FASEB JOURNAL, FED. OF AMERICAN SOC. FOR EXPERIMENTAL BIOLOGY, US, vol. 16, no. 4, 20 March 2002 (2002-03-20) , XP009064584, ISSN: 0892-6638
- CRAXTON ANDREW ET AL: "Macrophage- and dendritic cell-dependent regulation of human B-cell proliferation requires the TNF family ligand BAFF", 1 June 2003 (2003-06-01), BLOOD, AMERICAN SOCIETY OF HEMATOLOGY, US, PAGE(S) 4464 - 4471, XP002638817, ISSN: 0006-4971 * the whole document *
- BAKER KEVIN P ET AL: "Generation and characterization of LymphoStat-B, a human monoclonal antibody that antagonizes the bioactivities of B lymphocyte stimulator", 20031101; 20031100, vol. 48, no. 11, 1 November 2003 (2003-11-01), pages 3253-3265, XP002422474,

## Description

### FIELD OF THE INVENTION

The invention relates to novel combination therapies for the treatment of B cell malignancies as well as autoimmune disorders.

### BACKGROUND OF THE INVENTION

Lymphocytes are one of several populations of white blood cells; they specifically recognize and respond to foreign antigen. The three major classes of lymphocytes are B lymphocytes (B cells), T lymphocytes (T cells) and natural killer (NK) cells. B lymphocytes are the cells responsible for antibody production and provide humoral immunity. B cells mature within the bone marrow and leave the marrow expressing an antigen-binding antibody on their cell surface. When a naive B cell first encounters the antigen for which its membrane-bound antibody is specific, the cell begins to divide rapidly and its progeny differentiate into memory B cells and effector cells called "plasma cells". Memory B cells have a longer life span and continue to express membrane-bound antibody with the same specificity as the original parent cell. Plasma cells do not produce membrane-bound antibody but instead produce secreted form of the antibody. Secreted antibodies are the major effector molecules of humoral immunity.

The CD20 antigen (also called human B-lymphocyte-restricted differentiation antigen, Bp35) is a hydrophobic transmembrane protein with a molecular weight of approximately 35 kD located on pre-B and mature B lymphocytes (Valentine et al. J. Biol. Chem. 264(19):11282-11287 (1989); and Einfeld et al. EMBO J. 7(3):711-717 (1988)). The antigen is also expressed on greater than 90% of B cell non-Hodgkin's lymphomas (NHL) (Anderson et al. Blood 63(6):1424-1433 (1984)), but is not found on hematopoietic stem cells, pro-B cells, normal plasma cells or other normal tissues (Tedder et al. J. Immunol. 135(2):973-979 (1985)). CD20 is thought to regulate an early step(s) in the activation process for cell cycle initiation and differentiation (Tedder *et al., supra*) and possibly functions as a calcium ion channel (Tedder et al. J. Cell. Biochem. 14D:195 (1990)).

Given the expression of CD20 in B cell lymphomas, this antigen has been a useful therapeutic target to treat such lymphomas. There are more than 300,000 people in the United States with B-cell NHL and more than 56,000 new cases are diagnosed each year. For example, the rituximab (RITUXAN®) antibody which is a genetically engineered chimeric murine/human monoclonal antibody directed against human CD20 antigen (commercially available from Genentech, Inc., South San Francisco, California, U.S.) is used for the treatment of patients with relapsed or refractory low-grade or follicular, CD20 positive, B cell non-Hodgkin's lymphoma. Rituximab is the antibody referred to as "C2B8" in US Patent No. 5,736,137 issued April 7, 1998 (Anderson et al.). *In vitro* mechanism of action studies have demonstrated that RITUXAN® binds human complement and lyses lymphoid B cell lines through complement-dependent cytotoxicity (CDC) (Reff et al. Blood 83(2):435-445 (1994)). Additionally, it has significant activity in assays for antibody-dependent cellular cytotoxicity (ADCC). *In vivo* preclinical studies have shown that RITUXAN® depletes B cells from the peripheral blood, lymph nodes, and bone marrow of cynomolgus monkeys, presumably through complement and cell-mediated processes (Reff et al. Blood 83(2):435-445 (1994)). Other anti-CD20 antibodies indicated for the treatment of NHL include the murine antibody Zevalin^{™} which is linked to the radioisotope, Yttrium-90 (IDEC Pharmaceuticals, San Diego, CA), Bexxar^{™} which is a another fully murine antibody conjugated to I-131 (Corixa, WA).

BLyS (also known as BAFF, TALL-1, THANK, TNFSF13B, or zTNF4) is a member of the TNF1 ligand superfamily that is essential for B cell survival and maturation. BLyS overexpression in transgenic mice leads to B cell hyperplasia and development of severe autoimmune disease (Mackay, et al. (1999) J. Exp. Med. 190, 1697-1710; Gross, et al. (2000) Nature 404, 995-999; Khare, et al. (2000) Proc. Natl. Acad. Sci. U.S.A. 97, 3370-33752-4). BLyS levels are elevated in human patients with a variety of autoimmune disorders, such as systemic lupus erythematosus, rheumatoid arthritis, and Sjögren's syndrome (Cheema, G. S, et al., (2001) Arthritis Rheum. 44, 1313-1319; Groom, J., et al, (2002) J. Clin. Invest. 109, 59-68; Zhang, J., et al., (2001) J. Immunol. 166, 6-10). Furthermore, BLyS levels correlate with disease severity, suggesting that BLyS can play a direct role in the pathogenesis of these illnesses. BLyS acts on B cells by binding to three members of the TNF receptor superfamily, TACI, BCMA, and BR3 (also known as BAFF-R) (Gross, et al., *supra*; 8. Thompson, J. S., et al., (2001) Science 293, 2108-2111; Yan, M., et al., (2001) Curr. Biol. 11, 1547-1552; Yan, M., et al., (2000) Nat. Immunol. 1, 37-41; Schiemann, B., et al., (2001) Science 293, 2111-2114). Of the three, only BR3 is specific for BLyS; the other two also bind the related TNF family member, APRIL. Comparison of the phenotypes of BLyS and receptor knockout or mutant mice indicates that signaling through BR3 mediates the B cell survival functions of BLyS (Thompson, et al., *supra;* Yan, (2002), *supra*; Schiemann, *supra).* In contrast, TACI appears to act as an inhibitory receptor (Yan, M., (2001) Nat. Immunol. 2, 638-643), while the role of BCMA is unclear (Schiemann, *supra).*

BR3 is a 184-residue type III transmembrane protein expressed on the surface of B cells (Thompson, et al., *supra*; Yan, (2002), *supra).* The intracellular region bears no sequence similarity to known structural domains or protein-protein interaction motifs. Nevertheless, BLyS-induced signaling through BR3 results in processing of the transcription factor NF-B2/p100 to p52 (Claudio, E, et al., (2002) Nat. Immunol. 3, 958-965; Kayagaki, N., et al., (2002) Immunity 10, 515-524). The extracellular domain (ECD) of BR3 is also divergent. TNFR family members are usually characterized by the presence of multiple cysteine-rich domains (CRDs) in their extracellular region; each CRD is typically composed of ~40 residues stabilized by six cysteines in three disulfide bonds. Conventional members of this family make contacts with ligand through two CRDs interacting with two distinct patches on the ligand surface (reviewed in Bodmer, J.-L., et al., (2002) Trends Biochem. Sci. 27, 19-26). However, the BR3 ECD contains only four cysteine residues, capable of forming a partial CRD at most, raising the question of how such a small receptor imparts high-affinity ligand binding.

Previously it has been shown that the BLyS-binding domain of BR3 resides within a 26-residue core region (Kayagaki, et al., *supra).* Six BR3 residues, when structured within a β-hairpin peptide (bhpBR3), were sufficient to confer BLyS binding and block BR3-mediated signaling. Others have reported polypeptides that have been purported to interact with BLyS (e.g., WO 02/24909, WO 03/035846, WO 02/16312, WO02/02641).

### SUMMARY OF THE INVENTION

Disclosed herein is a method of depleting B cells from a mixed population of cells comprising contacting the mixed population of cells with a BLyS antagonist and a CD20 binding antibody. This method is useful e.g., in a commercial in vitro assay to effectively and selectively deplete B cells from a mixed population of cells, by contacting the B cells with a BLyS antagonist and an anti-CD20 antibody. Another aspect of the preceding method of B cell depletion is to specifically deplete certain subsets of B cells such a germinal center B cells and marginal zone B cells. The germinal center B cells may be in the spleen and Peyer's patches. Disclosed herein is a method of depleting all B cell subsets in vitro or in vivo by contacting the B cells with a BLyS antagonist and a CD20 binding antibody.

Disclosed herein is a method of depleting all populations of B cells in the spleen by administering to a mammal, a BLyS antagonist and an anti-CD20 antibody in amounts effective to deplete all populations of B cells. The method may be effective to deplete marginal zone and germinal center B cells in the spleen, lymph node and Peyer's patches.

Disclosed herein is a method of treating a B cell neoplasm or malignancy characterized by B cells expressing CD20, comprising administering to a patient suffering from the neoplasm or malignancy, a therapeutically effective amount of a CD20 binding antibody and of a BLyS antagonist. The CD20 binding antibody and BLyS antagonist may be administered concurrently. The CD20 binding antibody and BLyS antagonist may be administered sequentially. The BLyS antagonist is administered before the CD20 binding antibody. The B cell neoplasm may be non-Hodgkin's lymphoma (NHL), small lymphocytic (SL) NHL, lymphocyte predominant Hodgkin's disease (LPHD), follicular center cell (FCC) lymphomas, acute lymphocytic leukemia (ALL), chronic lymphocytic leukemia (CLL) and Hairy cell leukemia. In this method of treatment, BR3-Fc and Rituxan are administered at dosages disclosed in the section under Dosing. The BLyS antagonist and the CD20 binding antibody may be administered in conjunction with chemotherapy.

Disclosed herein is a method of alleviating a B-cell regulated autoimmune disorders comprising administering to a patient suffering from the disorder, a therapeutically effective amount of a CD20 binding antibody and of a BLyS antagonist. The autoimmune disorder may be selected from the group consisting of rheumatoid arthritis, juvenile rheumatoid arthritis, systemic lupus erythematosus (SLE), Wegener's disease, inflammatory bowel disease, idiopathic thrombocytopenic purpura (ITP), thrombotic throbocytopenic purpura (TTP), autoimmune thrombocytopenia, multiple sclerosis, psoriasis, IgA nephropathy, IgM polyneuropathies, myasthenia gravis, vasculitis, diabetes mellitus, Reynaud's syndrome, Sjorgen's syndrome and glomerulonephritis. Wherein the autoimmune disorder is rheumatoid arthritis or systemic lupus erythematosus, the BLyS antagonist and the CD20 binding antibody may be administered in conjunction with therapy using a drug selected from nonsteroidal anti-inflammatory drugs (NSAIDs), glucocorticoid, prednisone, and disease-modifying antirheumatic drug (DMARD).

In any of the methods of treatment or alleviation of a disorder disclosed herein where the CD20 binding antibody and BLyS antagonist are administered to a patient, the CD20 binding antibody and BLyS antagonist can be administered concurrently or sequentially. The BLyS antagonist may be administered before the CD20 binding antibody.

A composition comprising a CD20 binding antibody and a BLyS antagonist may also be provided.

Disclosed herein is an article of manufacture comprising CD20 binding antibody, a BLyS antagonist, and a label wherein the label indicates that the composition is for treating a B cell neoplasm or a B cell regulated autoimmune disorder.

In the compositions and articles of manufacture disclosed herein, the anti-CD20 antibody may include chimeric and humanized antibody. Specific examples of the anti-CD20 antibody include rituximab (RITUXAN®), m2H7 (murine 2H7), hu2H7 (humanized 2H7) and all its functional variants, hu2H7.v16 (v stands for version), v31, v96, v114, v115, having the amino acid sequences. Intact hu2H7.v16 has the mature L chain sequence of SEQ ID NO. 15 and H chain of SEQ ID NO. 16.

In any of disclosed herein of the methods, compositions and articles of manufacture disclosed herein, the BLyS antagonist, may be an immunoadhesin. The immunoadhesin may be selected from the group consisting of BR3 immunoadhesin comprising the extracellular domain of BR3, TACI immunoadhesin comprising the extracellular domain of TACI, and BCMA immunoadhesin comprising the extracellular domain of BCMA. The BLyS antagonist may be an anti-BLyS antibody, in particular, an anti-BLyS antibody that binds BLyS within a region of BLyS comprising residues 162-275. The BLyS antagonist may be an anti-BR3 antibody including one that binds BR3 in a region comprising residues 23-38 of human BR3. The amino acid positions of human BR3 refered to herein is according to the sequence numbering under human BR3 or alternative human BR3 dislosed herein under the "BR3" definition.

BLyS antagonist may be selected from the group consisting of a 17-mer polypeptide having the sequence of ECFDLLVRAWVPCSVLK (SEQ ID NO 5), ECFDLLVRHWVPCGLLR (SEQ ID NO 6), ECFDLLVRRWVPCEMLG (SEQ ID NO 7), ECFDLLVRSWVPCHMLR (SEQ ID NO 8), or ECFDLLVRHWVACGLLR (SEQ ID NO 9) as well as PEGylated forms of these 17mers;
a polypeptide having the sequence of 
MLPGCKWDLLIKQWVCDPLGSGSATGGSGSTASSGSGSATHMLPGCKWDLLIKQWVCD
PLGGGGGVDKTHTCPPCPAPELLGGPSVFLFPPKPKDTLMISRTPEVTCVVVDVSHEDPEVKFNWY
VDGVEVHNAKTKPREEQYNSTYRVVSVLTVLHQDWLNGKEYKCKVSNKALPAPIEKTISKAKGQP
REPQVYTLPPSRDELTKNQVSLTCLVKGFYPSDIAVEWESNGQPENNYKTTPPVLDSDGSFFLYSK
LTVDKSRWQQGNVFSCSVMHEALHNHYTQKSLSLSPGK (SEQ ID NO. 10);
 hBR3-Fc immunoadhesin having the sequence of

MSALLILALVGAAVASTRRGPRSLRGRDAPAPTPCVPAECFDLLVRHCVACGLLRTPRPKPAGASS
PAPRTALQPQESQVTDKAAHYTLCPPCPAPELLGGPSVFLFPPKPKDTLMISRTPEVTCVVVAVSHE
DPEVKFNWYVDGVEVHNAKTKPREEQYNSTYRVVSVLTVLHQDWLNGKEYKCKVSNKALPAPI
EKTISKAKGQPREPQVYTLPPSREEMTKNQVSLTCLVKGFYPSDIAVEWESNGQPENNYKTTPPVL
DSDGSFFLYSKLTVDKSRWQQGNVFSCSVMHEALHNHYTQKSLSLSPGK (SEQ ID NO. 2).

In any of the methods disclosed herein, the BLyS antagonist and the anti-CD20 antibody act synergistically to deplete the B cells.

### BRIEF DESCRIPTION OF THE FIGURES

Figures 1A-1B show a polynucleotide sequence encoding a native sequence human TACI (SEQ ID NO:__) and its amino acid sequence (SEQ ID NO:__).
Figure 2 shows a polynucleotide sequence encoding a native sequence human BCMA (SEQ ID NO:__) and its amino acid sequence (SEQ ID NO:__).
Figure 3 shows a polynucleotide sequence encoding a native sequence human BLyS (SEQ ID NO:__) and its amino acid sequence (SEQ ID NO: __).

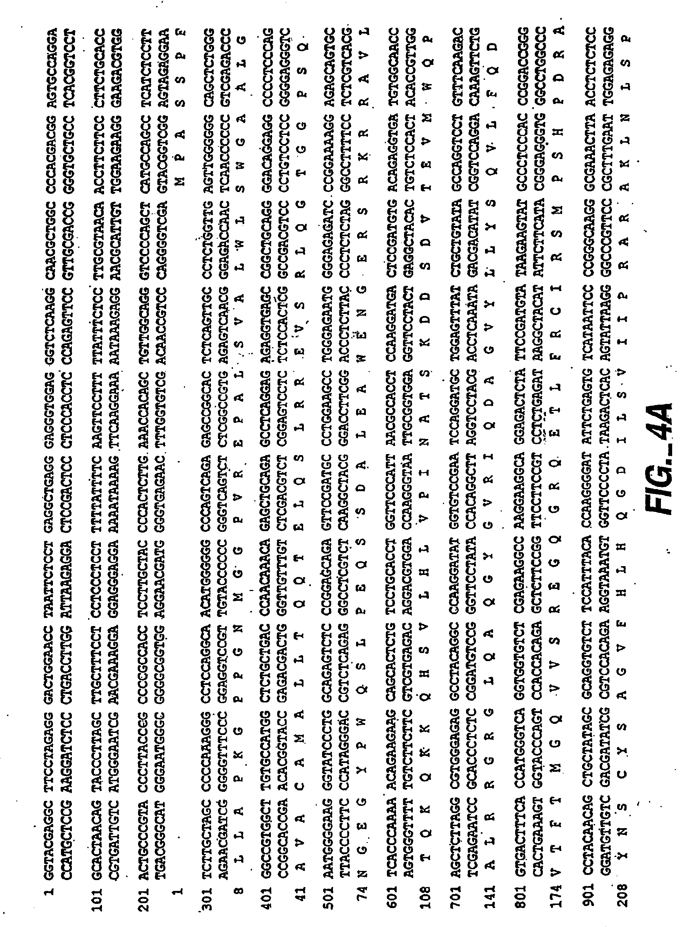

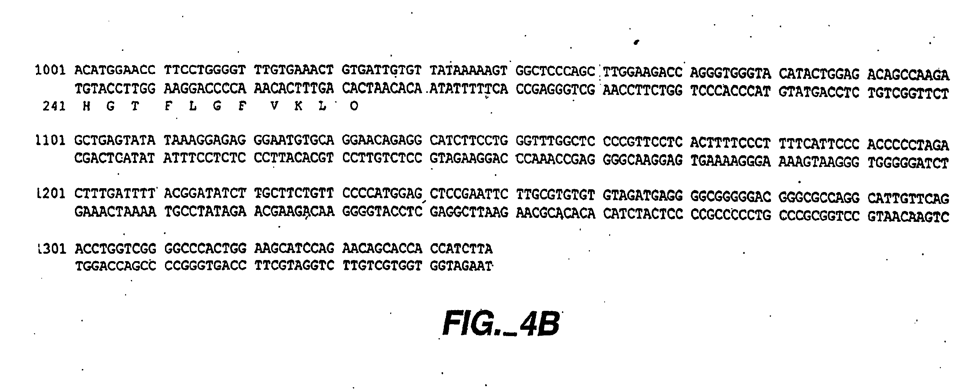

Figures 4A-4B show a polynucleotide sequence encoding a native sequence human APRIL (SEQ ID NO:__) and its putative amino acid sequence (SEQ ID NO:__).
Figure 5A shows a polynucleotide sequence (start and stop codons are underlined) encoding a native sequence human TACIs (SEQ ID NO:__) and Figure 5B shows its amino acid sequence (SEQ ID NO:__).

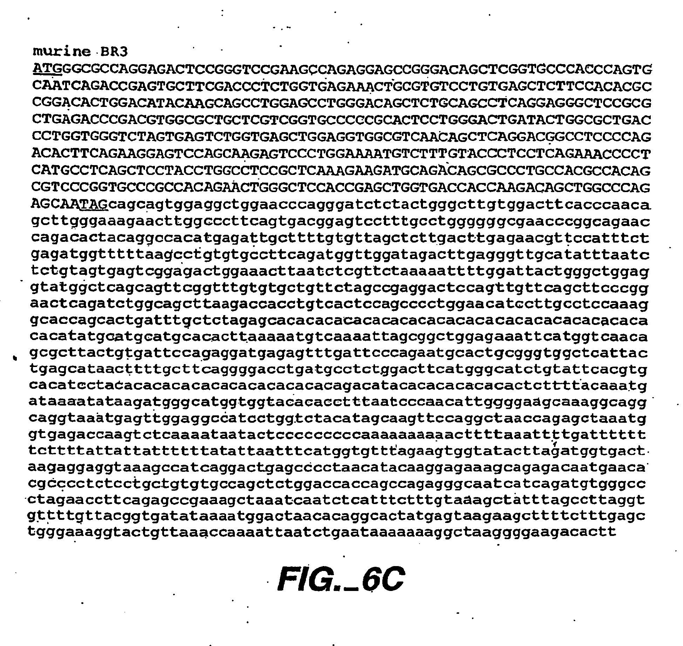

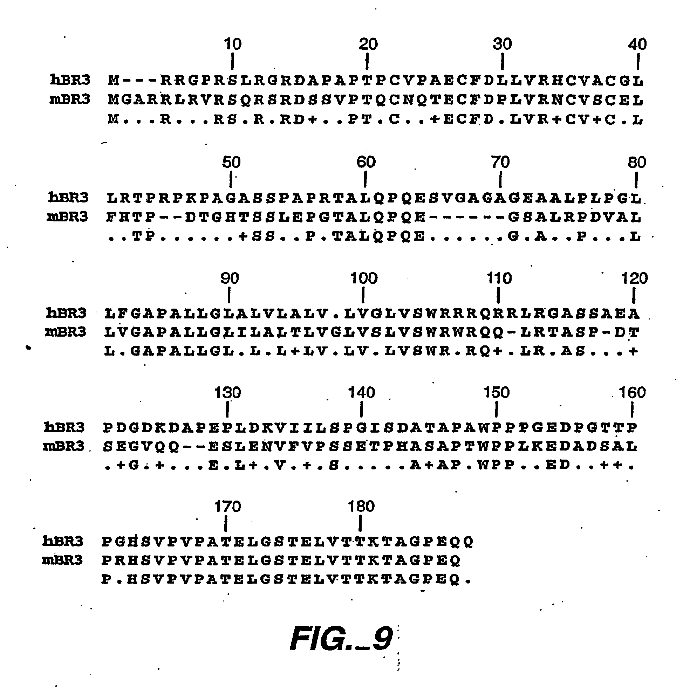

Figure 6A shows a polynucleotide sequence (start and stop codons are underlined) encoding a native sequence human BR3 (SEQ ID NO:__), and Figure 6B shows its amino acid sequence (SEQ ID NO:_); Figure 6C shows a polynucleotide sequence (start and stop codons are underlined) encoding murine BR3 (SEQ ID NO:__), and Figure 9A shows its amino acid sequence (SEQ ID NO:__).

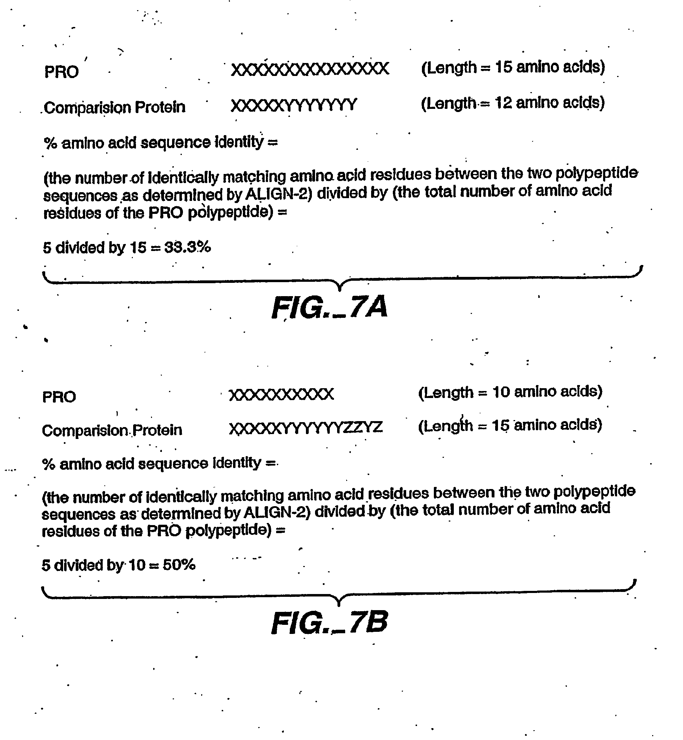

Figures 7A-7B show exemplary methods for calculating the % amino acid sequence identity of the amino acid sequence designated "Comparison Protein" to the amino acid sequence designated "PRO". For purposes herein, the "PRO" sequence may be the TACI, BCMA, TALL-1, APRIL, TACIs, or BR3 sequences referred to in the Figures herein.
Figure 8 shows an alignment of two amino acid sequences for the TACI receptor, referred to as "hTACI (265)" (SEQ ID NO:__), believed to be a spliced variant, and "hTACI", also referred to in Figures 1A-1B (SEQ ID NO:__)
Figure 9 shows a sequence alignment of human (SEQ ID NO:_) and murine BR3 (SEQ ID NO:__) with identical amino acids indicated by letter and conserved amino acids indicated by a plus sign below.
Figure 10 shows the amino acid sequence of human CD20 showing predicted transmembrane (boxed) and extracellular (underlined) regions. Potental Domains are 1-63: Cytoplasmic; 64-84: Transmembrane; 85-105: Transmembrane; 106-120: Cytoplasmic; 121-141: Transmembrane; 142-188: Extracellular; 189-209: Transmembrane; 210-297: Cytoplasmic; 81-167: Disulfide bond.
Figure 11 shows the nucleotide sequence for human CD20.
FIG. 12 is a sequence alignment comparing the amino acid sequences of the light chain variable domain (V_{L}) of murine 2H7 (SEQ ID NO._), humanized 2H7 v16 variant (SEQ ID NO._), and human kappa light chain subgroup I (SEQ ID NO._). The CDRs of V_{L} of 2H7 and hu2H7.v16 are as follows: CDR1 (SEQ ID NO._), CDR2 (SEQ ID NO._), and CDR3 (SEQ ID NO._).
FIG. 13 is a sequence alignment which compares the V_{H} sequences of murine 2H7 (SEQ ID NO._ ), humanized 2H7 v16 variant (SEQ ID NO._), and the human consensus sequence of heavy chain subgroup III (SEQ ID NO._). The CDRs of V_{H} of 2H7 and hu2H7.v16 are as follow: CDR1 (SEQ ID NO._), CDR2 (SEQ ID NO._), and CDR3 (SEQ ID NO._).

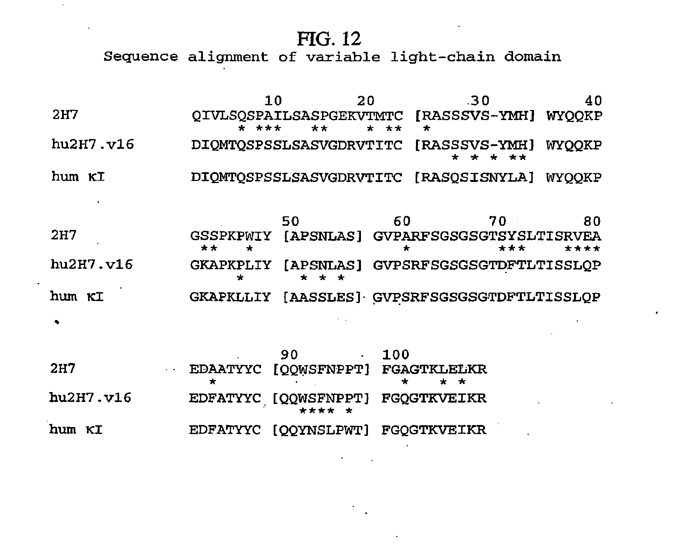

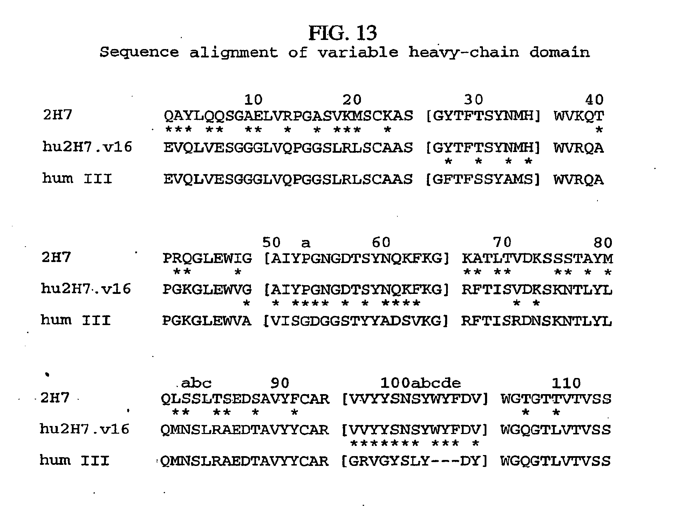

In FIG. 12 and FIG. 13, the CDR1, CDR2 and CDR3 in each chain are enclosed within brackets, flanked by the framework regions, FR1-FR4, as indicated. The asterisks in between two rows of sequences indicate the positions that are different between the two sequences. Residue numbering is according to Kabat et al., Sequences of Immunological Interest. 5th Ed. Public Health Service, National Institutes of Health, Bethesda, Md. (1991), with insertions shown as a, b, c, d, and e.

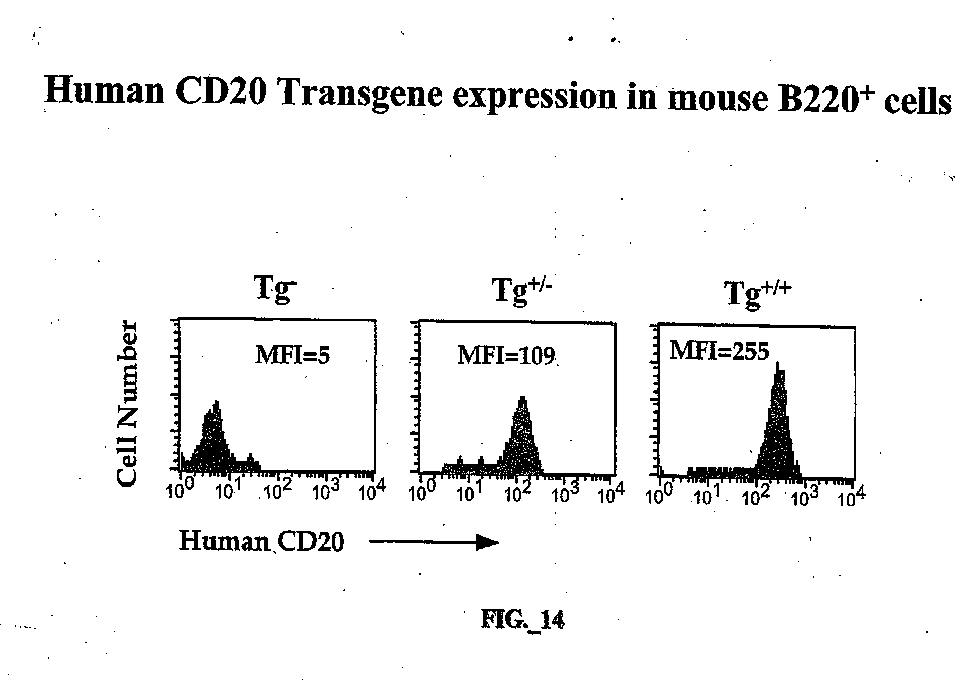

Figure 14 shows human CD20 transgene expression in mouse B220⁺ cells (B cells) of hCD20 BAC Tg+ mice.

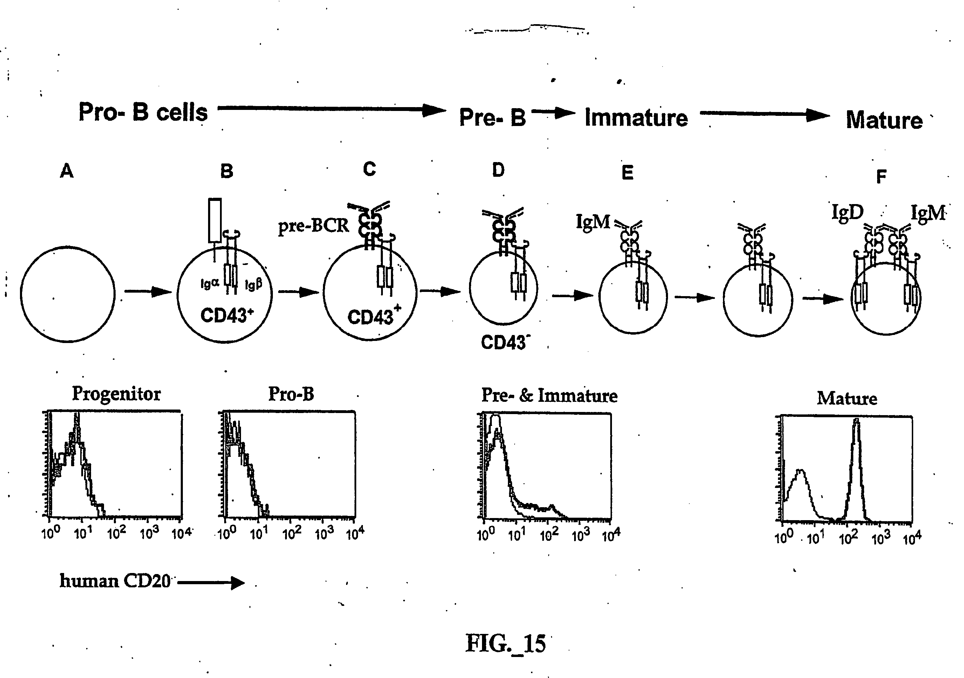

Figure 15 shows expression of human CD20 during B cell maturation in hCD20 BAC Tg mice. In Figures 15-19, the red line shows the negative control, staining of cells from transgene negative (Tg-) littermates with an anti-hCD20 mAb. Green line shows staining for human CD20 in Tg+ mice. Tg+ mice refers to hC20 BAC transgenic mice.

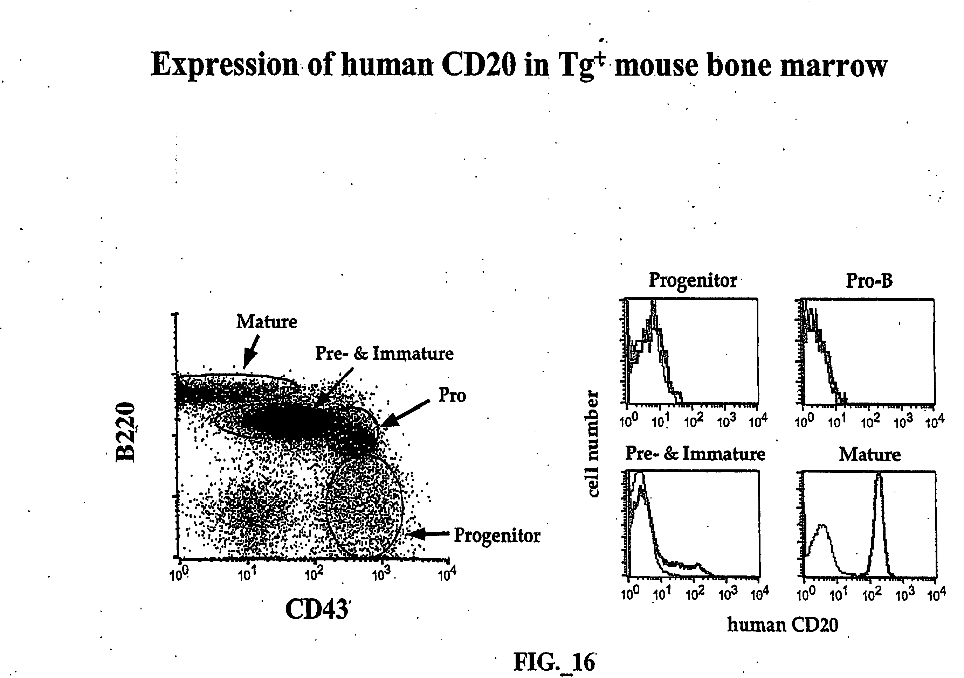

Figure 16 shows FACS plots demonstrating expression of human CD20 in the B cells of different maturation/differentiation stages (mature, pre-B and immature B, pro-B and progenitor B) in Tg+ mouse bone marrow.

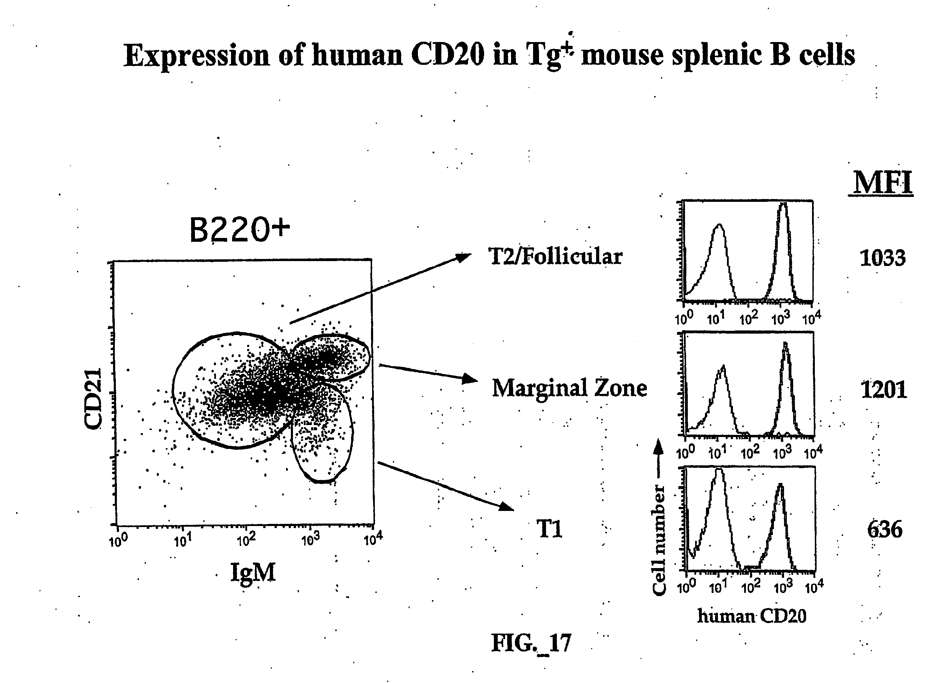

Figure 17 shows FACS plots demonstrating expression of human CD20 in Tg+ mouse splenic B cells. Cells were gated on B220+ to obtain B cells. IgM and CD21 allow delineation into the various B cell subsets of T2/follicular, marginal zone and T1.

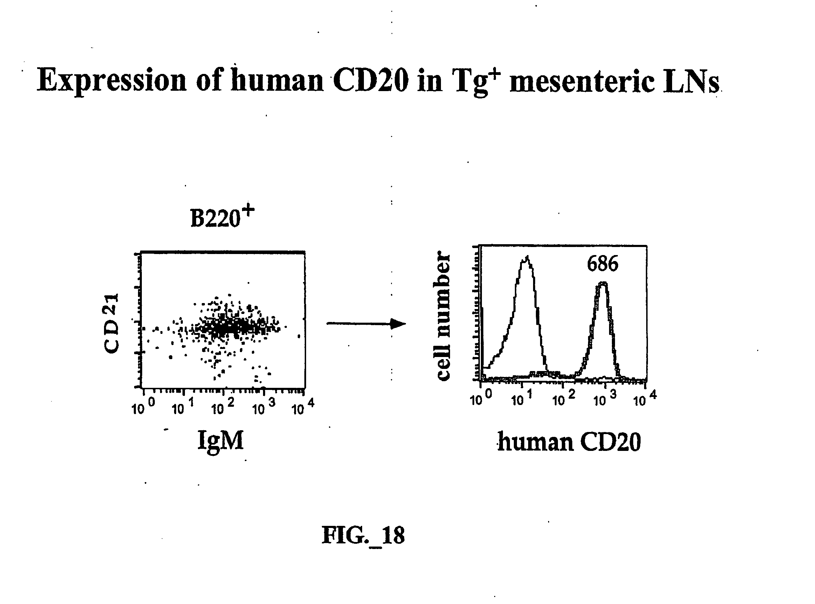

Figure 18 shows FACS plots demonstrating expression of human CD20 in Tg+ mouse mesenteric lymph nodes (LN).

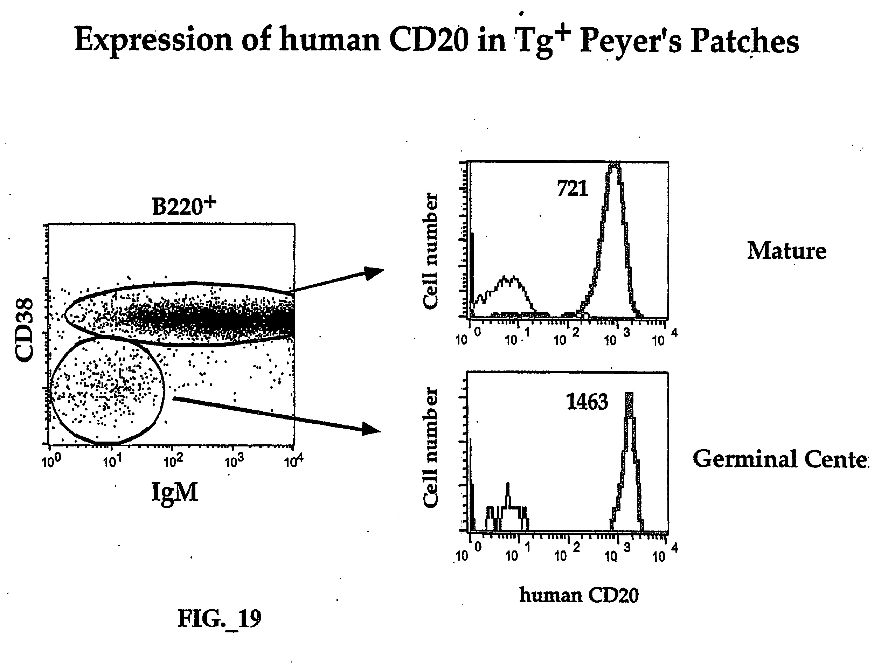

Figure 19 shows FACS plots demonstrating expression of human CD20 in Tg+ mouse Peyer's Patches. Cells were gated for B220+. The CD38 marker distinguishes mature from germinal center B cells.

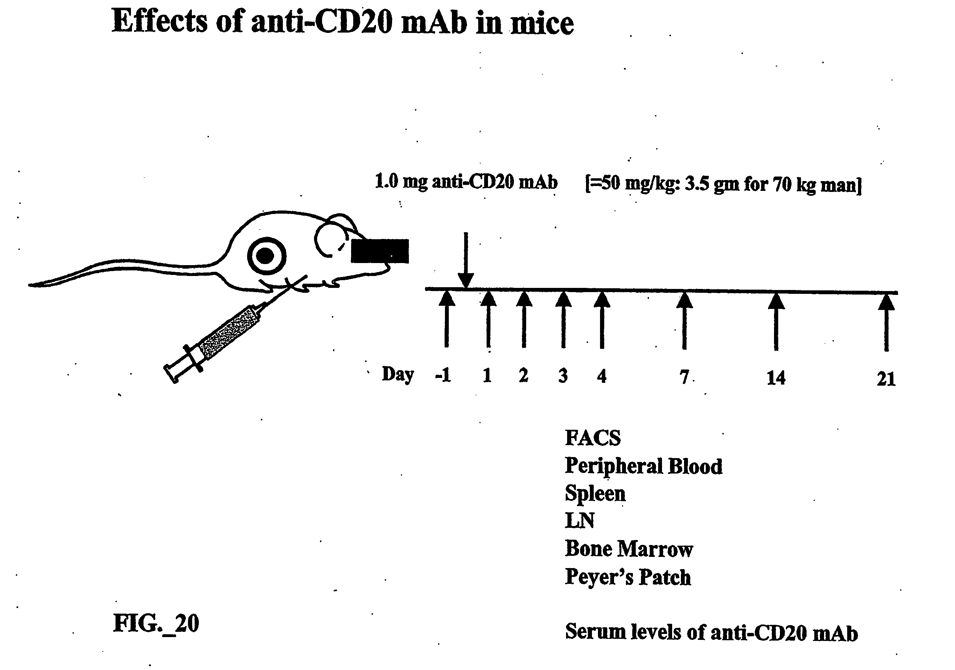

Figure 20 outlines studies on the effects of anti-hCD20 mAb in the human CD20 Tg+ mice. Mice were injected with 1.0mg [equivalent to 50mg/kg] anti-CD20 antibody on day 0 (black arrow above horizontal line) and cells were analyzed on the days indicated by red arrows below the horizontal line. FACS analyses were done on peripheral blood, spleen, lymph node, bone marrow, and Peyer's Patches. Serum levels of anti-hCD20 mAb were monitored.

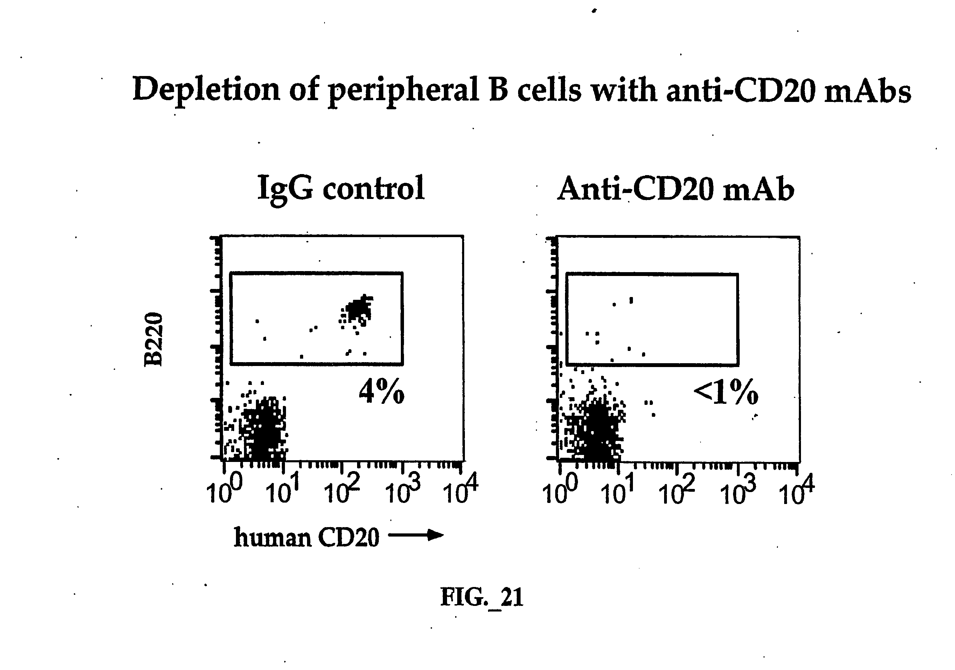

Figure 21 shows FACS plots demonstrating depletion of peripheral blood B cells with anti-hCD20 mAbs. The left panel shows the IgG control, i.e., animals treated with non-specific, isotype matched antibody.

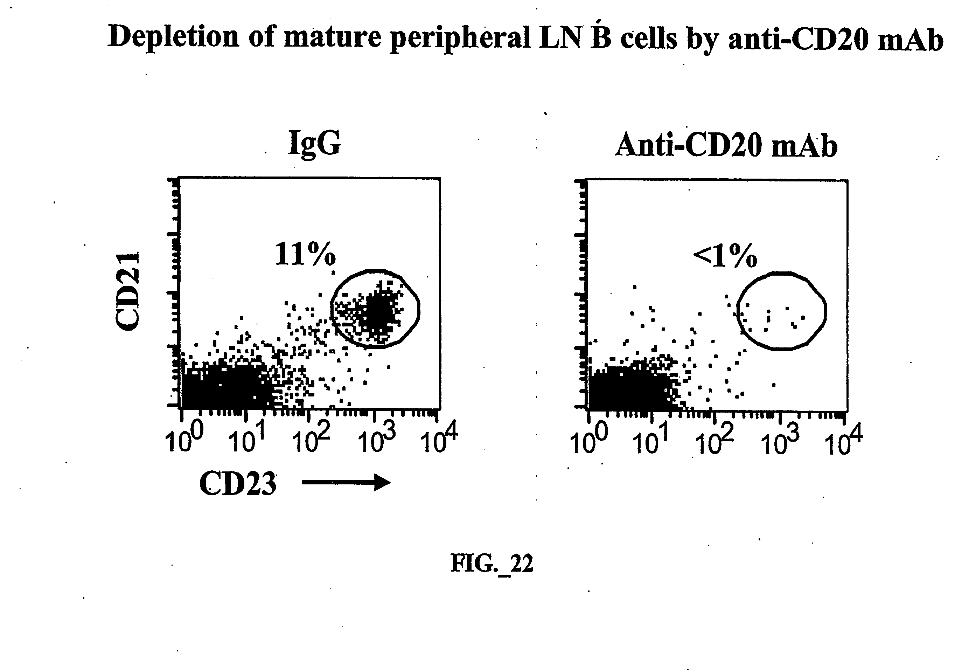

Figure 22 shows FACS plots demonstrating depletion of mature peripheral LN B cells by anti-hCD20 mAb in the right panel. The left panel shows the IgG control, i.e., animals treated with non-specific, isotype matched antibody. CD21⁺CD23⁺ gates for all B cells.

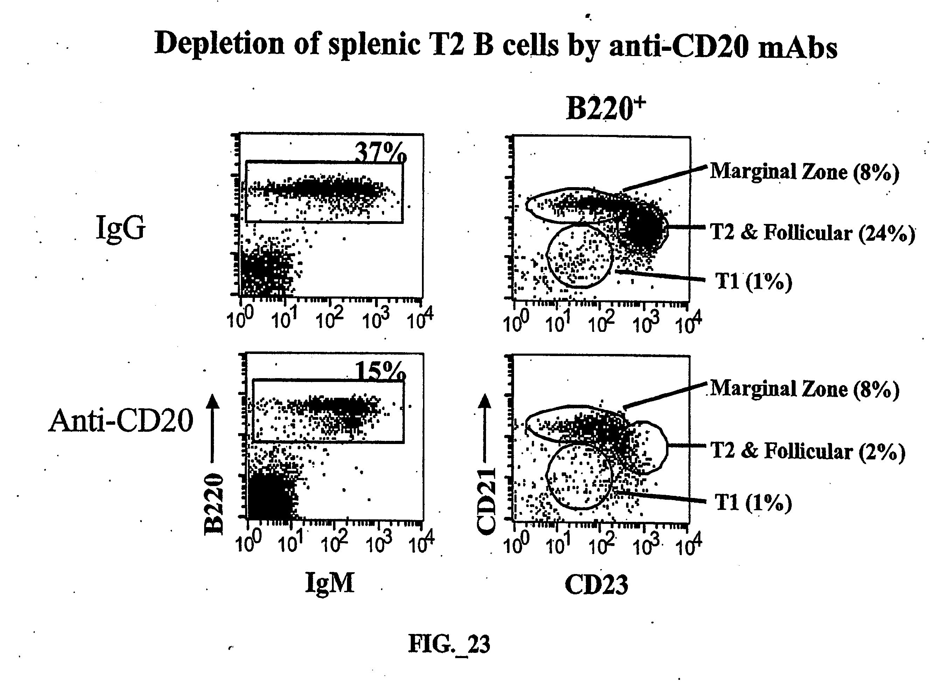

Figure 23 shows FACS plots demonstrating depletion of splenic T2 B cells, but not marginal zone B cells, by anti-hCD20 mAb.

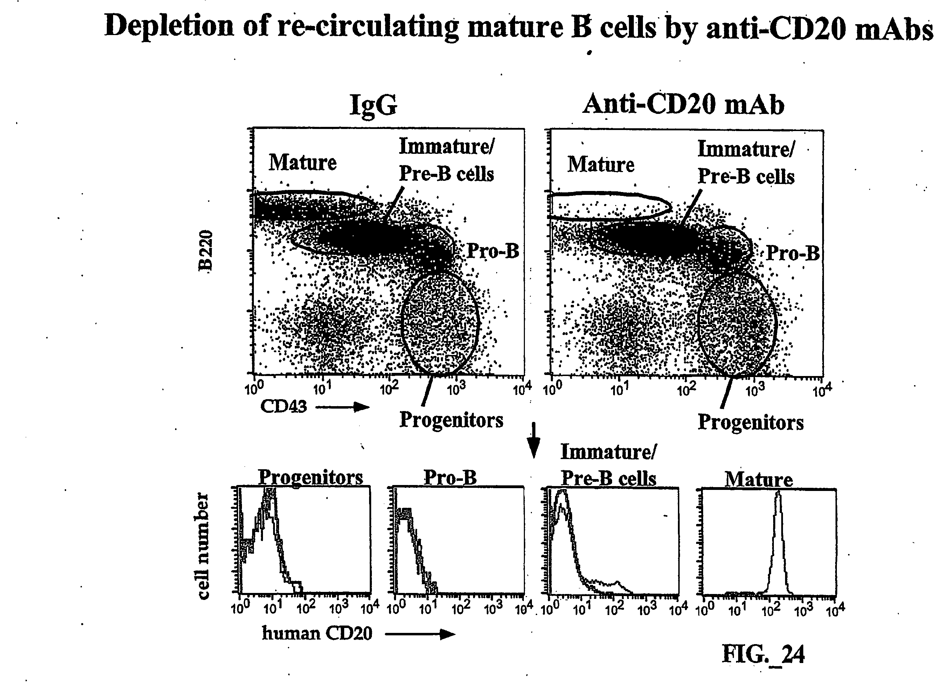

Figure 24 shows FACS plots demonstrating depletion of recirculating mature B cells, but not immature/pre-B or pro-B cells, by anti-hCD20 mAb. Red represents IgG-treated while green represents anti-hCD20 mAb treated mice expressing the hCD20. IgG treated mice (red) retained hCD20 expressing mature B cells, while anti-hCD20 mAb depleted hCD20 bearing cells. Human CD20 expression was monitored for detection of both unbound and Ab-bound CD20.

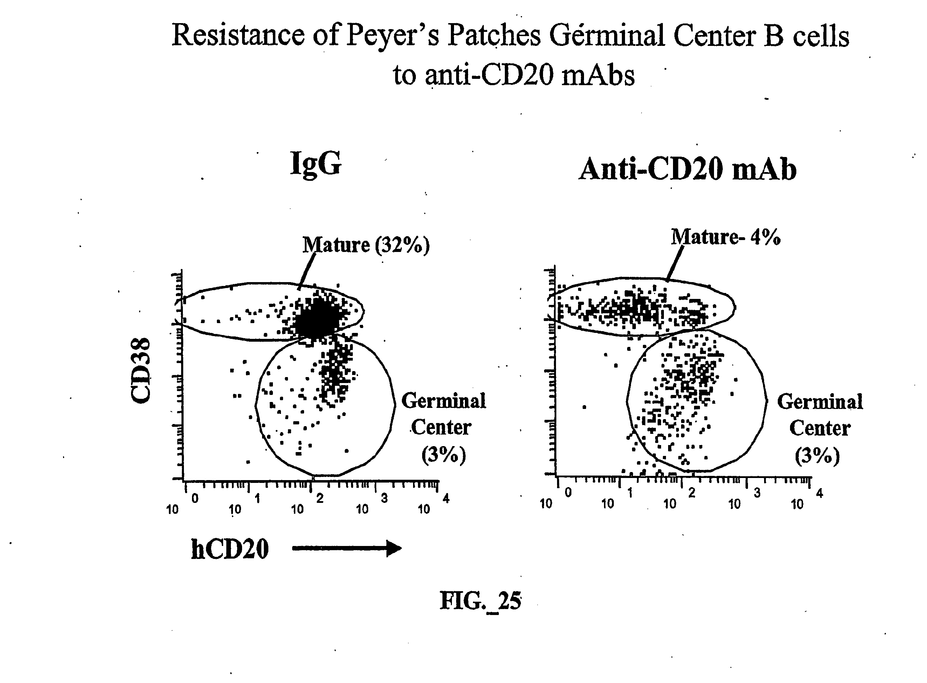

Figure 25 shows FACS plots demonstrating resistance of Peyer's patches germinal center B cells to anti-hCD20 mAbs. The left panel shows cells from the control IgG treated mice. The right panel shows cells from anti-CD20 mAb treated Tg+ mice.

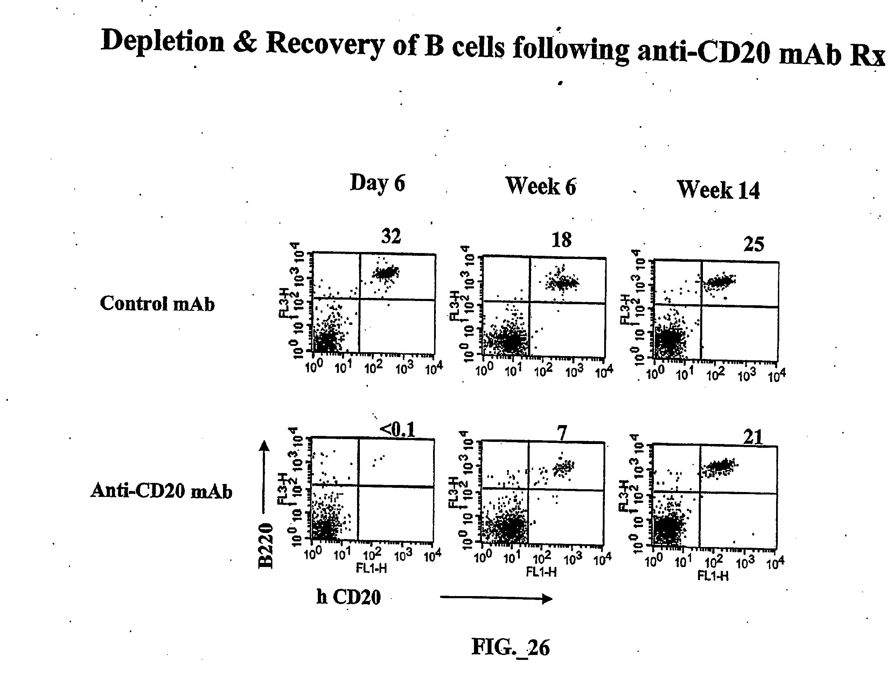

Figure 26 shows FACS plots demonstrating depletion and recovery of B cells in peripheral blood following treatment of the Tg+ mice with anti-hCD20 mAb. The top row panels show staining of cells from control mAb treated mice. Mice were administered antibody at day 1. With time, precursor B cells which do not express hCD20 develop into CD20+ mature B cells (see staining at week 6 and 14).

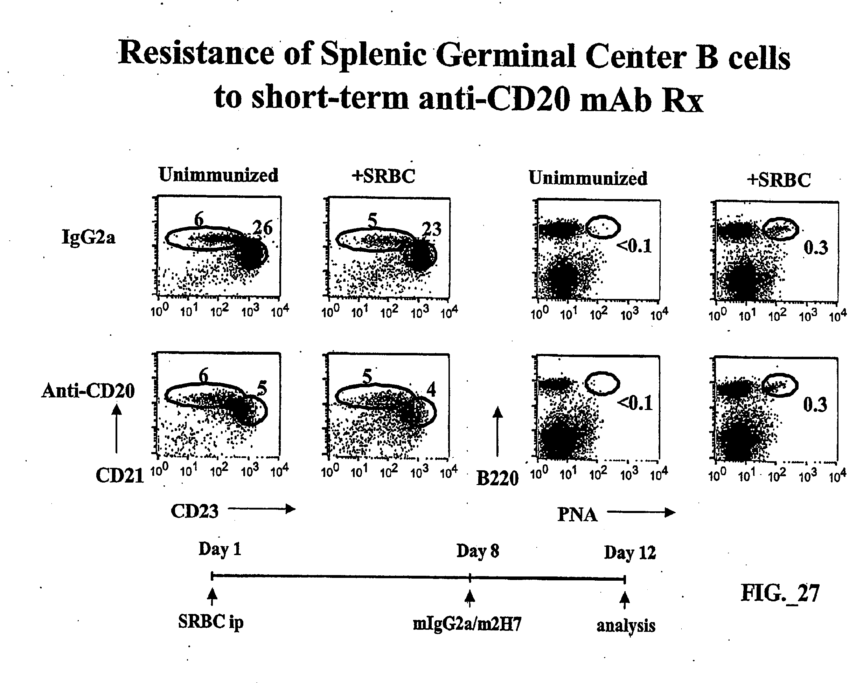

Figure 27 shows FACS plots demonstrating that resistance of splenic germinal center B cells to short-term (single injection) anti-CD20 mAb treatment. At day 8 following sheep red blood cell immunization to induce germinal center formation, one group of mice was treated with the m2H7 mAb to human CD20. The control set of mice was treated with mIgG2a isotype control antibody. Spleen cells from the mice were analyzed at day 12. PNA (peanut agglutinin) stains for germinal center. No depletion of germinal center B cells was detected with anti-CD20 treatment.

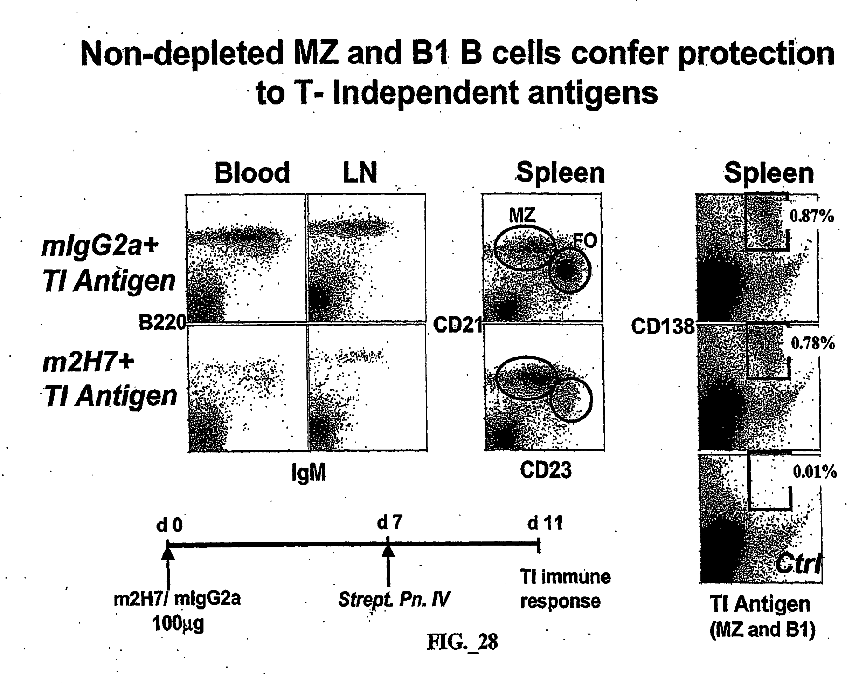

Figure 28 shows FACS plots demonstrating that non-depleted marginal zone (MZ) and B1 B cells confer protection to T-independent antigens. On the 3 panels at the right, the spleen cells were stained for the streptococcus polysaccharide-phosphatidyl choline (the TI antigen). CD138 is a marker for plasma cells.

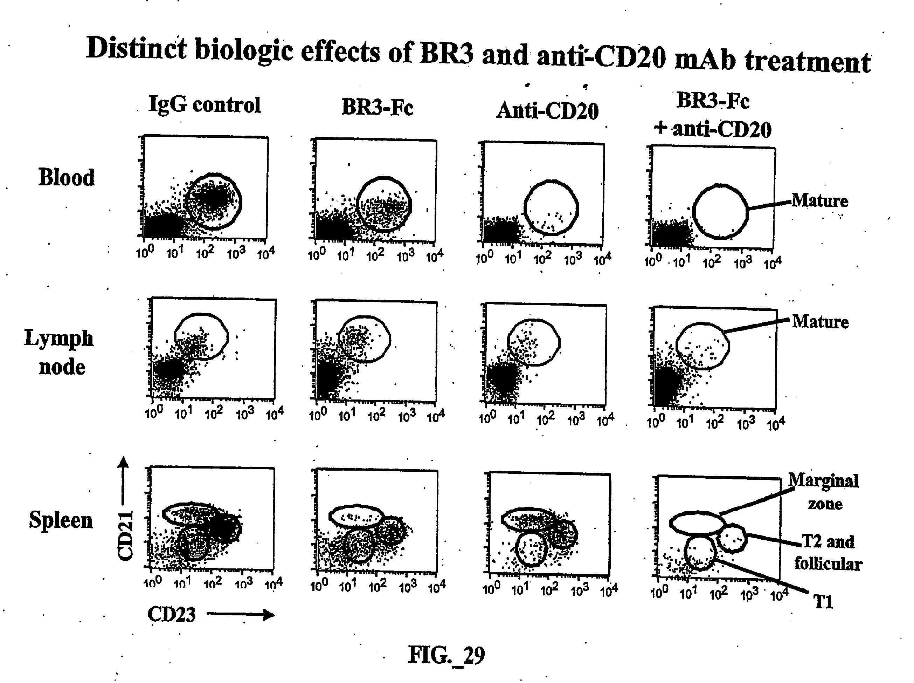

Figure 29 shows the distinct biological effects of the combination of a BLyS antagonist, BR3-Fc, and anti-CD20 mAb, m2H7, treatment in an animal model as described in Example 4. FACS analysis was performed on of spleen; blood, lymph node B cells (gated on CD21⁺CD23⁺). The combination therapy clearly produced a synergistic effect in depleting B cells, and especially marginal zone, T2 and follicular B cells in the spleen.

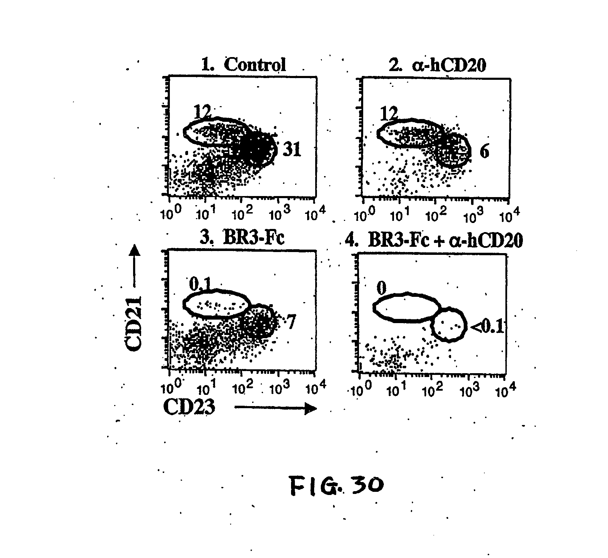

Figure 30 shows the synergistic effects on B cell depletion of the combination of anti-hCD20 mAb and BR3-Fc in the human CD20 Tg+ mice, as described in Example 4. Mice were treated with control IgG₂ₐ, BAFFR/BR3-Fc (100 µg/mouse IP daily for 12 days), anti-hCD20 mAb (100 µg/mouse IP on day 9) or the combination of BAFFR/BR3-Fc and anti-hCD20 mAb (same dosing as single treatment groups). B220⁺ splenocytes were isolated on day 13 and stained for CD21 and CD23. N=5 mice/group.

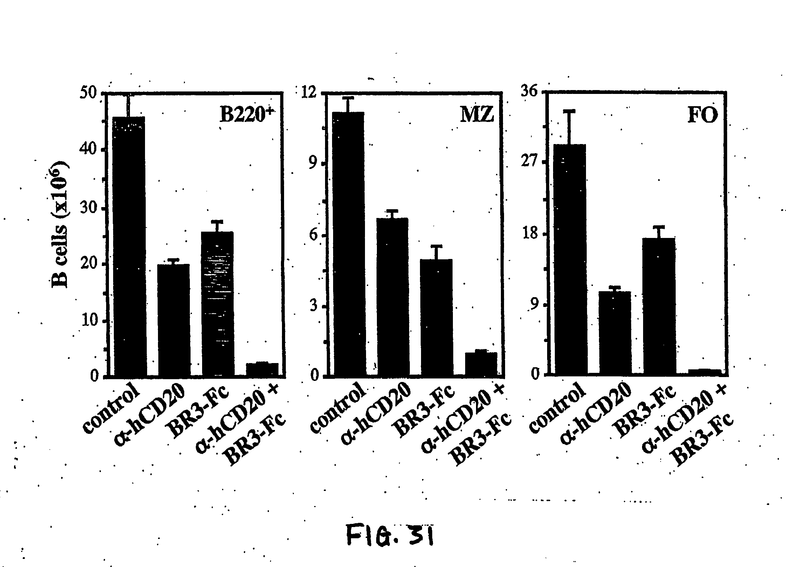

Figure 31 shows quantitation of depletion of the B220+ total spleen B cells (all subsets of MZ + FO + T1 + T2), marginal zone (MZ) and follicular (FO) B cells from hCD20 Tg+ mice as described in Example 4 and Fig. 30 except the mice were treated with single doses of 0.1 mg control IgG₂ₐ, BAFF/BR3-Fc or anti-hCD20 mAb. Splenocytes were analyzed on day 4. N=5 mice/group.

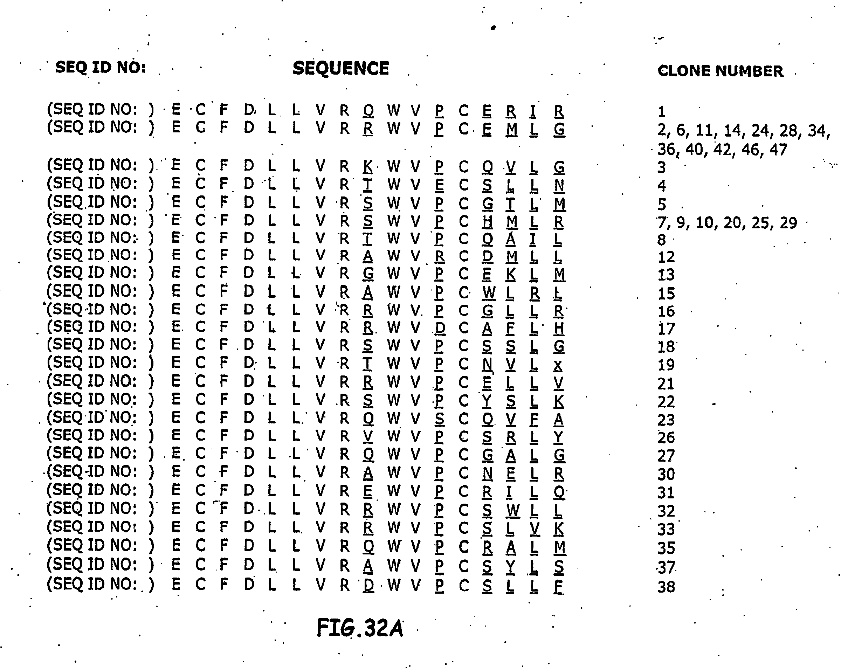

Figure 32A-C show the amino acid sequence of 17mers selected from phage display libraries for high affinity BLyS binding.

### DETAILED DESCRIPTION OF THE PREFERRED EMBODIMENTS

While anti-CD20 MAb treatment depletes certain subsets of B cells, we have previously observed that the marginal zone B cells, germinal center B cells and plasma cells are preserved. In contrast, blockade of B cell survival signals with BR3-Fc also depletes B cells or modulates B cell numbers, but to a different magnitude. It is believed that BR3 affects the survival of all B cells. It was discovered from the experiments described herein that administration of a combination of anti-CD20 antibody with a BLyS antagonist produced surprisingly synergistic results in depleting B cells in vivo. The combination of anti-CD20 antibody and therapies directed against the BLyS pathway provides a novel method of treating B cell-mediated diseases including B cell based malignancies and B-cell regulated autoimmune disorders. The combination therapy of anti-CD20 antibody with BLyS antagonist may offer effective and less-toxic alternatives to existing treatments for certain diseases, e.g., chronic lymphocytic leukemia (CLL) and small lymphocytic lymphoma (SLL).

An "autoimmune disease" herein is a non-malignant disease or disorder arising from and directed against an individual's own (self) antigens and/or tissues.

As used herein, "B cell depletion" refers to a reduction in B cell levels in an animal or human after drug or antibody treatment, as compared to the level before treatment. B cell levels are measurable using well known assays such as by getting a complete blood count, by FACS analysis staining for known B cell markers, and by methods such as described in the Experimental Examples. B cell depletion can be partial or complete. In one embodiment, the depletion of CD20 expressing B cells is at least 25%. In a patient receiving a B cell depleting drug, B cells are generally depleted for the duration of time when the drug is circulating in the patient's body and the time for recovery of B cells.

The "CD20" antigen is a non-glycosylated, transmembrane phosphoprotein with a molecular weight of approximately 35 kD that is found on the surface,of greater than 90% ofB cells from peripheral blood or lymphoid organs. CD20 is expressed during early pre-B cell development and remains until plasma cell differentiation; it is not found on human stem cells, lymphoid progenitor cells or normal plasma cells. CD20 is present on both normal B cells as well as malignant B cells. Other names for CD20 in the literature include "B-lymphocyte-restricted differentiation antigen" and "Bp35". The CD20 antigen is described in, for example, Clark and Ledbetter, Adv. Can. Res. 52:81-149 (1989) and Valentine et al. J. Biol. Chem. 264(19):11282-11287 (1989).

CD20 binding antibody and anti-CD20 antibody are used interchangeably herein and encompass all antibodies that bind CD20 with sufficient affinity such that the antibody is useful as a therapeutic agent in targeting a cell expressing the antigen, and do not significantly cross-react with other proteins such as a negative control protein in the assays described below. Bispecific antibodies wherein one arm of the antibody binds CD20 are also contemplated. Also encompassed by this definition of CD20 binding antibody are functional fragments of the preceding antibodies. The CD20 binding antibody will bind CD20 with a Kd of < 10nM. In preferred embodiments, the binding is at a Kd of < 7.5nM, more preferably < 5nM, even more preferably at between 1-5nM, most preferably, <1nM.

Examples of antibodies which bind the CD20 antigen include: "C2B8" which is now called "Rituximab" ("RITUXAN®") (US Patent No. 5,736,137) the yttrium-[90]-labeled 2B8 murine antibody designated "Y2B8" or "Ibritumomab Tiuxetan" ZEVALIN® (US Patent No. 5,736,137); murine IgG2a "B1," also called "Tositumomab," (Beckman Coulter) optionally labeled with ¹³¹I to generate the "131I-B1" antibody (iodine I131 tositumomab, BEXXAR™) (US Patent No. 5,595,721); murine monoclonal antibody "1F5" (Press et al. Blood 69(2):584-591 (1987) and variants thereof including "framework patched" or humanized 1F5 (WO03/002607, Leung, S.); ATCC deposit HB-96450); murine 2H7 and chimeric 2H7 antibody (US Patent No. 5,677,180); humanized 2H7; huMax-CD20 (Genmab, Denmark); AME-133 (Applied Molecular Evolution); A20 antibody or variants thereof such as chimeric or humanized A20 antibody (cA20, hA20, respectively) (US 2003/0219433, Immunomedics); and monoclonal antibodies L27, G28-2, 93-1B3, B-C1 or NU-B2 available from the International Leukocyte Typing Workshop (Valentine et al., In: Leukocyte Typing III (McMichael, Ed., p. 440, Oxford University Press (1987)).

The terms "rituximab" or "RITUXAN®" herein refer to the genetically engineered chimeric murine/human monoclonal antibody directed against the CD20 antigen and designated "C2B8" in US Patent No. 5,736,137, including fragments thereof which retain the ability to bind CD20.

The anti-CD20 antibodies may bind human and primate CD20. The antibodies that bind CD20 may be humanized or chimeric. CD20 binding antibodies include rituximab (RITUXAN®), m2H7 (murine 2H7), hu2H7 (humanized 2H7) and all its functional variants, including without limitation, hu2H7.v16 (v stands for version), v31, v73, v75, as well as fucose deficient variants. The sequences of some of the hu2H7 variant antibodies are provided below, with the sequences N-terminal sequence in bold being the leader sequence which is removed in the mature polypeptide:
hu2H7.v16 L chain [232 aa] (SEQ ID NO. 3) 
MGWSCIILFLVATATGVHSDIQMTQSPSSLSASVGDRVTITCRASSSVSYMHWYQQKPGK
APKPLIYAPSNLASGVPSRFSGSGSGTDFTLTISSLQPEDFATYYCQQWSFNPPTFGQGT
KVEIKRTVAAPSVFIFPPSDEQLKSGTASVVCLLNNFYPREAKVQWKVDNALQSGNSQES
VTEQDSKDSTYSLSSTLTLSKADYEKHKVYACEVTHQGLSSPVTKSFNRGEC
hu2H7.v16 H chain [471 aa] (SEQ ID NO. 4) 
MGWSCIILFLVATATGVHSEVQLVESGGGLVQPGGSLRLSCAASGYTFTSYNMHWVRQAP
GKGLEWVGAIYPGNGDTSYNQKFKGRFTISVDKSKNTLYLQMNSLRAEDTAVYYCARVVY
YSNSYWYFDVWGQGTLVTVSSASTKGPSVFPLAPSSKSTSGGTAALGCLVKDYFPEPVTV
SWNSGALTSGVHTFPAVLQSSGLYSLSSVVTVPSSSLGTQTYICNVNHKPSNTKVDKKVE
PKSCDKTHTCPPCPAPELLGGPSVFLFPPKPKDTLMISRTPEVTCVVVDVSHEDPEVKFN
WYVDGVEVHNAKTKPREEQYNSTYRVVSVLTVLHQDWLNGKEYKCKVSNKALPAPIEKTI
SKAKGQPREPQVYTLPPSREEMTKNQVSLTCLVKGFYPSDIAVEWESNGQPENNYKTTPP
VLDSDGSFFLYSKLTVDKSRWQQGNVFSCSVMHEALHNHYTQKSLSLSPGK
 hu2H7.v31 H chain [471 aa] SEQ ID NO. 11: 
MGWSCIILFLVATATGVHSEVQLVESGGGLVQPGGSLRLSCAASGYTFTSYNMHWVRQAP
GKGLEWVGAIYPGNGDTSYNQKFKGRFTISVDKSKNTLYLQMNSLRAEDTAVYYCARVVY
YSNSYWYFDVWGQGTLVTVSSASTKGPSVFPLAPSSKSTSGGTAALGCLVKDYFPEPVTV
SWNSGALTSGVHTFPAVLQSSGLYSLSSVVTVPSSSLGTQTYICNVNHKPSNTKVDKKVE
PKSCDKTHTCPPCPAPELLGGPSVFLFPPKPKDTLMISRTPEVTCVVVDVSHEDPEVKFN
WYVDGVEVHNAKTKPREEQYNATYRVVSVLTVLHQDWLNGKEYKCKVSNKALPAPIAATI
SKAKGQPREPQVYTLPPSREEMTKNQVSLTCLVKGFYPSDIAVEWESNGQPENNYKTTPP
VLDSDGSFFLYSKLTVDKSRWQQGNVFSCSVMHEALHNHYTQKSLSLSPGK
The L chain of v31 is the same as that of v16 above, i.e., SEQ ID NO. 3.

Purely for the purposes herein, "humanized 2H7v.16" refers to an intact antibody or antibody fragment comprising the variable light chain sequence: 
DIQMTQSPSSLSASVGDRVTITCRASSSVSYMHWYQQKPGKAPKPLIYAPSNLASGVPSRFSGSGSG
TDFTLTISSLQPEDFATYYCQQWSFNPPTFGQGTKVEIKR
(SEQ ID NO:13); and
 variable heavy sequence: 


(SEQ ID NO: 14)
 Where the humanized 2H7v.16 antibody is an intact antibody, preferably it comprises the v16 light chain amino acid sequence: 
DIQMTQSPSSLSASVGDRVTITCRASSSVSYMHWYQQKPGKAPKPLIYAPSNLASGVPSRFSGSGSG
TDFTLTISSLQPEDFATYYCQQWSFNPPTFGQGTKVEIKRTVAAPSVFIFPPSDEQLKSGTASVVCLL
NNFYPREAKVQWKVDNALQSGNSQESVTEQDSKDSTYSLSSTLTLSKADYEKHKVYACEVTHQG
LSSPVTKSFNRGEC (SEQ ID NO: 15); and
 v16 heavy chain amino acid sequence

EVQLVESGGGLVQPGGSLRLSCAASGYTFTSYNMHWVRQAPGKGLEWVGAIYPGNGDTSYNQKF
KGRFTISVDKSKNTLYLQMNSLRAEDTAVYYCARVVYYSNSYWYFDVWGQGTLVTVSSASTKGP
SVFPLAPSSKSTSGGTAALGCLVKDYFPEPVTVSWNSGALTSGVHTFPAVLQSSGLYSLSSVVTVPS
SSLGTQTYICNVNHKPSNTKVDKKVEPKSCDKTHTCPPCPAPELLGGPSVFLFPPKPKDTLMISRTP
EVTCVVVDVSHEDPEVKFNWYVDGVEVHNAKTKPREEQYNSTYRVVSVLTVLHQDWLNGKEYK
CKVSNKALPAPIEKTISKAKGQPREPQVYTLPPSREEMTKNQVSLTCLVKGFYPSDIAVEWESNGQ
PENNYKTTPPVLDSDGSFFLYSKLTVDKSRWQQGNVFSCSVMHEALHNHYTQKSLSLSPGK
(SEQ ID NO: 16).

The V region of all other variants based on version 16 will have the amino acid sequences of v16 except at the positions of amino acid substitutions which are indicated in the table below. Unless otherwise indicated, the 2H7 variants will have the same L chain as that of v16.

| 2H7 version | Heavy chain (V_{H}) changes | Light chain (V_{L}) changes | Fc changes |
|---|---|---|---|
| 31 | - | - | S298A, E333A, K334A (SEQ ID NO. 17) |
| 96 | D56A, N100A | S92A (SEQ ID NO. 18) | |
| 114 | D56A, N100A | M32L, S92A (SEQ ID NO. 19) | S298A, E333A, K334A (SEQ ID NO. 20) |
| 115 | D56A, N100A | M32L, S92A (SEQ ID NO. 21) | S298A, E333A, K334A, E356D, M358L (SEQ ID NO. 22) |

A variant of the preceding humanized 2H7 mAb is 2H7v.31 having the same L chain sequence as SEQ ID NO: 15 above, with the H chain amino acid sequence: 
EVQLVESGGGLVQPGGSLRLSCAASGYTFTSYNMHWVRQAP
GKGLEWVGAIYPGNGDTSYNQKFKGRFTISVDKSKNTLYLQMNSLRAEDTAVYYCARVVY
YSNSYWYFDVWGQGTLVTVSSASTKGPSVFPLAPSSKSTSGGTAALGCLVKDYFPEPVTV
SWNSGALTSGVHTFPAVLQSSGLYSLSSVVTVPSSSLGTQTYICNVNHKPSNTKVDKKVE
PKSCDKTHTCPPCPAPELLGGPSVFLFPPKPKDTLMISRTPEVTCVVVDVSHEDPEVKFN
WYVDGVEVHNAKTKPREEQYNATYRVVSVLTVLHQDWLNGKEYKCKVSNKALPAPIAATI
SKAKGQPREPQVYTLPPSREEMTKNQVSLTCLVKGFYPSDIAVEWESNGQPENNYKTTPP
VLDSDGSFFLYSKLTVDKSRWQQGNVFSCSVMHEALHNHYTQKSLSLSPGK (SEQ ID NO. 17).
 The murine anti-human CD20 antibody, m2H7, has the VH sequence: 
QAYLQQSGAELVRPGASVKMSCKASGYTFTSYNMHWVKQTPRQGLEWIGAIYPGNGDTSY
NQKFKGKATLTVDKSSSTAYMQLSSLTSEDSAVYFCARVVYYSNSYWYFDVWGTGTTVTV
S (SEQ ID NO: 23)
 And VL sequence: 

 (SEQ ID NO: 24)
 Unless indicated, the sequences disclosed herein of the humanized 2H7v.16 and variants thereof are of the mature polypeptide, i.e., without the leader sequence.

Patents and patent publications concerning CD20 antibodies include US Patent Nos. 5,776,456, 5,736,137, 5,843,439, 6,399,061, and 6,682,734, as well as US patent appln nos. US 2002/0197255A1, US 2003/0021781A1, US 2003/0082172 A1, US 2003/0095963 A1, US 2003/0147885 A1 (Anderson et al.*);* US Patent No. 6,455,043B1 and WO00/09160 (Grillo-Lopez, A.); WO00/27428 (Grillo-Lopez and White); WO00/27433 (Grillo-Lopez and Leonard); WO00/44788 (Braslawsky et al.*);* WO01/10462 (Rastetter, W.); WO01/10461 (Rastetter and White); WO01/10460 (White and Grillo-Lopez); US2001/0018041A1, US2003/0180292A1, WO01/34194 (Hanna and Hariharan); US appln no. US2002/0006404 and WO02/04021 (Hanna and Hariharan); US appln no. US2002/0012665 A1 and WO01/74388 (Hanna, N.); US appln no. US 2002/0058029 A1 (Hanna, N.); US appln no. US 2003/0103971 A1 (Hariharan and Hanna); US appln no. US2002/0009444A1, and WO01/80884 (Grillo-Lopez, A.); WO01/97858 (White, C.); US appln no. US2002/0128488A1 and WO02/34790 (Reff, M.);WO02/060955 (Braslawsky et al.);WO2/096948 (Braslawsky et al.);WO02/079255 (Reff and Davies); US Patent No. 6,171,586B 1, and WO98/56418 (Lam et al.); WO98/58964 (Raju, S.); WO99/22764 (Raju, S.);WO99/51642, US Patent No. 6,194,551B1, US Patent No. 6,242,195B1, US Patent No. 6,528,624B1 and US Patent No. 6,538,124 (Idusogie et al.); WO00/42072 (Presta, L.); WO00/67796 (Curd et al.); WO01/03734 (Grillo-Lopez et al.); US appln no. US 2002/0004587A1 and WO01/77342 (Miller and Presta); US appln no. US2002/0197256 (Grewal, I.); US Appln no. US 2003/0157108 A1 (Presta, L.); US Patent Nos. 6,565,827B 1, 6,090,365B 1, 6,287,53781, 6,015,542, 5,843,398, and 5,595,721, (Kaminski et al.); US Patent Nos. 5,500,362, 5,677,180, 5,721,108, 6,120,767, 6,652,852B1 (Robinson et al.); US Pat No. 6,410,391B1 (Raubitschek et al.); US Patent No. 6.224,866B1 and WO00/20864 (Barbera-Guillem, E.); WO01/13945 (Barbera-Guillem, E.); WO00/67795 (Goldenberg); US Appl No. US 2003/0133930 A1 and WO00/74718 (Goldenberg and Hansen); WO00/76542 (Golay et al.);WO01/72333 (Wolin and Rosenblatt); US Patent No. 6,368,596B1 (Ghetie et al.); US Patent No. 6,306,393 and US Appln no. US2002/0041847 A1, (Goldenberg, D.); US Appln no. US2003/0026801A1 (Weiner and Hartmann); WO02/102312 (Engleman, E.); US Patent Application No. 2003/0068664 (Albitar et al.); WO03/002607 (Leung, S.); WO 03/049694, US2002/0009427A1, and US 2003/0185796 A1 (Wolin et al.); WO03/061694 (Sing and Siegall); US 2003/0219818 A1 (Bohen et al.); US 2003/0219433 A1 and WO 03/068821 (Hansen et al.); US2003/0219818A1 (Bohen et al.); US2002/0136719A1 (Shenoy et al.); WO2004/032828 (Wahl et al.). See, also, US Patent No. 5,849,898 and EP appln no. 330,191 (Seed et al.*);* US Patent No. 4,861,579 and EP332,865A2 (Meyer and Weiss); USP 4,861,579 (Meyer et al.*);* WO95/03770 (Bhat et al.); US 2003/0219433 A1 (Hansen et al.).

The CD20 antibodies can be naked antibody or conjugated to a cytotoxic compound such as a radioisotope, or a toxin. Such antibodies include the antibody Zevalin^{™} which is linked to the radioisotope, Yttrium-90 (IDEC Pharmaceuticals, San Diego, CA), and Bexxar^{™} which is conjugated to I-131 (Corixa, WA). The humanized 2H7 variants include those that have amino acid substitutions in the FR and affinity maturation variants with changes in the grafted CDRs. The substituted amino acids in the CDR or FR are not limited to those present in the donor or acceptor antibody. The anti-CD20 antibodies may further comprise changes in amino acid residues in the Fc region that lead to improved effector function including enhanced CDC and/or ADCC function and B-cell killing (also referred to herein as B-cell depletion). In particular, three mutations have been identified for improving CDC and ADCC activity: S298A/E333A/K334A (also referred to herein as a triple Ala mutant or variant; numbering in the Fc region is according to the EU numbering system; Kabat et al., *supra)* as described (Idusogie et al., *supra* (2001); Shields et al., *supra*).

Other anti-CD20 antibodies of the invention include those having specific changes that improve stability. The chimeric anti-CD20 antibody may be murine V regions and human C region. One such specific chimeric anti-CD20 antibody is Rituxan® (Rituximab®; Genentech, Inc.). Rituximab and hu2H7 can mediate lysis of B-cells through both complement-dependent cytotoxicity (CDC) and antibody-dependent cellular cytotoxicity (ADCC). Antibody variants with altered Fc region amino acid sequences and increased or decreased C1q binding capability are described in US patent No. 6,194,551B 1 and WO99/51642. See, also, Idusogie et al. J. Immunol. 164: 4178-4184 (2000).

WO00/42072 (Presta) describes polypeptide variants with improved or diminished binding to FcRs. See, also, Shields et al. J. Biol. Chem. 9(2): 6591-6604 (2001).

The N-glycosylation site in IgG is at Asn297 in the CH2 domain. Encompassed herein are humanized CD20-binding antibodies having a Fc region, wherein about 80-100% (and preferably about 90-99%) of the antibody in the composition comprises a mature core carbohydrate structure which lacks fucose, attached to the Fc region of the glycoprotein. Such antibodies show improvement in binding to FcγRIIIA(F158), which is not as effective as FcγRIIIA (V158) in interacting with human IgG.

The term "antibody" is used in the broadest sense and specifically covers, for example, monoclonal antibodies, polyclonal antibodies, antibodies with polyepitopic specificity, single chain antibodies, and fragments of antibodies. The polypeptide may be is fused into an antibody framework, for example, in the variable region or in a CDR such that the antibody can bind to and inhibit BLyS binding to BR3 or BLyS signaling. The antibodies comprising a polypeptide of this invention can be chimeric, humanized, or human. The antibodies comprising a polypeptide disclosed herein can be an antibody fragment. Such antibodies and methods of generating them are described in more detail below. Alternatively, an antibody of this invention can be produced by immunizing an animal with a polypeptide disclosed herein. Thus, an antibody directed against a polypeptide of this invention is contemplated.

The term "monoclonal antibody" as used herein refers to an antibody obtained from a population of substantially homogeneous antibodies, i.e., the individual antibodies comprising the population are identical except for possible naturally occurring mutations that can be present in minor amounts. Monoclonal antibodies are highly specific, being directed against a single antigenic site. Furthermore, in contrast to conventional (polyclonal) antibody preparations which typically include different antibodies directed against different determinants (epitopes), each monoclonal antibody is directed against a single determinant on the antigen. In addition to their specificity, the monoclonal antibodies are advantageous in that they are synthesized by the hybridoma culture, uncontaminated by other immunoglobulins. The modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by the hybridoma method first described by Kohler et al., Nature, 256:495 (1975), or may be made by recombinant DNA methods (see, e.g., U.S. Patent No. 4,816,567). The "monoclonal antibodies" may also be isolated from phage antibody libraries using the techniques described in Clackson et al., Nature, 352:624-628 (1991) and Marks et al., J. Mol. Biol., 222:581-597 (1991), for example.

The monoclonal antibodies herein specifically include "chimeric" antibodies (immunoglobulins) in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity (U.S. Patent No. 4,816,567; Morrison et al., Proc. Natl. Acad. Sci. USA, 81:6851-6855 (1984)). Methods of making chimeric antibodies are known in the art.

"Humanized" forms of non-human (e.g., murine) antibodies are chimeric immunoglobulins, immunoglobulin chains or fragments thereof (such as Fv, Fab, Fab', F(ab')2 or other antigen-binding subsequences of antibodies) which contain minimal sequence derived from non-human immunoglobulin. For the most part, humanized antibodies are human immunoglobulins (recipient antibody) in which residues from a complementarity-determining region (CDR) of the recipient are replaced by residues from a CDR of a non-human species (donor antibody) such as mouse, rat or rabbit having the desired specificity, affinity, and capacity. In some instances, Fv framework region (FR) residues of the human immunoglobulin are replaced by corresponding non-human residues. Furthermore, humanized antibodies may comprise residues which are found neither in the recipient antibody nor in the imported CDR or framework sequences. These modifications are made to further refine and maximize antibody performance. In general, the humanized antibody will comprise substantially all of at least one, and typically two, variable domains, in which all or substantially all of the hypervariable loops correspond to those of a non-human immunoglobulin and all or substantially all of the FR regions are those of a human immunoglobulin sequence although the FR regions may include one or more amino acid substitutions that improve binding affinity. The number of these amino acid substitutions in the FR are typically no more than 6 in the H chain, and in the L chain, no more than 3. The humanized antibody optimally also will comprise at least a portion of an immunoglobulin constant region (Fc), typically that of a human immunoglobulin. For further details, see Jones et al., Nature, 321:522-525 (1986); Reichmann et al., Nature, 332:323-329 (1988); and Presta, Curr. Op. Struct. Biol., 2:593-596 (1992). The humanized antibody includes a PRIMATIZED^{®} antibody wherein the antigen-binding region of the antibody is derived from an antibody produced by, e.g., immunizing macaque monkeys with the antigen of interest. Methods of making humanized antibodies are known in the art.

Human antibodies can also be produced using various techniques known in the art, including phage-display libraries. Hoogenboom and Winter, J. Mol. Biol., 227:381 (1991); Marks et al., J. Mol. Biol., 222:581 (1991). The techniques of Cole et al. and Boerner et al. are also available for the preparation of human monoclonal antibodies. Cole et al., Monoclonal Antibodies and Cancer Therapy, Alan R. Liss, p. 77 (1985); Boerner et al., J. Immunol., 147(1):86-95 (1991).

"Functional fragments" of the CD20 binding antibodies of the invention are those fragments that retain binding to CD20 with substantially the same affinity as the intact full chain molecule from which they are derived and are able to deplete B cells as measured by in vitro or in vivo assays such as those described herein.
Antibody "effector functions" refer to those biological activities attributable to the Fc region (a native sequence Fc region or amino acid sequence variant Fc region) of an antibody, and vary with the antibody isotype. Examples of antibody effector functions include: C1q binding and complement dependent cytotoxicity; Fc receptor binding; antibody-dependent cell-mediated cytotoxicity (ADCC); phagocytosis; down regulation of cell surface receptors (*e.g*. B cell receptor); and B cell activation.

"Antibody-dependent cell-mediated cytotoxicity" or "ADCC" refers to a form of cytotoxicity in which secreted Ig bound onto Fc receptors (FcRs) present on certain cytotoxic cells (*e.g.* Natural Killer (NK) cells, neutrophils, and macrophages) enable these cytotoxic effector cells to bind specifically to an antigen-bearing target cell and subsequently kill the target cell with cytotoxins. The antibodies "arm" the cytotoxic cells and are absolutely required for such killing. The primary cells for mediating ADCC, NK cells, express FcγRIII only, whereas monocytes express FcγRI, FcγRII and FcγRIII. FcR expression on hematopoietic cells is summarized in Table 3 on page 464 of Ravetch and Kinet, Annu. Rev. Immunol 9:457-92 (1991). To assess ADCC activity of a molecule of interest, an *in vitro* ADCC assay, such as that described in US Patent No. 5,500,362 or 5,821,337 may be performed. Useful effector cells for such assays include peripheral blood mononuclear cells (PBMC) and Natural Killer (NK) cells. Alternatively, or additionally, ADCC activity of the molecule of interest may be assessed *in vivo*, e*.*g*.*, in a animal model such as that disclosed in Clynes et al. PNAS (USA) 95:652-656 (1998).

"Complement dependent cytotoxicity" or "CDC" refers to the lysis of a target cell in the presence of complement. Activation of the classical complement pathway is initiated by the binding of the first component of the complement system (C1q) to antibodies (of the appropriate subclass) which are bound to their cognate antigen. To assess complement activation, a CDC assay, *e.g.* as described in Gazzano-Santoro et al., J. Immunol. Methods 202:163 (1996), may be performed.

An "isolated" antibody is one which has been identified and separated and/or recovered from a component of its natural environment. Contaminant components of its natural environment are materials which would interfere with diagnostic or therapeutic uses for the antibody, and may include enzymes, hormones, and other proteinaceous or nonproteinaceous solutes. In preferred embodiments, the antibody will be purified (1) to greater than 95% by weight of antibody as determined by the Lowry method, and most preferably more than 99% by weight, (2) to a degree sufficient to obtain at least 15 residues of N-terminal or internal amino acid sequence by use of a spinning cup sequenator, or (3) to homogeneity by SDS-PAGE under reducing or nonreducing conditions using Coomassie blue or, preferably, silver stain. Isolated antibody includes the antibody *in situ* within recombinant cells since at least one component of the antibody's natural environment will not be present. Ordinarily, however, isolated antibody will be prepared by at least one purification step.

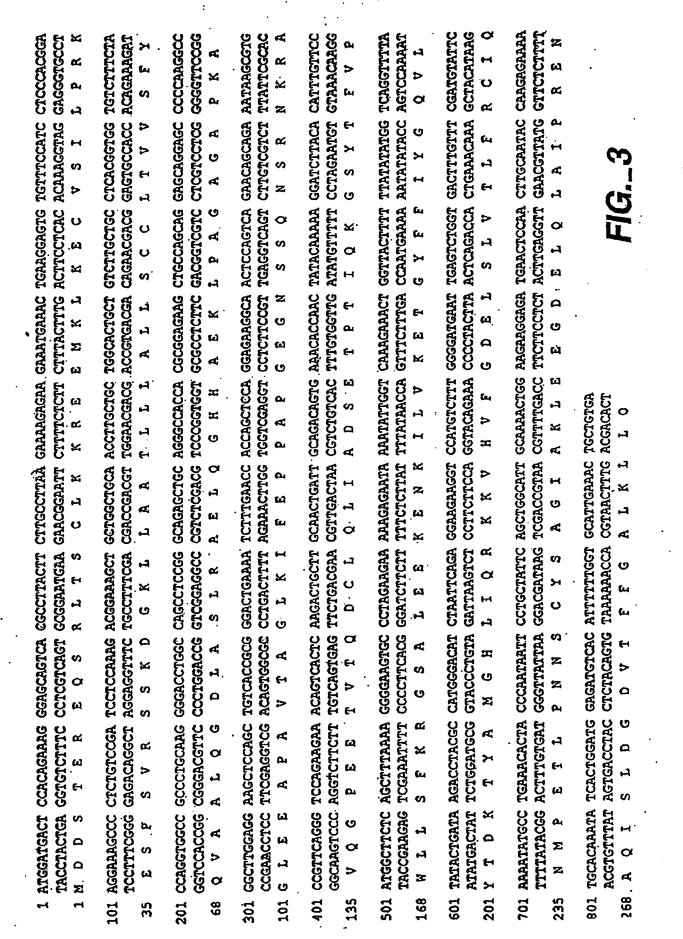

The terms "BLyS," "BLyS polypeptide," "TALL-1" or "TALL-1 polypeptide," "BAFF" when used herein encompass "native sequence BLyS polypeptides" and "BLyS variants". "BLyS" is a designation given to those polypeptides which are encoded by any one of the amino acid sequences shown below:
Human BLyS sequence (Fig. 3; SEQ ID NO._) 
1 mddstereqs rltsclkkre emklkecvsi lprkespsvr sskdgkllaa tlllallscc
61 ltvvsfyqva alqgdlaslr aelqghhaek lpagagapka gleeapavta glkifeppap
121 gegnssqnsr nkravqgpee tvtqdclqli adsetptiqk gsytfvpwll sfkrgsalee
181 kenkilvket gyffiygqvl ytdktyamgh liqrkkvhvf gdelslvtlf rciqnmpetl
241 pnnscysagi akleegdelq laiprenaqi sldgdvtffg alkll
Mouse BLyS sequence (SEQ ID NO._) 
1 mdesaktlpp pclcfcsekg edmkvgydpi tpqkeegawf gicrdgrlla atlllallss
61 sftamslyql aalqadlmnl rmelqsyrgs atpaaagape ltagvklltp aaprphnssr
121 ghrnrrafqg peeteqdvdl sappapclpg crhsqhddng mnlrniiqdc lqliadsdtp
181 tirkgtytfv pwllsfkrgn aleekenkiv vrqtgyffiy sqvlytdpif amghviqrkk
241 vhvfgdelsl vtlfrciqnm pktlpnnscy sagiarleeg deiqlaipre naqisrngdd.
301 tffgalkll
and in Figure 3 and homologs and fragments and variants thereof, which have the biological activity of the native sequence BLyS. A biological activity of BLyS can be selected from the group consisting of promoting B cell survival, promoting B cell maturation and binding to BR3. Variants of BLyS will preferably have at least 80% or any successive integer up to 100% including, more preferably, at least 90%, and even more preferably, at least 95% amino acid sequence identity with a native sequence of a BLyS polypeptide. A "native sequence" BLyS polypeptide comprises a polypeptide having the same amino acid sequence as the corresponding BLyS polypeptide derived from nature. For example, BLyS, exists in a soluble form following cleavage from the cell surface by furin-type proteases. Such native sequence BLYS polypeptides can be isolated from nature or can be produced by recombinant and/or synthetic means. The term "native sequence BLyS polypeptide" specifically encompasses naturally-occurring truncated or secreted forms (e.g., an extracellular domain sequence), naturally-occurring variant forms (e.g., alternatively spliced forms) and naturally-occurring allelic variants of the polypeptide. The term "BLyS" includes those polypeptides described in Shu et al., J. Leukocyte Biol., 65:680 (1999); GenBank Accession No. AF136293; WO98/18921 published May 7, 1998; EP 869,180 published October 7, 1998; WO98/27114 published June 25, 1998; WO99/12964 published March 18, 1999; WO99/33980 published July 8, 1999; Moore et al., Science, 285:260-263 (1999); Schneider et al., J. Exp. Med., 189:1747-1756 (1999); Mukhopadhyay et al., J. Biol. Chem., 274:15978-15981 (1999).

The term "BLyS antagonist" as used herein is used in the broadest sense, and includes any molecule that (1) binds a native sequence BLyS polypeptide or binds a native sequence BR3 polypeptide to partially or fully block BR3 interaction with BLyS polypeptide, and (2) partially or fully blocks, inhibits, or neutralizes native sequence BLyS signaling. Native sequence BLyS polypeptide signaling promotes, among other things, B cell survival and B cell maturation. The inhibition, blockage or neutralization of BLyS signaling results in, among other things, a reduction in the number of B cells. A BLyS antagonist according to this invention will partially or fully block, inhibit, or neutralize one or more biological activities of a BLyS polypeptide, *in vitro* or *in vivo.* A biologically active BLyS may potentiates any one or combination of the following events *in vitro* or *in vivo*: an increased survival of B cells, an increased level of IgG and/or IgM, an increased numbers of plasma cells, and processing of NF-κb2/100 to p52 NF-κb in splenic B cells (e.g., Batten, M et al., (2000) J. Exp. Med. 192:1453-1465; Moore, et al., (1999) Science 285:260-263; Kayagaki, et al., (2002) 10:515-524). Several assays useful for testing BLyS antagonists according to this invention are described herein.

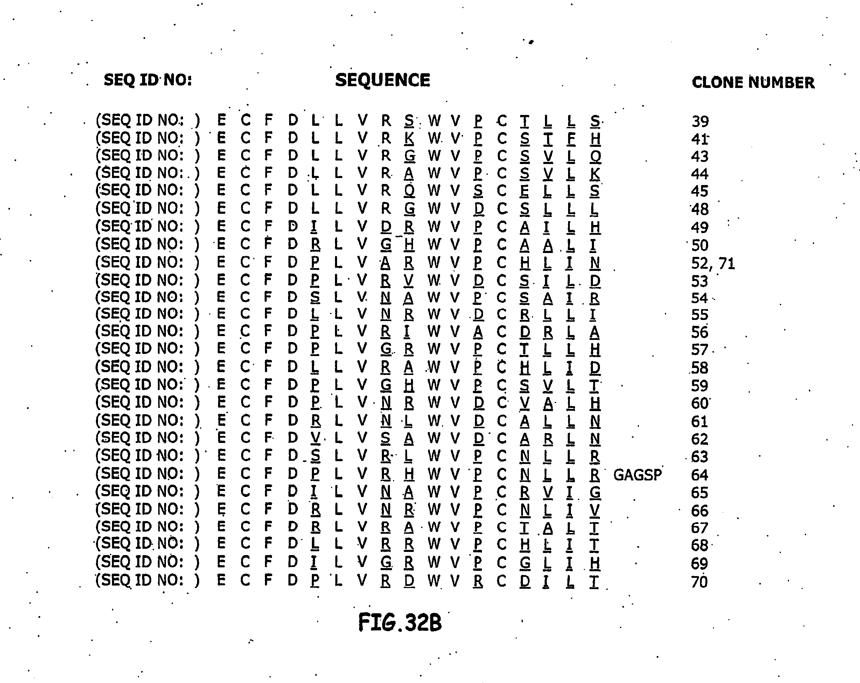

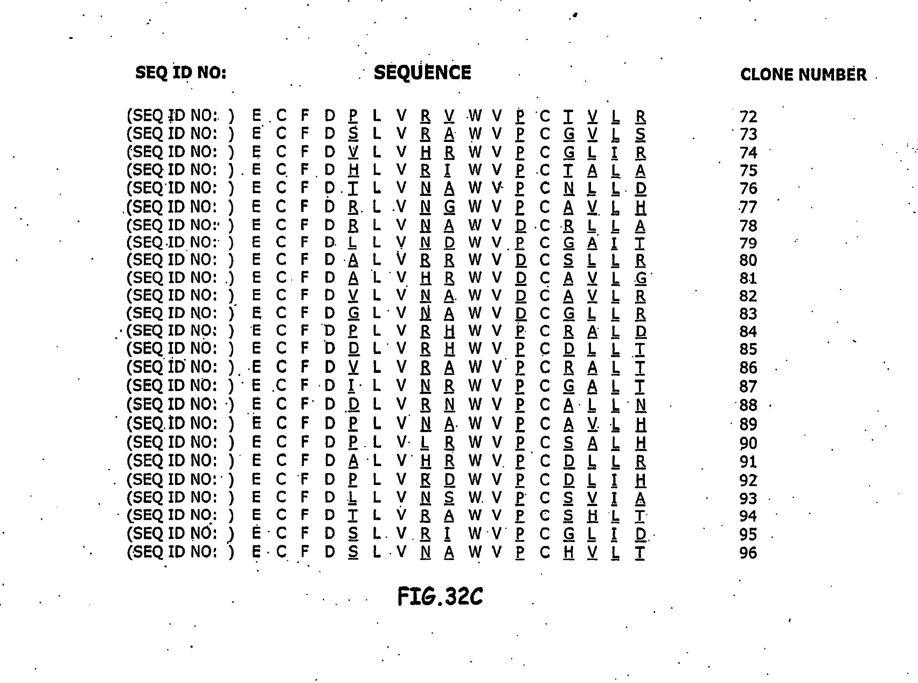

As mentioned above, a BLyS antagonist can function in a direct or indirect manner to partially or fully block, inhibit or neutralize BLyS signaling, *in vitro* or *in vivo.* For instance, the BLyS antagonist can directly bind BLyS. For example, anti-BLyS antibodies that bind within a region of human BLyS comprising residues 162-275 and/or a neighboring residue of a residue selected from the group consisting of 162, 163, 206, 211, 231, 233, 264 and 265 of human BLyS such that the antibody sterically hinders BLyS binding to BR3 is contemplated. In another example, a direct binder is a polypeptide comprising the extracellular domain of a BLyS receptor such as TACI, BR3 and BCMA. In another example, BLyS antagonists include the polypeptides having a sequence of that of Formula I, Formula II, Formula III, ECFDLLVRAWVPCSVLK (SEQ ID NO 5), ECFDLLVRHWVPCGLLR (SEQ ID NO 6), ECFDLLVRRWVPCEMLG (SEQ ID NO 7), ECFDLLVRSWVPCHMLR (SEQ ID NO 8), ECFDLLVRHWVACGLLR (SEQ ID NO 9), or sequences listed in FIG. 32, as described herein. Alternatively, the BLyS antagonist can bind an extracellular domain of a native sequence BR3 at its BLyS binding region to partially or fully block, inhibit or neutralize BLyS binding to BR3 *in vitro*, *in situ*, or *in vivo.* For example, such indirect antagonist is an anti-BR3 antibody that binds in a region of BR3 comprising residues 23-38 of human BR3 or a neighboring region of those residues such that binding of human BR3 to BLyS is sterically hindered.

A BLyS antagonist may include anti-BLyS antibodies, immunoadhesins and small molecules. The immunoadhesin may comprise a BLyS binding region of a BLyS receptor (e.g., an extracellular domain of BR3, BCMA or TACI). The immunoadhesin may be BR3-Fc, or polypeptides having a sequence of that of Formula I, Formula II, Formula III, ECFDLLVRAWVPCSVLK (SEQ ID NO 5), ECFDLLVRHWVPCGLLR (SEQ ID NO 6), ECFDLLVRRWVPCEMLG (SEQ ID NO 7), ECFDLLVRSWVPCHMLR (SEQ ID NO 8), ECFDLLVRHWVACGLLR (SEQ ID NO 9), or sequences listed in FIG.32, optionally, fused or conjugated to an Fc portion of an immunoglobulin.

The BLyS antagonist may bind to a BLyS polypeptide or a BR3 polypeptide with a binding affinity of 100nM or less. The BLyS antagonist may bind to a BLyS polypeptide or a BR3 polypeptide with a binding affinity of 10nM or less. The BlyS antagonist may bind to a BLyS polypeptide or a BR3 polypeptide with a binding affinity of 1nM or less

The terms "BR3", "BR3 polypeptide" or "BR3 receptor" when used herein encompass "native sequence BR3 polypeptides" and "BR3 variants" (which are further defined herein). "BR3" is a designation given to those polypeptides comprising any one of the following polynucleotide sequences and homologs thereof: 
(a) human BR3 sequence (SEQ ID NO:__)
1 MRRGPRSLRG RDAPAPTPCV PAECFDLLVR HCVACGLLRT PRPKPAGASS PAPRTALQPQ
61 ESVGAGAGEA ALPLPGLLFG APALLGLALV LALVLVGLVS WRRRQRRLRG ASSAEAPDGD
121 KDAPEPLDKV IILSPGISDA TAPAWPPPGE DPGTTPPGHS VPVPATELGS TELVTTKTAG
181 PEQQ

(b) alternative human BR3 sequence (SEQ ID NO:___)
1 MRRGPRSLRG RDAPAPTPCV PAECFDLLVR HCVACGLLRT PRPKPAGAAS SPAPRTALQP
61 QESVGAGAGE AALPLPGLLF GAPALLGLAL VLALVLVGLV SWRRRQRRLR GASSAEAPDG
121 DKDAPEPLDK VIILSPGISD ATAPAWPPPG EDPGTTPPGH SVPVPATELG STELVTTKTA
181 GPEQQ

(c) murine BR3 sequence (SEQ ID NO:__)
1 MGARRLRVRS QRSRDSSVPT QCNQTECFDP LVRNCVSCEL FHTPDTGHTS SLEPGTALQP
61 QEGSALRPDV ALLVGAPALL GLILALTLVG LVSLVSWRWR QQLRTASPDT SEGVQQESLE
121 NVFVPSSETP HASAPTWPPL KEDADSALPR HSVPVPATEL GSTELVTTKT AGPEQ

(d) rat BR3 sequence (SEQ ID NO:___)
1 MGVRRLRVRS RRSRDSPVST QCNQTECFDP LVRNCVSCEL FYTPETRHAS SLEPGTALQP
61 QEGSGLRPDV ALLFGAPALL GLVLALTLVG LVSLVGWRWR QQRRTASLDT SEGVQQESLE
121 NVFVPPSETL HASAPNWPPF KEDADNILSC HSIPVPATEL GSTELVTTKT AGPEQ
 and variants or fragments thereof. The BR3 polypeptides can be isolated from a variety of sources, such as from human tissue types or from another source, or prepared by recombinant and/or synthetic methods. The term BR3, includes the BR3 polypeptides described in WO 02/24909 and WO 03/14294.

A "native sequence" BR3 polypeptide comprises a polypeptide having the same amino acid sequence as the corresponding BR3 polypeptide derived from nature. Such native sequence BR3 polypeptides can be isolated from nature or can be produced by recombinant and/or synthetic means. The term "native sequence BR3 polypeptide" specifically encompasses naturally-occurring truncated, soluble or secreted forms (e.g., an extracellular domain sequence), naturally-occurring variant forms (e.g., alternatively spliced forms) and naturally-occurring allelic variants of the polypeptide. The BR3 polypeptides of the invention include the BR3 polypeptide comprising or consisting of the contiguous sequence of amino acid residues 1 to 184 of a human BR3.

A BR3 "extracellular domain" or "ECD" refers to a form of the BR3 polypeptide which is essentially free of the transmembrane and cytoplasmic domains. ECD forms of BR3 include those comprising any one of amino acids 1 to 77, 2 to 62, 2-71, 1-61 and 2-63 of BR3.

Mini-BR3 is a 26-residue core region of the BLyS-binding domain of BR3:
TPCVPAECFD LLVRHCVACG LLRTPR (SEQ. ID:_)

"BR3 variant" means a BR3 polypeptide having at least about 80% amino acid sequence identity with the amino acid sequence of a native sequence full length BR3 or BR3 ECD and binds a native sequence BlyS polypeptide. Optionally, the BR3 variant includes a single cysteine rich domain. Such BR3 variant polypeptides include, for instance, BR3 polypeptides wherein one or more amino acid residues are added, or deleted, at the N- and/or C-terminus, as well as within one or more internal domains, of the full-length amino acid sequence. Fragments of the BR3 ECD that bind a native sequence BlyS polypeptide are also contemplated. Ordinarily, a BR3 variant polypeptide will have at least about 80% amino acid sequence identity, more preferably at least about 81 % amino acid sequence identity, more preferably at least about 82% amino acid sequence identity, more preferably at least about 83% amino acid sequence identity, more preferably at least about 84% amino acid sequence identity, more preferably at least about 85% amino acid sequence identity, more preferably at least about 86% amino acid sequence identity, more preferably at least about 87% amino acid sequence identity, more preferably at least about 88% amino acid sequence identity, more preferably at least about 89% amino acid sequence identity, more preferably at least about 90% amino acid sequence identity, more preferably at least about 91% amino acid sequence identity, more preferably at least about 92% amino acid sequence identity, more preferably at least about 93% amino acid sequence identity, more preferably at least about 94% amino acid sequence identity, more preferably at least about 95% amino acid sequence identity, more preferably at least about 96% amino acid sequence identity, more preferably at least about 97% amino acid sequence identity, more preferably at least about 98% amino acid sequence identity and yet more preferably at least about 99% amino acid sequence identity with a human BR3 polypeptide or a specified fragment thereof. BR3 variant polypeptides do not encompass the native BR3 polypeptide sequence. Ordinarily, BR3 variant polypeptides are at least about 10 amino acids in length, often at least about 20 amino acids in length, more often at least about 30 amino acids in length, more often at least about 40 amino acids in length, more often at least about 50 amino acids in length, more often at least about 60 amino acids in length, more often at least about 70 amino acids in length, more often at least about 80 amino acids in length, more often at least about 90 amino acids in length, more often at-least about 100 amino acids in length, more often at least about 150 amino acids in length, more often at least about 200 amino acids in length, more often at least about 250 amino acids in length, more often at least about 300 amino acids in length, or more.

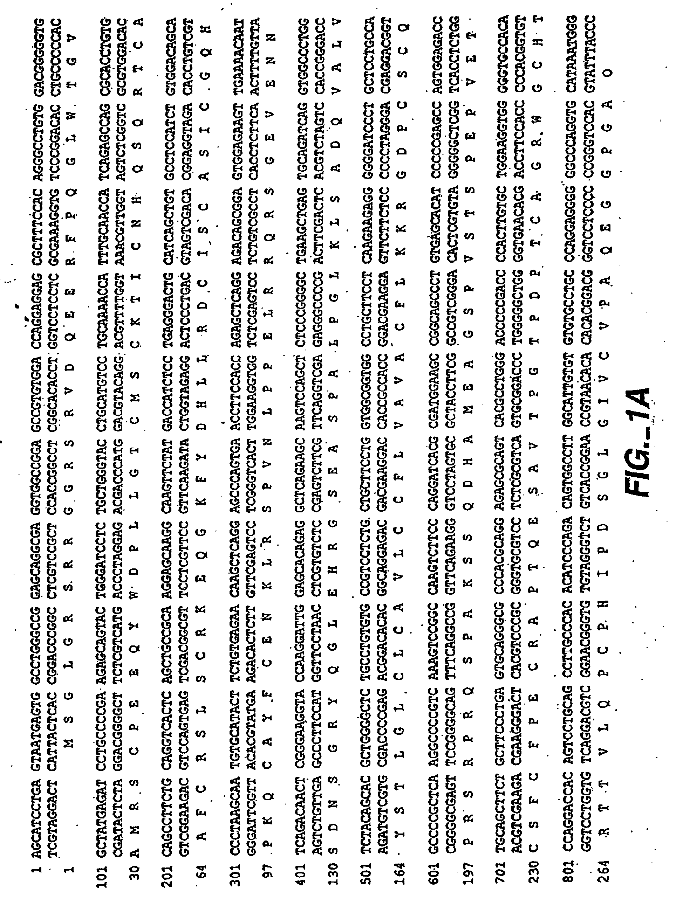

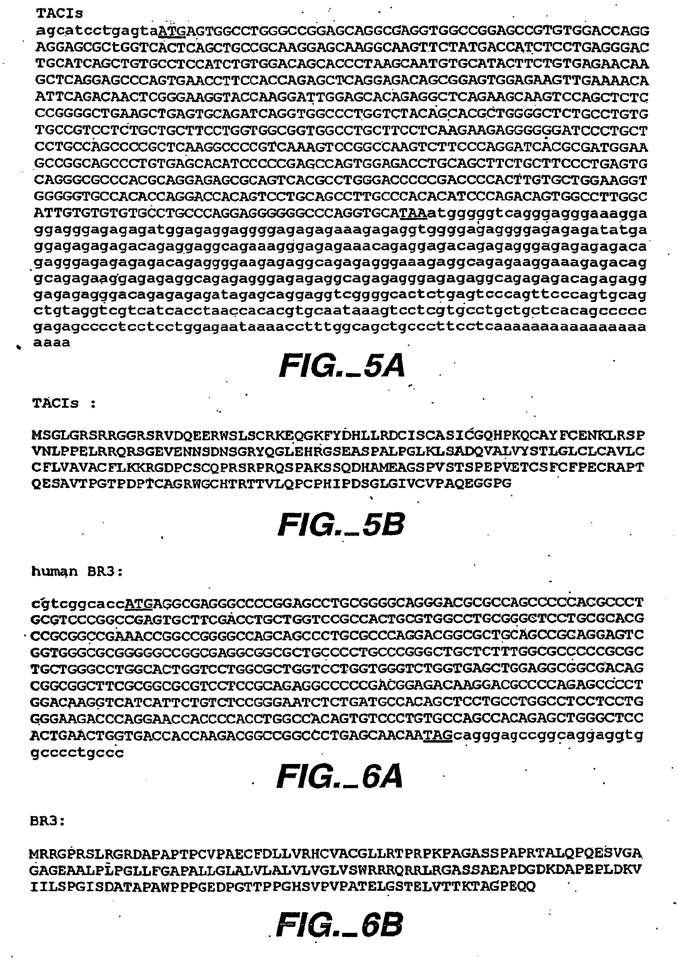

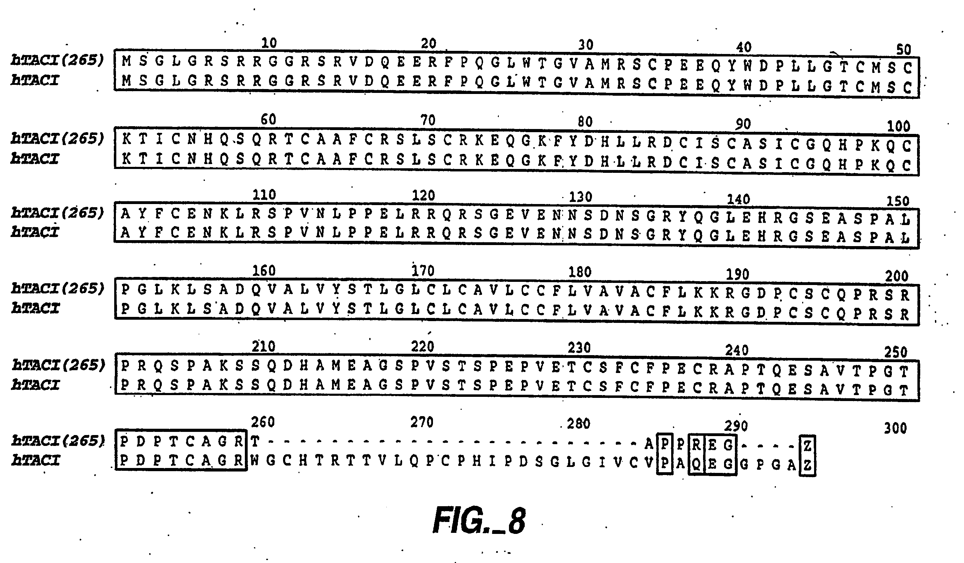

The terms "TACI" or "TACI polypeptide" or "TACI receptor" when used herein encompass "native sequence TACI polypeptides" and "TACI variants" (which are further defined herein). "TACI" is a designation given to those polypeptides comprising the amino acid sequences of Figures 1A-1B, amino acids 1-246 of Figure 5B and the amino acid sequences of Figure 8, polypeptides which are encoded by nucleic acid molecules comprising the polynucleotide sequence shown in Figures 1A-1B and 5A and homologs, variants and fragments thereof, nucleic acid molecules comprising the sequence shown in the Figures 1A-1B and 5A and variants thereof as well as fragments of the above. The TACI polypeptides of the invention can be isolated from a variety of sources, such as from human tissue types or from another source, or prepared by recombinant and/or synthetic methods.

A "native sequence" TACI polypeptide comprises a polypeptide having the same amino acid sequence as the corresponding TACI polypeptide derived from nature. Such native sequence TACI polypeptide can be isolated from nature or can be produced by recombinant and/or synthetic means. The term "native sequence TACI polypeptide" specifically encompasses naturally-occurring truncated, soluble or secreted forms (e.g., an extracellular domain sequence), naturally-occurring variant forms (e.g., alternatively spliced forms) and naturally-occurring allelic variants of the polypeptide. The TACI polypeptides of the invention include but are not limited to the polypeptides described in von Bulow et al., supra and WO98/39361 published September 11, 1998, the spliced variant (referred to as "hTACI(265)" above and shown in Figure 8, the TACI polypeptide comprising the contiguous sequence of amino acid residues 1-293 of Figure 8).

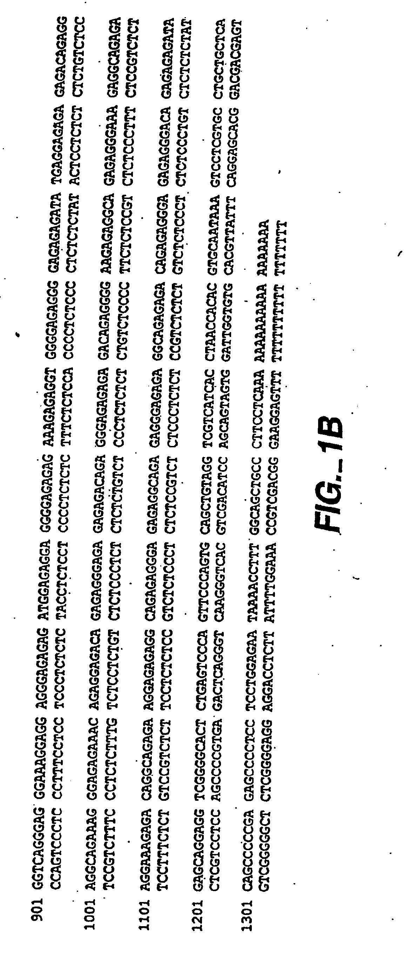

A TACI "extracellular domain" or "ECD" refers to a form of the TACI polypeptide which is essentially free of the transmembrane and cytoplasmic domains. ECD forms of TACI include those described in von Bulow et al., supra, WO 98/39361, WO 00/40716, WO 01/85782, WO 01/87979, WO 01/81417, amino acid residues 1-166 of Figure 1, amino acid residues 1-165 of Figure 1, amino acid residues 1-154 of Figure 1, amino acid residues 1-114 of Figure 1, amino acid residues 1-119 of Figure 5B, amino acid residues 1-120 of Figure 5B, and amino acid residues 1-126 of Figure 5B.

"TACI variant" means a TACI polypeptide having at least about 80% amino acid sequence identity with the amino acid sequence of a native sequence full length TACI or TACI ECD and binds a native sequence BlyS polypeptide. Such TACI variant polypeptides include, for instance, TACI polypeptides wherein one or more amino acid residues are added, or deleted, at the N- and/or C-terminus, as well as within one or more internal domains, of the full-length amino acid sequence. Fragments of the TACI ECD that bind a native sequence BlyS polypeptide are also contemplated. Ordinarily, a TACI variant polypeptide will have at least about 80% amino acid sequence identity, more preferably at least about 81% amino acid sequence identity, more preferably at least about 82% amino acid sequence identity, more preferably at least about 83% amino acid sequence identity, more preferably at least about 84% amino acid sequence identity, more preferably at least about 85% amino acid sequence identity, more preferably at least about 86% amino acid sequence identity, more preferably at least about 87% amino acid sequence identity, more preferably at least about 88% amino acid sequence identity, more preferably at least about 89% amino acid sequence identity, more preferably at least about 90% amino acid sequence identity, more preferably at least about 91% amino acid sequence identity, more preferably at least about 92% amino acid sequence identity, more preferably at least about 93% amino acid sequence identity, more preferably at least about 94% amino acid sequence identity, more preferably at least about 95% amino acid sequence identity, more preferably at least about 96% amino acid sequence identity, more preferably at least about 97% amino acid sequence identity, more preferably at least about 98% amino acid sequence identity and yet more preferably at least about 99% amino acid sequence identity with a TACI polypeptide encoded by a nucleic acid molecule shown in Figure 1A or a specified fragment thereof. TACI variant polypeptides do not encompass the native TACI polypeptide sequence. Ordinarily, TACI variant polypeptides are at least about 10 amino acids in length, often at least about 20 amino acids in length, more often at least about 30 amino acids in length, more often at least about 40 amino acids in length, more often at least about 50 amino acids in length, more often at least about 60 amino acids in length, more often at least about 70 amino acids in length, more often at least about 80 amino acids in length, more often at least about 90 amino acids in length, more often at least about 100 amino acids in length, more often at least about 150 amino acids in length, more often at least about 200 amino acids in length, more often at least about 250 amino acids in length, more often at least about 300 amino acids in length, or more.

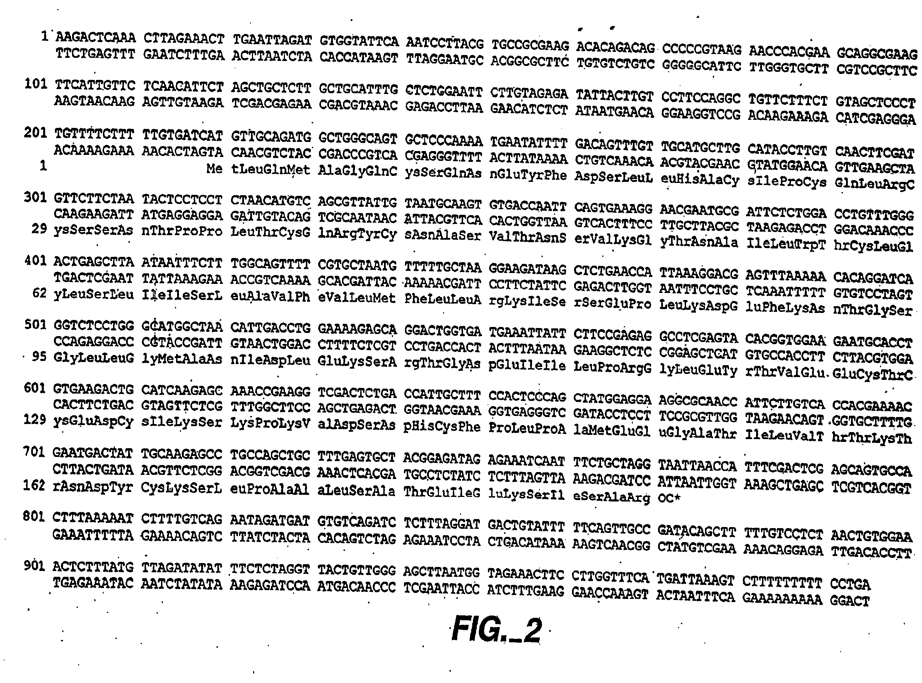

The terms "BCMA" or "BCMA polypeptide" or "BCMA receptor" when used herein encompass "native sequence BCMA polypeptides" and "BCMA variants" (which are further defined herein). "BCMA" is a designation given to those polypeptides which are encoded by the nucleic acid molecules comprising the polynucleotide sequences shown in Figure 2 and variants thereof, nucleic acid molecules comprising the sequence shown in the Figure 2 and variants thereof as well as fragments of the above. The BCMA polypeptides can be isolated from a variety of sources, such as from human tissue types or from another source, or prepared by recombinant and/or synthetic methods.

A "native sequence" BCMA polypeptide comprises a polypeptide having the same amino acid sequence as the corresponding BCMA polypeptide derived from nature. Such native sequence BCMA polypeptides can be isolated from nature or can be produced by recombinant and/or synthetic means. The term "native sequence BCMA polypeptide" specifically encompasses naturally-occurring truncated or secreted forms (e.g., an extracellular domain sequence), naturally-occurring variant forms (e.g., alternatively spliced forms) and naturally-occurring allelic variants of the polypeptide. BCMA polypeptides include the polypeptides described in Laabi et al., EMBO J., 11:3897-3904 (1992); Laabi et al., Nucleic Acids Res., 22:1147-1154 (1994); Gras et al., Int. Immunology, 7:1093-1106 (1995); Madry et al., Int. Immunology, 10:1693-1702 (1998); and the BCMA polypeptide comprising the contiguous sequence of amino acid residues 1-184 of Fig. 2 (SEQ ID NO:4).

A BCMA "extracellular domain" or "ECD" refers to a form of the BCMA polypeptide which is essentially free of the transmembrane and cytoplasmic domains. ECD forms of TACI include those described in Laabi et al., EMBO J., 11:3897-3904 (1992); Laabi et al., Nucleic Acids Res., 22:1147-1154 (1994); Gras et al., Int. Immunology, 7:1093-1106 (1995); Madry et al., Int. Immunology, 10:1693-1702 (1998), amino acid residues 4-55 of Figure 2, amino acid residues 1-48 of Figure 2, amino acid residues 8-41 of Figure 2, amino acid residues 4-51 of Figure 2 or amino acid residues 21-53 of Figure 2.

"BCMA variant" means a BCMA polypeptide having at least about 80% amino acid sequence identity with the amino acid sequence of a native sequence BCMA or BCMA ECD and binds a native sequence BlyS polypeptide. Such BCMA variant polypeptides include, for instance, BCMA polypeptides wherein one or more amino acid residues are added, or deleted, at the N- and/or C-terminus, as well as within one or more internal domains, of the full-length amino acid sequence. Fragments of the BCMA ECD that bind a native sequence BlyS polypeptide are also contemplated. Ordinarily, a BCMA variant polypeptide will have at least about 80% amino acid sequence identity, more preferably at least about 81% amino acid sequence identity, more preferably at least about 82% amino acid sequence identity, more preferably at least about 83% amino acid sequence identity, more preferably at least about 84% amino acid sequence identity, more preferably at least about 85% amino acid sequence identity, more preferably at least about 86% amino acid sequence identity, more preferably at least about 87% amino acid sequence identity, more preferably at least about 88% amino acid sequence identity, more preferably at least about 89% amino acid sequence identity, more preferably at least about 90% amino acid sequence identity, more preferably at least about 91% amino acid sequence identity, more preferably at least about 92% amino acid sequence identity, more preferably at least about 93% amino acid sequence identity, more preferably at least about 94% amino acid sequence identity, more preferably at least about 95% amino acid sequence identity, more preferably at least about 96% amino acid sequence identity, more preferably at least about 97% amino acid sequence identity, more preferably at least about 98% amino acid sequence identity and yet more preferably at least about 99% amino acid sequence identity with a BCMA polypeptide encoded by a nucleic acid molecule shown in Figure 2 or a specified fragment thereof. BCMA variant polypeptides do not encompass the native BCMA polypeptide sequence. Ordinarily, BCMA variant polypeptides are at least about 10 amino acids in length, often at least about 20 amino acids in length, more often at least about 30 amino acids in length, more often at least about 40 amino acids in length, more often at least about 50 amino acids in length, more often at least about 60 amino acids in length, more often at least about 70 amino acids in length, more often at least about 80 amino acids in length, more often at least about 90 amino acids in length, more often at least about 100 amino acids in length, more often at least about 150 amino acids in length, more often at least about 200 amino acids in length, more often at least about 250 amino acids in length, more often at least about 300 amino acids in length, or more.

BAFF is expressed in spleen, lymph nodes, PBLs, monocytes, macrophages, DCs, T cells, K562, HL-60 and G361. APRIL is weakly expressed in spleen, pancreas, colon. APRIL is expressed in PBLs and various tumor cell lines and tissues. BCMA is expressed in spleen, lymph nodes, thymus, PBLs, liver, adrenals and B cells. TACI is expressed in spleen, thymus, PBLs, small intestine, B cells and activated T cells. BAFF-R is expressed in spleen, lymph nodes, thymus, PBLs, B cells. BAFF-R is expressed in low levels on resting T cells. (Mackay and Browning, July 2002, Nature Reviews, Immunology, 2: 465-475).

Amino acids may be grouped according to similarities in the properties of their side chains (in A. L. Lehninger, in Biochemistry, second ed., pp. 73-75, Worth Publishers, New York (1975)):
(1) non-polar: Ala (A), Val (V), Leu (L), Ile (I), Pro (P), Phe (F), Trp (W), Met (M)
(2) uncharged polar: Gly (G), Ser (S), Thr (T), Cys (C), Tyr (Y), Asn (N), Gln (Q)
(3) acidic: Asp (D), Glu (E)
(4) basic: Lys (K), Arg (R), His(H)

Alternatively, naturally occurring residues may be divided into groups based on common side-chain properties:
(1) hydrophobic: Norleucine, Met, Ala, Val, Leu, Ile;
(2) neutral hydrophilic: Cys, Ser, Thr, Asn, Gln;
(3) acidic: Asp, Glu;
(4) basic: His, Lys, Arg;
(5) residues that influence chain orientation: Gly, Pro;
(6) aromatic: Trp, Tyr, Phe.

The term "conservative" amino acid substitution as used within this invention is meant to refer to amino acid substitutions which substitute functionally equivalent amino acids. Conservative amino acid changes result in silent changes in the amino acid sequence of the resulting peptide. For example, one or more amino acids of a similar polarity act as functional equivalents and result in a silent alteration within the amino acid sequence of the peptide. In general, substitutions within a group may be considered conservative with respect to structure and function. However, the skilled artisan will recognize that the role of a particular residue is determined by its context within the three-dimensional structure of the molecule in which it occurs. For example, Cys residues may occur in the oxidized (disulfide) form, which is less polar than the reduced (thiol) form. The long aliphatic portion of the Arg side chain may constitute a critical feature of its structural or functional role, and this may be best conserved by substitution of a nonpolar, rather than another basic residue. Also, it will be recognized that side chains containing aromatic groups (Trp, Tyr, and Phe) can participate in ionic-aromatic or "cation-pi" interactions. In these cases, substitution of one of these side chains with a member of the acidic or uncharged polar group may be conservative with respect to structure and function. Residues such as Pro, Gly, and Cys (disulfide form) can have direct effects on the main chain conformation, and often may not be substituted without structural distortions.

"Percent (%) amino acid sequence identity" with respect to the ligand or receptor polypeptide sequences identified herein is defined as the percentage of amino acid residues in a candidate sequence that are identical with the amino acid residues in such a ligand or receptor sequence identified herein, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN, ALIGN-2 or Megalign (DNASTAR) software. Those skilled in the art can determine appropriate parameters for measuring alignment, including any algorithms needed to achieve maximal alignment over the full-length of the sequences being compared. For purposes herein, however, % amino acid sequence identity values are obtained as described below by using the sequence comparison computer program ALIGN-2, wherein the complete source code for the ALIGN-2 program is provided in the table below. The ALIGN-2 sequence comparison computer program was authored by Genentech, Inc. and the source code shown in the table below has been filed with user documentation in the U.S. Copyright Office, Washington D.C., 20559, where it is registered under U.S. Copyright Registration No. TXU510087. The ALIGN-2 program is publicly available through Genentech, Inc.; South San Francisco, California or can be compiled from the source code provided in the table below. The ALIGN-2 program should be compiled for use on a UNIX operating system, preferably digital UNIX V4.0D. All sequence comparison parameters are set by the ALIGN-2 program and do not vary.

A useful method for identification of certain residues or regions in a protein that are preferred locations for mutagenesis is called "alanine scanning mutagenesis" as described by Cunningham and Wells Science, 244:1081-1085 (1989). A residue or group of target residues are identified (*e.g.*, charged residues such as arg, asp, his, lys, and glu) and replaced by a neutral or negatively charged amino acid (most preferably alanine or polyalanine) to affect the interaction of the amino acids with a binding target. Those amino acid locations demonstrating functional sensitivity to the substitutions then are refined by introducing further or other variants at, or for, the sites of substitution. Thus, while the site for introducing an amino acid sequence variation is predetermined, the nature of the mutation *per se* need not be predetermined. For example, to analyze the performance of a mutation at a given site, ala scanning or random mutagenesis is conducted at the target codon or region and the expressed variants are screened for the desired activity.

The term, "dihedral angle" refers to a rotation about a bond. See e.g., Creighton, T.E., (1993) Protein: Structures and Molecular Properties, 2 ed., W. H. Freeman and Company, New York, NY.

The term, "phi," is a dihedral angle that denotes a rotation about the N-C^{α} bond of an amino acid. See e.g., Creighton, T.E., (1993) Protein:Structures and Molecular Properties, 2 ed., W. H. Freeman and Company, New York, NY.

Type I beta turns are described in Hutchinson, E. G. & Thornton, J. M. (1994) A revised set of potentials for beta turn formation in proteins. Protein Science 3, 2207-2216.

A "fusion protein" and a "fusion polypeptide" refer to a polypeptide having two portions covalently linked together, where each of the portions is a polypeptide having a different property. The property may be a biological property, such as activity *in vitro* or *in vivo.* The property may also be a simple chemical or physical property, such as binding to a target molecule, catalysis of a reaction, etc. The two portions may be linked directly by a single peptide bond or through a peptide linker containing one or more amino acid residues. Generally, the two portions and the linker will be in reading frame with each other.

A "conjugate" refers to any hybrid molecule, including fusion proteins and as well as molecules that contain both amino acid or protein portions and non-protein portions. Conjugates may be synthesized by a variety of techniques known in the art including, for example, recombinant DNA techniques, solid phase synthesis, solution phase synthesis, organic chemical synthetic techniques or a combination of these techniques. The choice of synthesis will depend upon the particular molecule to be generated. For example, a hybrid molecule not entirely "protein" in nature may be synthesized by a combination of recombinant techniques and solution phase techniques.

As used herein, the term "immunoadhesin" designates antibody-like molecules which combine the binding specificity of a heterologous protein (an "adhesin") with the effector functions of immunoglobulin constant domains. Structurally, the immunoadhesins comprise a fusion of an amino acid sequence with the desired binding specificity which is other than the antigen recognition and binding site of an antibody (i.e., is "heterologous"), and an immunoglobulin constant domain sequence. The adhesin part of an immunoadhesin molecule typically is a contiguous amino acid sequence comprising at least the binding site of a receptor or a ligand. The immunoglobulin constant domain sequence in the immunoadhesin can be obtained from any immunoglobulin, such as IgG-1, IgG-2, IgG-3, or IgG-4 subtypes, IgA (including IgA-1 and IgA-2), IgE, IgD or IgM. For example, useful immunoadhesins according to this invention are polypeptides that comprise the BLyS binding portions of a BLyS receptor without the transmembrane or cytoplasmic sequences of the BLyS receptor. In one embodiment, the extracellular domain of BR3, TACI or BCMA is fused to a constant domain of an immunoglobulin sequence. For example, a mouse BR3-Fc immunoadhesin and human BR3-Fc immunoadhesin according to this invention can be represented by the formulae:
mBR3-Fc (SEQ ID NO. 1): 
MSALLILALVGAAVASTGARRLRVRSQRSRDSSVPTQCNQTECFDPLVRNCVSCELFHTPDTGHTS
SLEPGTALQPQEGQVTGDKKIVPRDCGCKPCICTVPEVSSVFIFPPKPKDVLTITLTPKVTCVVVDIS
KDDPEVQFSWFVDDVEVHTAQTQPREEQFNSTFRSVSEL
PIMHQDWLNGKEFKCRVNSAAFPAPIEKTISKTKGRPKAPQVYTIPPPKEQMAKDKVSLTCMITDF
FPEDITVEWQWNGQPAENYKNTQPIMNTNGSYFVYSKLNVQKSNWEAGNTFTCSVLHEGLHNHH
TEKSLSHSPGK
hBR3-hIgG1 Fc (SEQ ID NO. 2)

MSALLILALVGAAVASTRRGPRSLRGRDAPAPTPCVPAECFDLLVRHCVACGLLRTPRPKPAGASS
PAPRTALQPQESQVTDKAAHYTLCPPCPAPELLGGPSVFLFPPKPKDTLMISRTPEVTCVVVAVSHE
DPEVKFNWYVDGVEVHNAKTKPREEQYNSTYRVVSVLTVLHQDWLNGKEYKCKVSNKALPAPI
EKTISKAKGQPREPQVYTLPPSREEMTKNQVSLTCLVKGFYPSDIAVEWESNGQPENNYKTTPPVL
DSDGSFFLYSKLTVDKSRWQQGNVFSCSVMHEALHNHYTQKSLSLSPGK

A "chemotherapeutic agent" is a chemical compound useful in the treatment of cancer. Examples of chemotherapeutic agents include alkylating agents such as thiotepa and cyclosphosphamide (CYTOXAN™); alkyl sulfonates such as busulfan, improsulfan and piposulfan; aziridines such as benzodopa, carboquone, meturedopa, and uredopa; ethylenimines and methylamelamines including altretamine, triethylenemelamine, trietylenephosphoramide, triethylenethiophosphaoramide and trimethylolomelamine; nitrogen mustards such as chlorambucil, chlornaphazine, cholophosphamide, estramustine, ifosfamide, mechlorethamine, mechlorethamine oxide hydrochloride, melphalan, novembichin, phenesterine, prednimustine, trofosfamide, uracil mustard; nitrosureas such as carmustine, chlorozotocin, fotemustine, lomustine, nimustine, ranimustine; antibiotics such as aclacinomysins, actinomycin, authramycin, azaserine, bleomycins, cactinomycin, calicheamicin, carabicin, carminomycin, carzinophilin, chromomycins, dactinomycin, daunorubicin, detorubicin, 6-diazo-5-oxo-L-norleucine, doxorubicin, epirubicin, esorubicin, idarubicin, marcellomycin, mitomycins, mycophenolic acid, nogalamycin, olivomycins, peplomycin, potfiromycin, puromycin, quelamycin, rodorubicin, streptonigrin, streptozocin, tubercidin, ubenimex, zinostatin, zorubicin; anti-metabolites such as methotrexate and 5-fluorouracil (5-FU); folic acid analogues such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine, 5-FU; androgens such as calusterone, dromostanolone propionate, epitiostanol, mepitiostane, testolactone; anti-adrenals such as aminoglutethimide, mitotane, trilostane; folic acid replenisher such as frolinic acid; aceglatone; aldophosphamide glycoside; aminolevulinic acid; amsacrine; bestrabucil; bisantrene; edatraxate; defofamine; demecolcine; diaziquone; elfomithine; elliptinium acetate; etoglucid; gallium nitrate; hydroxyurea; lentinan; lonidamine; mitoguazone; mitoxantrone; mopidamol; nitracrine; pentostatin; phenamet; pirarubicin; podophyllinic acid; 2-ethylhydrazide; procarbazine; PSK®; razoxane; sizofiran; spirogermanium; tenuazonic acid; triaziquone; 2, 2',2"-trichlorotriethylamine; urethan; vindesine; dacarbazine; mannomustine; mitobronitol; mitolactol; pipobroman; gacytosine; arabinoside ("Ara-C"); cyclophosphamide; thiotepa; taxoids, *e.g.* paclitaxel (TAXOL^{®}, Bristol-Myers Squibb Oncology, Princeton, NJ) and doxetaxel (TAXOTERE^{®}, Rhône-Poulenc Rorer, Antony, France); chlorambucil; gemcitabine; 6-thioguanine; mercaptopurine; methotrexate; platinum analogs such as cisplatin and carboplatin; vinblastine; platinum; etoposide (VP-16); ifosfamide; mitomycin C; mitoxantrone; vincristine; vinorelbine; navelbine; novantrone; teniposide; daunomycin; aminopterin; xeloda; ibandronate; CPT-11; topoisomerase inhibitor RFS 2000; difluoromethylornithine (DMFO); retinoic acid; esperamicins; capecitabine; and pharmaceutically acceptable salts, acids or derivatives of any of the above. Also included in this definition are anti-hormonal agents that act to regulate or inhibit hormone action on tumors such as anti-estrogens including for example tamoxifen, raloxifene, aromatase inhibiting 4(5)-imidazoles, 4-hydroxytamoxifen, trioxifene, keoxifene, LY117018, onapristone, and toremifene (Fareston); and anti-androgens such as flutamide, nilutamide, bicalutamide, leuprolide, and goserelin; and pharmaceutically acceptable salts, acids or derivatives of any of the above.

The term "cytotoxic agent" as used herein refers to a substance that inhibits or prevents the function of cells and/or causes destruction of cells. The term is intended to include radioactive isotopes (*e.g.* At²¹¹, I¹³¹, I¹²⁵, Y⁹⁰, Re¹⁸⁶, Re¹⁸⁸, Sm¹⁵³, Bi²¹², P³² and radioactive isotopes of Lu), chemotherapeutic agents e.g. methotrexate, adriamicin, vinca alkaloids (vincristine, vinblastine, etoposide), doxorubicin, melphalan, mitomycin C, chlorambucil, daunorubicin or other intercalating agents, enzymes and fragments thereof such as nucleolytic enzymes, antibiotics, and toxins such as small molecule toxins or enzymatically active toxins of bacterial, fungal, plant or animal origin, including fragments and/or variants thereof, and the various antitumor or anticancer agents disclosed below. Other cytotoxic agents are described below.

A "growth inhibitory agent" when used herein refers to a compound or composition which inhibits growth of a cell, especially a CD20 expressing cancer cell, either *in vitro* or *in vivo.* Thus, the growth inhibitory agent may be one which significantly reduces the percentage of PSCA expressing cells in S phase. Examples of growth inhibitory agents include agents that block cell cycle progression (at a place other than S phase), such as agents that induce G1 arrest and M-phase arrest. Classical M-phase blockers include the vincas (vincristine and vinblastine), taxanes, and topoisomerase II inhibitors such as doxorubicin, epirubicin, daunorubicin, etoposide, and bleomycin. Those agents that arrest G1 also spill over into S-phase arrest, for example, DNA alkylating agents such as tamoxifen, prednisone, dacarbazine, mechlorethamine, cisplatin, methotrexate, 5-fluorouracil, and ara-C. Further information can be found in The Molecular Basis of Cancer, Mendelsohn and Israel, eds., Chapter 1, entitled "Cell cycle regulation, oncogenes, and antineoplastic drugs" by Murakami et al. (WB Saunders: Philadelphia, 1995), especially p. 13. The taxanes (paclitaxel and docetaxel) are anticancer drugs both derived from the yew tree. Docetaxel (TAXOTERE®, Rhone-Poulenc Rorer), derived from the European yew, is a semisynthetic analogue of paclitaxel (TAXOL®, Bristol-Myers Squibb). Paclitaxel and docetaxel promote the assembly of microtubules from tubulin dimers and stabilize microtubules by preventing depolymerization, which results in the inhibition of mitosis in cells.

The term "mammal" refers to any animal classified as a mammal, including humans, domestic and farm animals, and zoo, sports, or pet animals, such as dogs, horses, cats, cows, etc. Preferably, the mammal herein is human.

The term "therapeutically effective amount" refers to an amount of an antibody or a antagonist drug effective to "alleviate" or "treat" a disease or disorder in a subject or mammal. In the case of cancer, the therapeutically effective amount of the drug may reduce the number of cancer cells; reduce the tumor size; inhibit (*i.e.,* slow to some extent and preferably stop) cancer cell infiltration into peripheral organs; inhibit (*i.e*., slow to some extent and preferably stop) tumor metastasis; inhibit, to some extent, tumor growth; and/or relieve to some extent one or more of the symptoms associated with the cancer. See the definition of "treated"below. To the extent the drug may prevent growth and/or kill existing cancer cells, it may be cytostatic and/or cytotoxic.

The anti-CD20 antibodies can be produced by transient or stable transfection eukaryotic host cells such as CHO cells.

"Carriers" as used herein include pharmaceutically acceptable carriers, excipients, or stabilizers which are nontoxic to the cell or mammal being exposed thereto at the dosages and concentrations employed. Often the physiologically acceptable carrier is an aqueous pH buffered solution. Examples of physiologically acceptable carriers include buffers such as phosphate, citrate, and other organic acids; antioxidants including ascorbic acid; low molecular weight (less than about 10 residues) polypeptide; proteins, such as serum albumin, gelatin, or immunoglobulins; hydrophilic polymers such as polyvinylpyrrolidone; amino acids such as glycine, glutamine, asparagine, arginine or lysine; monosaccharides, disaccharides, and other carbohydrates including glucose, mannose, or dextrins; chelating agents such as EDTA; sugar alcohols such as mannitol or sorbitol; salt-forming counterions such as sodium; and/or nonionic surfactants such as TWEEN™, polyethylene glycol (PEG), and PLURONICS™.

### 1. Polypeptide BLyS Antagonists

Polypeptides useful as antagonists of BLyS include, e.g., the polypeptide referred to as TALL-1 12-3 as SEQ ID No. 123 in WO 02/092620, the amino acid sequence provided here: 
MLPGCKWDLLIKQWVCDPLGSGSATGGSGSTASSGSGSATHMLPGCKWDLLIKQWVCDPLGGGG
GVDKTHTCPPCPAPELLGGPSVFLFPPKPKDTLMISRTPEVTCVVVDVSHEDPEVKFNWYVDGVEV
HNAKTKPREEQYNSTYRVVSVLTVLHQDWLNGKEYKCKVSNKALPAPIEKTISKAKGQPREPQVY
TLPPSRDELTKNQVSLTCLVKGFYPSDIAVEWESNGQPENNYKTTPPVLDSDGSFFLYSKLTVDKS
RWQQGNVFSCSVMHEALHNHYTQKSLSLSPGK (SEQ ID NO. 10)
 This polypeptide binds BLyS and inhibits BR3 binding to BLyS.
In some embodiments, the 17-mer peptides are soluble (preferably not membrane bound), and may be used as core sequences or otherwise combined or conjugated with a variety of structures as is described below. Some amino acids in the 17-mer polypeptide were randomized and screened for conservative and non-conservative substitutions. As is understood by one of skill in the art and described herein, additions and substitutions may be accomplished on a limited basis without impairing the BLyS binding of the resulting 17mer peptide and constructs including the resulting 17mer peptide. Guidance as to allowed substitutions that yield BLyS binding function is provided below and in the examples. In some embodiments, resides implicated in structural or binding affinity relationships are conserved, meaning that either the amino acid identity is retained or a conservative substitution is made as described in the formulas and description below.

A BLyS polypeptide antagonist may comprise a sequence selected from the group consisting of: Formula I, Formula II, Formula III, a sequence recited in FIG.32,

ECFDLLVRAWVPCSVLK (SEQ ID NO 5), ECFDLLVRHWVPCGLLR (SEQ ID NO 6),
ECFDLLVRRWVPCEMLG (SEQ ID NO 7), ECFDLLVRSWVPCHMLR (SEQ ID NO 8),
 (SEQ ID NO 9), and mixtures thereof.

The polypeptide may comprise an amino acid sequence of Formula I:

X₁-C_{N}-X₃-D-X₅-L-X₇-X₈-X₉-X₁₀-X₁₁-X₁₂-C_{T}-X₁₄-X₁₅-X₁₆-X₁₇ (Formula I)

wherein X₁, X₃, X₅, X₇, X₈, X₉, X₁₀, X₁₁, X₁₂, X₁₄, X₁₅ and X₁₇ are any amino acid except cysteine; and
wherein X₁₆ is an amino acid selected from the group consisting of L, F, I and V; and wherein the polypeptide does not comprise a cysteine within seven amino acid residues N-terminal to C_{N} (cysteine N terminal) and C-terminal to C_{T} (cysteine C terminal) of Formula I.

A polypeptide comprising the sequence of Formula I may have C_{N} and C_{T} joined by disulfide bonding; X₅LX₇X₈ forming the conformation of a type I beta turn structure with the center of the turn between L and X₇; and has a positive value for the dihedral angle phi of X₈.

X₁₀ may be selected from the group consisting of W, F, V, L, I, Y, M and a non-polar amino acid. In some embodiments, X₁₀ is W. X₃ may be an amino acid selected from the group consisting of M, V, L, I, Y, F, W and a non-polar amino acid. X₅ may be selected from the group consisting of V, L, P, S, I, A and R. X₇ may be selected from the group consisting of V, T, I and L. X₇ may not be T or I. X₈ may be selected from the group consisting of any R, K, G, N, H and a D-amino acid. X₉ may be selected from the group consisting of H, K, A, R and Q. X₁₁ may be I or V. In some embodiments, X₁₂ is selected from the group consisting of P, A, D, E and S. In some embodiments, X₁₆ is L.

The sequence of Formula I may be a sequence selected from the group consisting of ECFDLLVRAWVPCSVLK (SEQ ID NO 5), ECFDLLVRHWVPCGLLR (SEQ ID NO 6), ECFDLLVRRWVPCEMLG (SEQ ID NO 7), ECFDLLVRSWVPCHMLR (SEQ ID NO 8), ECFDLLVRHWVACGLLR (SEQ ID NO 9).

The polypeptide may comprise an amino acid sequence of Formula II:

X₁-C_{N}-X₃-D-X₅-L-V-X₈-X₈-W-X₁₁-X₁₂-C_{T}-X₁₄-X₁₅-L₇X₁₇ (Formula II)

wherein X₁, X₃, X₅, X₈, X₉, X₁₁, X₁₂, X₁₄, X₁₅ and X₁₇ are any amino acid, except cysteine; and wherein the polypeptide does not comprise a cysteine within seven amino acid residues N-terminal to C_{N} (cysteine N terminal) and C-terminal to C_{T} (cysteine C terminal) of Formula II.

The polypeptide comprising the sequence of Formula I may have C_{N} and C_{T} joined by disulfide bonding; X₅LVX₈ forming the conformation of a type I beta turn structure with the center of the turn between L and X₇; and has a positive value for the dihedral angle phi of X₈.

In the polypeptide of Formula II, X₃ may be an amino acid selected from the group consisting of M, A, V, L, I, Y, F, W and a non-polar amino acid. X₅ may be selected from the group consisting of V, L, P, S, I, A and R. X₈ may be selected from the group consisting of R, K, G, N, H and a D-amino acid. X₉ may be selected from the group consisting of H, K, A, R and Q. X₁₁ may be selected from the group consisting of I and V. X₁₂ may be selected from the group consisting of P, A, D, E and S.

The polypeptide may comprise a sequence selected from any one of the sequences described in FIG.32.

The polypeptide may comprise an amino acid sequence of Formula III:

E-C_{N}-F-D-X₅-L-V-X₈-X₉-W-V-X₁₂-C_{T}-X₁₄X₁₅X₁₆X₁₇ (Formula III)

wherein X₅, X₈, X₉, X₁₂, X₁₄, X₁₅ and X₁₇ are any amino acid except cysteine;
wherein X₁₆ is an amino acid selected from the group consisting of L, F, I and V;
wherein the polypeptide does not comprise a cysteine within seven amino acid residues N-terminal to C_{N} (cysteine N terminal) and C-terminal to C_{T} (cysteine C terminal) of Formula III; and
wherein C_{N} and C_{T} are joined by disulfide bonding.

The polypeptide comprising the contiguous sequence of Formula III may have a disulfide bond between C_{N} and C_{T} and forms a type I beta turn structure with the center of the turn between L and V at X₅LVX₈; and has a positive value for the dihedral angle phi of X₈.

In the polypeptide of Formula III, X₅, X₈, X₉, X₁₂, X₁₄, X₁₅ and X₁₇ may be selected from the group consisting of L, P, H, R, I, T, N, S, V, A, D, and G. In the polypeptide of Formula III, X₅ may be L and X₈ may be R. In the polypeptide of Formula III, X₉ may be selected from the group consisting of H, K, A, S, R and Q. In the polypeptide of Formula III, X₁₂ may be selected from the group consisting of P, A, D, E and S. In the polypeptide of Formula III, X₁₂ may be P. In the polypeptide of Formula III, X₁₆ may be L.

The sequence of Formula III may be selected from the group consisting of ECFDLLVRAWVPCSVLK(SEQ ID NO. 5), ECFDLLVRHWVPCGLLR (SEQ ID NO. 6), ECFDLLVRRWVPCEMLG (SEQ ID NO. 7), ECFDLLVRSWVPCHMLR (SEQ ID NO. 8) and ECFDLLVRHWVACGLLR (SEQ ID NO. 9).

Also disclosed is a polypeptide comprising a contiguous polypeptide sequence selected from the group consisting of ECFDLLVRAWVPCSVLK, ECFDLLVRHWVPCGLLR, ECFDLLVRRWVPCEMLG, ECFDLLVRSWVPCHMLR, and ECFDLLVRHWVACGLLR (SEQ ID NO. 5 TO 9). Also disclosed herein is a polypeptide comprising a sequence selected from any one of the sequences described in FIG. 32. Polypeptides comprising any one of the sequences described in FIG. 32 preferably join the cysteines of the sequence by disulfide bonding. The sequence between the fifth and eighth residues of the sequence may form a conformation of a type I beta turn structure with the center of the turn between L and X₇ and the eighth residue has a positive value for the dihedral angle phi.

The BlyS polypeptides may be contiguous sequences. Disclosed herein is a polypeptide comprising at least 88% sequence identity with a contiguous 17mer polypeptide sequence selected from the group consisting of: ECFDLLVRAWVPCSVLK, ECFDLLVRHWVPCGLLR, ECFDLLVRRWVPCEMLG, ECFDLLVRSWVPCHMLR, and ECFDLLVRHWVACGLLR (SEQ ID NOs 5-9). The sequence identity may be at least 64 %, and each successive integer to 100% after aligning to provide maximum homology. Homology is reduced for sequence gaps and sequences shorter than the 17mers of the present invention after aligning to provide maximum homology. Neither N-nor C-terminal extensions nor insertions shall be construed as reducing homology.

The polypeptide may be less than 50 amino acids in length, less than 25 amino acids in length, or is a 17-mer.

The polypeptides disclosed herein may comprise additional polypeptide sequences N-terminal, C-terminal or both N-terminal and C-terminal to the sequence of Formula I, Formula II, Formula III, ECFDLLVRAWVPCSVLK (SEQ ID NO 5), ECFDLLVRHWVPCGLLR (SEQ ID NO 6), ECFDLLVRRWVPCEMLG (SEQ ID NO 7), ECFDLLVRSWVPCHMLR (SEQ ID NO 8), ECFDLLVRHWVACGLLR (SEQ ID NO 9), or sequences listed in FIG.32. The additional polypeptide sequences are heterologous to a native sequence BR3 polypeptide, and include, for example, Fc portion of immunoglobulins.

The BlyS antagonist polypeptides may comprise at least one and more preferably, more than one of a polypeptide comprising a sequence of Formula I, Formula II, Formula III, ECFDLLVRAWVPCSVLK (SEQ ID NO 5), ECFDLLVRHWVPCGLLR (SEQ ID NO 6), ECFDLLVRRWVPCEMLG (SEQ ID NO 7), ECFDLLVRSWVPCHMLR (SEQ ID NO 8), ECFDLLVRHWVACGLLR (SEQ ID NO 9), or sequences listed in FIG. 32. The polypeptides that are linked together can have the same sequence or have different sequences. These polypeptides can be joined to one another, optionally, through the use of a linker. The linker serves as a spacer and can be made of a variety of chemical compounds. The linker may be a polypeptide that has about 1 to 50 amino acids, more preferably about 1 to 30 amino acids.. Linker sequences are known to those of skill in the art. For example, linker sequences include GGGKGGGG and GGGNSSGG and the like. The polypeptides linked together may have the same sequence and comprise a formula: PP1-L1-PP1-L2-PP1, wherein PP1 comprises an amino acid sequence selected from the group consisting of Formula I, Formula II, Formula III, ECFDLLVRAWVPCSVLK (SEQ ID NO 5), ECFDLLVRHWVPCGLLR (SEQ ID NO 6), ECFDLLVRRWVPCEMLG (SEQ ID NO 7), ECFDLLVRSWVPCHMLR (SEQ ID NO 8), ECFDLLVRHWVACGLLR (SEQ ID NO 9), and sequences listed in FIG.32, and LI and L2 are linker sequences that are different in sequence.

Antagonists for BLyS binding to BR3, such as the polypeptides described herein, preferably bind to BLyS with an affinity the same as or greater than a native BR3 sequence, such as BR3 ECD of SEQ ID NO:__ or mini-BR3 of SEQ ID NO:_. The polypeptides having a sequence of that of Formula I, Formula II, Formula III, ECFDLLVRAWVPCSVLK (SEQ ID NO 5), ECFDLLVRHWVPCGLLR (SEQ ID NO 6), ECFDLLVRRWVPCEMLG (SEQ ID NO 7), ECFDLLVRSWVPCHMLR (SEQ ID NO 8), ECFDLLVRHWVACGLLR (SEQ ID NO 9), or sequences listed in FIG.32 may have a binding affinity for BLyS of about 100nM or less, preferably 10 nM or less, or 1 nM or less. One method of measuring binding affinity is provided in the Examples.

A method disclosed herein to find BLyS antagonists involves identifying, modifying and selectively randomizing a core sequence of 17 residues of BR3. Specific techniques used are described further below and in the examples. The N terminal cysteine residue (C_{N}) at position X₂ and C-terminal cysteine (C_{T}) at position X₁₃ may be conserved and preferably form a disulfide bridge. In some embodiments, C_{N} and C_{T} are separated by 10 contiguous amino acids. Preferably, the 17mer sequence does not contain any cysteine residues other than at positions X₂ and X₁₃. Additionally, if the 17mer is included in a larger structure, sequences flanking the 17mer will preferably not include any cysteine residues within 7 amino acids of C_{N} or C_{T}. X₁₀ is substituted with any non-polar amino acid except for cysteine; for example: W, F, V, L, I, Y or M. X₁₀ may be W.

The motif D-X₅-L-X₇ may be conserved due to demonstrated contribution to BLyS binding. A beta-turn located between C_{N} and C_{T}, may be formed between X₄ and X₇. The center of the beta-turn may be positioned between L-X₇. The structure of the 17mer peptides disclosed herein is generally two beta-strands linked by a type I beta-turn, forming a beta-hairpin connected by a disulfide bond between C_{N} and C_{T}. Additionally, the residue at X₈ may adopt a positive value for the dihedral angle phi of X₈ to accommodate the type I beta turn in the beta hairpin structure.

Additional structural information indicates that D at X₄ and L at X₆ are buried in the BLyS-BR3 interface of BLyS-BR3 complex. These residues may be conserved. The residue at X₇ is also buried in the BLyS-BR3 interface. X₇ may be selected from the group consisting of V, T, I and L. X₇ is preferably V. The motif from X₄ to X₇ may be DLLV.

The length of the binding region of the BLyS antagonist may be 17 amino acids. The polypeptide BLyS antagonist may be 17 amino acids. Four amino acids, X₁₄-X₁₇, may follow C_{T} at the C-terminal end. X₁₆ may form a hydrophobic contact with BLyS when the 17mer is bound and is conserved. X₁₆ may be L.

### 2. Polynucleotides, Vectors, Host Cells

The BLyS antagonist polypeptides may be selected from the group consisting of: 17mer peptides described herein, polypeptides incorporating one or more 17mer peptides as core regions, and covalently modified forms of the 17mer peptides and polypeptides (e.g., immunoadhesins, labeled polypeptides, protected polypeptides, conjugated polypeptides, fusion proteins, etc.). Various techniques that are employed for making these forms of polypeptides are described herein. Methods for labeling polypeptides and conjugating molecules to polypeptides are known in the art.

Compositions disclosed herein can be prepared using recombinant techniques known in the art. The description below relates to methods of producing such polypeptides by culturing host cells transformed or transfected with a vector containing the encoding nucleic acid and recovering the polypeptide from the cell culture. (See, e.g., Sambrook et al., Molecular Cloning: A Laboratory Manual (New York: Cold Spring Harbor Laboratory Press, 1989); Dieffenbach et al., PCR Primer: A Laboratory Manual (Cold Spring Harbor Laboratory Press, 1995)).

The nucleic acid (e.g., cDNA or genomic DNA) encoding the desired polypeptide may be inserted into a replicable vector for further cloning (amplification of the DNA) or for expression. Various vectors are publicly available. The vector components generally include, but are not limited to, one or more of the following: a signal sequence, an origin of replication, one or more marker genes, an enhancer element, a promoter, and a transcription termination sequence, each of which is described below. Optional signal sequences, origins of replication, marker genes, enhancer elements and transcription terminator sequences that may be employed are known in the art and described in further detail in WO97/25428.

Expression and cloning vectors usually contain a promoter that is recognized by the host organism and is operably linked to the encoding nucleic acid sequence. Promoters are untranslated sequences located upstream (5') to the start codon of a structural gene (generally within about 100 to 1000 bp) that control the transcription and translation of a particular nucleic acid sequence, to which they are operably linked. Such promoters typically fall into two classes, inducible and constitutive. Inducible promoters are promoters that initiate increased levels of transcription from DNA under their control in response to some change in culture conditions, e.g., the presence or absence of a nutrient or a change in temperature. At this time a large number of promoters recognized by a variety of potential host cells are well known. These promoters are operably linked to the encoding DNA by removing the promoter from the source DNA by restriction enzyme digestion and inserting the isolated promoter sequence into the vector.

Construction of suitable vectors containing one or more of the above-listed components employs standard ligation techniques. Isolated plasmids or DNA fragments are cleaved, tailored, and re-ligated in the form desired to generate the plasmids required. For analysis to confirm correct sequences in plasmids constructed, the ligation mixtures can be used to transform E. coli K12 strain 294 (ATCC 31,446) and successful transformants selected by ampicillin or tetracycline resistance where appropriate. Plasmids from the transformants are prepared, analyzed by restriction endonuclease digestion, and/or sequenced using standard techniques known in the art. [See, e.g., Messing et al., Nucleic Acids Res., 9:309 (1981); Maxam et al., Methods in Enzymology, 65:499 (1980)].

Expression vectors that provide for the transient expression in mammalian cells of the encoding DNA may be employed. In general, transient expression involves the use of an expression vector that is able to replicate efficiently in a host cell, such that the host cell accumulates many copies of the expression vector and, in turn, synthesizes high levels of a desired polypeptide encoded by the expression vector [Sambrook et al., supra]. Transient expression systems, comprising a suitable expression vector and a host cell, allow for the convenient positive identification of polypeptides encoded by cloned DNAs, as well as for the rapid screening of such polypeptides for desired biological or physiological properties.

Other methods, vectors, and host cells suitable for adaptation to the synthesis of the desired polypeptide in recombinant vertebrate cell culture are described in Gething et al., Nature, 293:620-625 (1981); Mantei et al., Nature, 281:40-46 (1979); EP 117,060; and EP 117,058.

Suitable host cells for cloning or expressing the DNA in the vectors herein include prokaryote, yeast, or higher eukaryote cells. Suitable prokaryotes for this purpose include but are not limited to eubacteria, such as Gram-negative or Gram-positive organisms; for example, Enterobacteriaceae such as Escherichia, e.g., E. coli, Enterobacter, Erwinia, Klebsiella, Proteus, Salmonella, e.g., Salmonella typhimurium, Serratia, e.g., Serratia marcescans, and Shigella, as well as Bacilli such as B. subtilis and B. licheniformis (e.g., B. licheniformis 41P disclosed in DD 266,710 published 12 April 1989), Pseudomonas such as P. aeruginosa, and Streptomyces. Preferably, the host cell should secrete minimal amounts of proteolytic enzymes.

In addition to prokaryotes, eukaryotic microbes such as filamentous fungi or yeast are suitable cloning or expression hosts for vectors. Suitable host cells for the expression of glycosylated polypeptide are derived from multicellular organisms. Examples of all such host cells are described further in WO97/25428.

Host cells are transfected and preferably transformed with the above-described expression or cloning vectors and cultured in nutrient media modified as appropriate for inducing promoters, selecting transformants, or amplifying the genes encoding the desired sequences.

Transfection refers to the taking up of an expression vector by a host cell whether or not any coding sequences are in fact expressed. Numerous methods of transfection are known to the ordinarily skilled artisan, for example, CaPO4 and electroporation. Successful transfection is generally recognized when any indication of the operation of this vector occurs within the host cell.

Transformation means introducing DNA into an organism so that the DNA is replicable, either as an extrachromosomal element or by chromosomal integrant. Depending on the host cell used, transformation is done using standard techniques appropriate to such cells. The calcium treatment employing calcium chloride, as described in Sambrook et al., supra, or electroporation is generally used for prokaryotes or other cells that contain substantial cell-wall barriers. Infection with Agrobacterium tumefaciens is used for transformation of certain plant cells, as described by Shaw et al., Gene, 23:315 (1983) and WO 89/05859 published 29 June 1989. In addition, plants may be transfected using ultrasound treatment as described in WO 91/00358 published 10 January 1991.

For mammalian cells without such cell walls, the calcium phosphate precipitation method of Graham and van der Eb, Virology, 52:456-457 (1978) may be employed. General aspects of mammalian cell host system transformations have been described in U.S. Pat. No. 4,399,216. Transformations into yeast are typically carried out according to the method of Van Solingen et al., J. Bact., 130:946 (1977) and Hsiao et al., Proc. Natl. Acad. Sci. (USA), 76:3829 (1979). However, other methods for introducing DNA into cells, such as by nuclear microinjection, electroporation, bacterial protoplast fusion with intact cells, or polycations, e.g., polybrene, polyornithine, may also be used. For various techniques for transforming mammalian cells, see Keown et al., Methods in Enzymology, 185:527-537 (1990) and Mansour et al., Nature, 336:348-352 (1988).

Prokaryotic cells can be cultured in suitable culture media as described generally in Sambrook et al., supra. Examples of commercially available culture media include Ham's F10 (Sigma), Minimal Essential Medium ("MEM", Sigma), RPMI-1640 (Sigma), and Dulbecco's Modified Eagle's Medium ("DMEM", Sigma). Any such media may be supplemented as necessary with hormones and/or other growth factors (such as insulin, transferrin, or epidermal growth factor), salts (such as sodium chloride, calcium, magnesium, and phosphate), buffers (such as HEPES), nucleosides (such as adenosine and thymidine), antibiotics (such as gentamycin); trace elements (defined as inorganic compounds usually present at final concentrations in the micromolar range), and glucose or an equivalent energy source. Any other necessary supplements may also be included at appropriate concentrations that would be known to those skilled in the art. The culture conditions, such as temperature, pH, and the like, are those previously used with the host cell selected for expression, and will be apparent to the ordinarily skilled artisan.

In general, principles, protocols, and practical techniques for maximizing the productivity of mammalian cell cultures can be found in Mammalian Cell Biotechnology: A Practical Approach, M. Butler, ed. (IRL Press, 1991).

The expressed polypeptides may be recovered from the culture medium as a secreted polypeptide, although may also be recovered from host cell lysates when directly produced without a secretory signal. If the polypeptide is membrane-bound, it can be released from the membrane using a suitable detergent solution (e.g. Triton-X 100) or its extracellular region may be released by enzymatic cleavage.

When the polypeptide is produced in a recombinant cell other than one of human origin, it is free of proteins or polypeptides of human origin. However, it is usually necessary to recover or purify the polypeptide from recombinant cell proteins or polypeptides to obtain preparations that are substantially homogeneous. As a first step, the culture medium or lysate may be centrifuged to remove particulate cell debris. The following are procedures exemplary of suitable purification procedures: by fractionation on an ion-exchange column; ethanol precipitation; reverse phase HPLC; chromatography on silica or on a cation-exchange resin such as DEAE; chromatofocusing; SDS-PAGE; ammonium sulfate precipitation; gel filtration using, for example, Sephadex G-75; and protein A Sepharose columns to remove contaminants such as IgG.

### Phage Display

Polypeptides selected from the group consisting of: Formula I, Formula II, Formula III, ECFDLLVRAWVPCSVLK (SEQ ID NO 5), ECFDLLVRHWVPCGLLR (SEQ ID NO 6), ECFDLLVRRWVPCEMLG (SEQ ID NO 7), ECFDLLVRSWVPCHMLR (SEQ ID NO 8), ECFDLLVRHWVACGLLR (SEQ ID NO 9), and sequences listed in FIG.32, may utilized in phage display.

Using the techniques of phage display allows the generation of large libraries of protein variants which can be rapidly sorted for those sequences that bind to a target molecule with high affinity. Nucleic acids encoding variant polypeptides are fused to a nucleic acid sequence encoding a viral coat protein, such as the gene III protein or the gene VIII protein. Monovalent phage display systems where the nucleic acid sequence encoding the protein or polypeptide is fused to a nucleic acid sequence encoding a portion of the gene III protein have been developed. (Bass, S., Proteins, 8:309 (1990); Lowman and Wells, Methods: A Companion to Methods in Enzymology, 3:205 (1991)). In a monovalent phage display system, the gene fusion is expressed at low levels and wild type gene III proteins are also expressed so that infectivity of the particles is retained. Methods of generating peptide libraries and screening those libraries have been disclosed in many patents (e.g. U.S. Patent No. 5,723,286, U.S. Patent No. 5,432, 018, U.S. Patent No. 5,580,717, U.S. Patent No. 5,427,908 and U.S. Patent No. 5,498,530).

Formula I, Formula II or Formula III may be expressed as peptide libraries on phage. The phage expressing the library of polypeptides of Formula I, Formula II or Formula III are then subjected to selection based on BLyS binding. The selection process may involve allowing some phage bind to biotinylated BLyS which is subsequently bound to a neutravidin plate. Phage bound to the plate through the BLyS-biotin-neutravidin binding are recovered and propogated. The phage may be subject to several rounds of selection. The phage may be incubated with BLyS-biotin, followed by the addition of unbiotinylated BLyS as a competitive binder. Additional guidance of use of phage display is provided in the Examples.

### Polypeptides fused or conjugated to Heterologous polypeptides

Immunoadhesin molecules comprising the polypeptides are further contemplated for use in the methods herein. The molecule may comprise a fusion of a polypeptide disclosed herein with an immunoglobulin or a particular region of an immunoglobulin. For a bivalent form of the immunoadhesin, such a fusion usefully comprises the Fc region of an IgG molecule. The Fc region may be from a human IgG1 molecule. The immunoglobulin fusion may include the hinge, CH2 and CH3, or the hinge, CH1, CH2 and CH3 regions of an IgG1 molecule. For the production of immunoglobulin fusions, see also US Patent No. 5,428,130 issued June 27, 1995 and Chamow et al., TIBTECH, 14:52-60 (1996).

The simplest and most straightforward immunoadhesin design often combines the binding domain(s) of the adhesin (e.g. antagonist polypeptide of this invention) with the Fc region of an immunoglobulin heavy chain. For example, a polypeptide comprising a sequence of Formula I, Formula II, Formula III, ECFDLLVRAWVPCSVLK (SEQ ID NO 5), ECFDLLVRHWVPCGLLR (SEQ ID NO 6), ECFDLLVRRWVPCEMLG (SEQ ID NO 7), ECFDLLVRSWVPCHMLR (SEQ ID NO 8), ECFDLLVRHWVACGLLR (SEQ ID NO 9), or sequences listed in FIG.32 can be covalently linked to an Fc portion of an immunoglobulin. In addition, one or more of these polypeptides can be linked to one another and linked to an Fc portion of an immunoglobulin.

Ordinarily, when preparing the immunoadhesins disclosed herein, nucleic acid encoding the binding domain of the adhesin will be fused C-terminally to nucleic acid encoding the N-terminus of an immunoglobulin constant domain sequence, however N-terminal fusions are also possible.

Typically, in such fusions the encoded chimeric polypeptide will retain at least functionally active hinge, CH2 and CH3 domains of the constant region of an immunoglobulin heavy chain. Fusions are also made to the C-terminus of the Fc portion of a constant domain, or immediately N-terminal to the CH1 of the heavy chain or the corresponding region of the light chain. The precise site at which the fusion is made is not critical; particular sites are well known and may be selected in order to optimize the biological activity, secretion, or binding characteristics of the immunoadhesin.

The adhesin sequence may be fused to the N-terminus of the Fc region of immunoglobulin G1 (IgG1). It is possible to fuse the entire heavy chain constant region to the adhesin sequence. However, more preferably, a sequence beginning in the hinge region just upstream of the papain cleavage site which defines IgG Fc chemically (i.e. residue 216, taking the first residue of heavy chain constant region to be 114), or analogous sites of other immunoglobulins is used in the fusion. The adhesin amino acid sequence may be fused to (a) the hinge region and CH2 and CH3 or (b) the CH1, hinge, CH2 and CH3 domains, of an IgG heavy chain.

For bispecific immunoadhesins, the immunoadhesins are assembled as multimers, and particularly as heterodimers or heterotetramers. Generally, these assembled immunoglobulins will have known unit structures. A basic four chain structural unit is the form in which IgG, IgD, and IgE exist. A four chain unit is repeated in the higher molecular weight immunoglobulins; IgM generally exists as a pentamer of four basic units held together by disulfide bonds. IgA globulin, and occasionally IgG globulin, may also exist in multimeric form in serum. In the case of multimer, each of the four units may be the same or different.

Various exemplary assembled immunoadhesins within the scope herein are schematically diagrammed below:
(a) ACL-ACL;
(b) ACH-(ACH, ACL-ACH, ACL-VHCH, or VLCL-ACH);
(c) ACL-ACH-(ACL-ACH, ACL-VHCH, VLCL-ACH, or VLCL-VHCH)
(d) ACL-VHCH-(ACH, or ACL-VHCH, or VLCL-ACH);
(e) VLCL-ACH-(ACL-VHCH, or VLCL-ACH); and
(f) (A-Y)n-(VLCL-VHCH)2,
wherein each A represents identical or different polypeptides comprising an amino acid sequence of Formula I, Formula II, Formula III, ECFDLLVRAWVPCSVLK (SEQ ID NO 5), ECFDLLVRHWVPCGLLR (SEQ ID NO 6), ECFDLLVRRWVPCEMLG (SEQ ID NO 7), ECFDLLVRSWVPCHMLR (SEQ ID NO 8), ECFDLLVRHWVACGLLR (SEQ ID NO 9), or sequences listed in FIG.32 or combinations thereof;
VL is an immunoglobulin light chain variable domain;
VH is an immunoglobulin heavy chain variable domain;
CL is an immunoglobulin light chain constant domain;
CH is an immunoglobulin heavy chain constant domain;
n is an integer greater than 1;
Y designates the residue of a covalent cross-linking agent.

In the interests of brevity, the foregoing structures only show key features; they do not indicate joining (J) or other domains of the immunoglobulins, nor are disulfide bonds shown. However, where such domains are required for binding activity, they shall be constructed to be present in the ordinary locations which they occupy in the immunoglobulin molecules.

Alternatively, the adhesin sequences can be inserted between immunoglobulin heavy chain and light chain sequences, such that an immunoglobulin comprising a chimeric heavy chain is obtained. In this embodiment, the adhesin sequences are fused to the 3' end of an immunoglobulin heavy chain in each arm of an immunoglobulin, either between the hinge and the CH2 domain, or between the CH2 and CH3 domains. Similar constructs have been reported by Hoogenboom et al., Mol. Immunol., 28:1027-1037 (1991).

Although the presence of an immunoglobulin light chain is not required in the immunoadhesins disclosed herein, an immunoglobulin light chain might be present either covalently associated to an adhesin-immunoglobulin heavy chain fusion polypeptide, or directly fused to the adhesin. In the former case, DNA encoding an immunoglobulin light chain is typically coexpressed with the DNA encoding the adhesin-immunoglobulin heavy chain fusion protein. Upon secretion, the hybrid heavy chain and the light chain will be covalently associated to provide an immunoglobulin-like structure comprising two disulfide-linked immunoglobulin heavy chain-light chain pairs. Methods suitable for the preparation of such structures are, for example, disclosed in U.S. Patent No. 4,816,567, issued 28 March 1989.

Immunoadhesins are most conveniently constructed by fusing the cDNA sequence encoding the adhesin portion in-frame to an immunoglobulin cDNA sequence. However, fusion to genomic immunoglobulin fragments can also be used (see, e.g. Aruffo et al., Cell, 61:1303-1313 (1990); and Stamenkovic et al., Cell, 66:1133-1144 (1991)). The latter type of fusion requires the presence of Ig regulatory sequences for expression. cDNAs encoding IgG heavy-chain constant regions can be isolated based on published sequences from cDNA libraries derived from spleen or peripheral blood lymphocytes, by hybridization or by polymerase chain reaction (PCR) techniques. The cDNAs encoding the "adhesin" and the immunoglobulin parts of the immunoadhesin are inserted in tandem into a plasmid vector that directs efficient expression in the chosen host cells.

Leucine zipper forms of these molecules are also contemplated by the invention. "Leucine zipper" is a term in the art used to refer to a leucine rich sequence that enhances, promotes, or drives dimerization or trimerization of its fusion partner (e.g., the sequence or molecule to which the leucine zipper is fused or linked to). Various leucine zipper polypeptides have been described in the art. See, e.g., Landschulz et al., Science, 240:1759 (1988); US Patent 5,716,805; WO 94/10308; Hoppe et al., FEBS Letters, 344:1991 (1994); Maniatis et al., Nature, 341:24 (1989). Those skilled in the art will appreciate that a leucine zipper sequence may be fused at either the 5' or 3' end of the polypeptide disclosed herein.

The polypeptides disclosed herein can also be modified in a way to form chimeric molecules by fusing the polypeptide to another, heterologous polypeptide or amino acid sequence. Such heterologous polypeptide or amino acid sequence is one which acts to oligimerize the chimeric molecule. Such a chimeric molecule comprises a fusion of the polypeptide with a tag polypeptide which provides an epitope to which an anti-tag antibody can selectively bind. The epitope tag is generally placed at the amino- or carboxyl- terminus of the polypeptide. The presence of such epitope-tagged forms of the polypeptide can be detected using an antibody against the tag polypeptide. Also, provision of the epitope tag enables the polypeptide to be readily purified by affinity purification using an anti-tag antibody or another type of affinity matrix that binds to the epitope tag. Various tag polypeptides and their respective antibodies are well known in the art. Examples include poly-histidine (poly-his) or poly-histidine-glycine (poly-his-gly) tags; the flu HA tag polypeptide and its antibody 12CA5 [Field et al., Mol. Cell. Biol., 8:2159-2165 (1988)]; the c-myc tag and the 8F9, 3C7, 6E10, G4, B7 and 9E10 antibodies thereto [Evan et al., Molecular and Cellular Biology, 5:3610-3616 (1985)]; and the Herpes Simplex virus glycoprotein D (gD) tag and its antibody [Paborsky et al., Protein Engineering, 3(6):547-553 (1990)]. Other tag polypeptides include the Flag-peptide [Hopp et al., BioTechnology, 6:1204-1210 (1988)]; the KT3 epitope peptide [Martin et al., Science, 255:192-194 (1992)]; an "-tubulin epitope peptide [Skinner et al., J. Biol. Chem., 266:15163-15166 (1991)]; and the T7 gene 10 protein peptide tag [Lutz-Freyermuth et al., Proc. Natl. Acad. Sci. USA, 87:6393-6397 (1990)].

### Construction of Peptide-Polymer Conjugates

The strategy for the conjugation of a polymer, (e.g, PEGylation) of synthetic peptides may consist of combining, through forming a conjugate linkage in solution, a peptide and a PEG moiety, each bearing a special functionality that is mutually reactive toward the other. The peptides can be easily prepared with conventional solid phase synthesis. The peptides are "preactivated" with an appropriate functional group at a specific, site. The precursors are purified and fully characterized prior to reacting with the PEG moiety. Ligation of the peptide with PEG usually takes place in aqueous phase and can be easily monitored by reverse phase analytical HPLC. The PEGylated peptides can be easily purified by preparative HPLC and characterized by analytical HPLC, amino acid analysis and laser desorption mass spectrometry.

### a. Peptide reactive sites

A peptide may be covalently bonded via one or more of the amino acid residues of the peptide to a terminal reactive group on the polymer, depending mainly on the reaction conditions, the molecular weight of the polymer, etc. The polymer with the reactive group(s) is designated herein as activated polymer. The reactive group selectively reacts with free amino or other reactive groups on the peptide. Potential reactive sites include: N-terminal amino group, epsilon amino groups on lysine residues, as well as other amino, imino, carboxyl, sulfhydryl, hydroxyl, and other hydrophilic groups. It will be understood, however, that the type and amount of the reactive group chosen, as well as the type of polymer employed, to obtain optimum results, will depend on the particular peptide employed to avoid having the reactive group react with too many particularly active groups on the peptide. In some embodiments, a reactive residue, (e.g., lysine (K), a modified, non-natural amino acid, or other small molecule) may be substituted at a position suitable for conjugation.

The peptide may comprise the sequence of Formula I, Formula II, Formula III, ECFDLLVRAWVPCSVLK (SEQ ID NO 5), ECFDLLVRHWVPCGLLR (SEQ ID NO 6), ECFDLLVRRWVPCEMLG (SEQ ID NO 7), ECFDLLVRSWVPCHMLR (SEQ ID NO 8), ECFDLLVRHWVACGLLR (SEQ ID NO 9), or sequences listed in FIG. 32 have a terminal reactive group. The peptide may comprise at least one and more preferably, more than one of a polypeptide comprising a sequence of Formula I, Formula II, Formula III, ECFDLLVRAWVPCSVLK (SEQ ID NO 5), ECFDLLVRHWVPCGLLR (SEQ ID NO 6), ECFDLLVRRWVPCEMLG (SEQ ID NO 7), ECFDLLVRSWVPCHMLR (SEQ ID NO 8), ECFDLLVRHWVACGLLR (SEQ ID NO 9), or sequences listed in FIG.32. The polypeptides that are linked together can have the same sequence or have different sequences and a terminal reactive group. These polypeptides can be joined to one another, optionally, through the use of a linker.

While conjugation may occur at any reactive amino acid on the polypeptide, the reactive amino acid may be lysine, which is linked to the reactive group of the activated polymer through its free epsilon-amino group, or glutamic or aspartic acid, which is linked to the polymer through an amide bond. The reactive amino acids of the peptide may not be cysteine residues at positions X₂ and X₁₂.

The degree of polymer conjugation with each peptide will vary depending upon the number of reactive sites on the peptide, the molecular weight, hydrophilicity and other characteristics of the polymer, and the particular peptide derivatization sites chosen. The conjugate may have a final molar ratio of 1 to 10 polymer molecules per peptide molecule, but greater numbers of polymer molecules attached to the peptides of the invention are also contemplated. Each conjugate may contain one polymer molecule. The desired amount of derivatization is easily achieved by using an experimental matrix in which the time, temperature and other reaction conditions are varied to change the degree of substitution, after which the level of polymer substitution of the conjugates is determined by size exclusion chromatography or other means known in the art.

### b. Activated polymers

The polymer may contain only a single group which is reactive. This helps to avoid cross-linking of protein molecules. However, it is within the scope herein to maximize reaction conditions to reduce cross-linking, or to purify the reaction products through gel filtration or ion exchange chromatography to recover substantially homogenous derivatives. The polymer may contains two or more reactive groups for the purpose of linking multiple peptides to the polymer backbone. Again, gel filtration or ion exchange chromatography can be used to recover the desired derivative in substantially homogeneous form. The polymer may be covalently bonded directly to the peptide without the use of a multifunctional (ordinarily bifunctional) crosslinking agent. There may be a 1:1 molar ratio of PEG chain to peptide.

The covalent modification reaction may take place by any appropriate method generally used for reacting biologically active materials with inert polymers, preferably at about pH 5-9, more preferably 7-9 if the reactive groups on the peptide are lysine groups. Generally, the process involves preparing an activated polymer (the polymer typically having at least one terminal hydroxyl group to be activated), preparing an active substrate from this polymer, and thereafter reacting the peptide with the active substrate to produce the peptide suitable for formulation. The above modification reaction can be performed by several methods, which may involve one or more steps. Examples of modifying agents that can be used to produce the activated polymer in a one-step reaction include cyanuric acid chloride (2,4,6-trichloro-S-triazine) and cyanuric acid fluoride.

The modification reaction may take place in two steps wherein the polymer is reacted first with an acid anhydride such as succinic or glutaric anhydride to form a carboxylic acid, and the carboxylic acid is then reacted with a compound capable of reacting with the carboxylic acid to form an activated polymer with a reactive ester group that is capable of reacting with the peptide. Examples of such compounds include N-hydroxysuccinimide, 4-hydroxy-3-nitrobenzene sulfonic acid, and the like, and preferably N-hydroxysuccinimide or 4-hydroxy-3-nitrobenzene sulfonic acid is used. For example, monomethyl substituted PEG may be reacted at elevated temperatures, preferably about 100-110°C for four hours, with glutaric anhydride. The monomethyl PEG-glutaric acid thus produced is then reacted with N-hydroxysuccinimide in the presence of a carbodiimide reagent such as dicyclohexyl or isopropyl carbodiimide to produce the activated polymer, methoxypolyethylene glycolyl-N-succinimidyl glutarate, which can then be reacted with the GH. This method is described in detail in Abuchowski et al., Cancer Biochem. Biophys., 7: 175-186 (1984). In another example, the monomethyl substituted PEG may be reacted with glutaric anhydride followed by reaction with 4-hydroxy-3-nitrobenzene sulfonic acid (HNSA) in the presence of dicyclohexyl carbodiimide to produce the activated polymer. HNSA is described by Bhatnagar et al., Peptides: Synthesis-Structure-Func- tion. Proceedings of the Seventh American Peptide Symposium, Rich et al. (eds.) (Pierce Chemical Co., Rockford III., 1981), p. 97-100, and in Nitecki et al., High-Technology Route to Virus Vaccines (American Society for Microbiology: 1986) entitled "Novel Agent for Coupling Synthetic Peptides to Carriers and Its Applications.

Covalent binding to amino groups is accomplished by known chemistries based upon cyanuric chloride, carbonyl diimidazole, aldehyde reactive groups (PEG alkoxide plus diethyl acetal of bromoacetaldehyde; PEG plus DMSO and acetic anhydride, or PEG chloride plus the phenoxide of 4-hydroxybenzaldehyde, activated succinimidyl esters, activated dithiocarbonate PEG, 2,4,5-trichlorophenylcloroformate or P-nitrophenylcloroformate activated PEG.). Carboxyl groups are derivatized by coupling PEG-amine using carbodiimide. Sulfhydryl groups are derivatized by coupling to maleimido-substituted PEG (e.g. alkoxy-PEG amine plus sulfosuccinimidyl 4-(N-maleimidomethyl)cyclohexane-1-carboxylate) as described in WO 97/10847 published Mar. 27, 1997, or PEG-maleimide commercially available from Nektar Technologies, San Carlos, CA (formerly Shearwater Polymers, Inc.). Alternatively, free amino groups on the peptide (e.g. epsilon amino groups on lysine residues) may be coupled to N-hydroxysucciminidyl substituted PEG (PEG-NHS available from Nektar Technologies;) or can be thiolated with 2-imino-thiolane (Traut's reagent) and then coupled to maleimide-containing derivatives of PEG as described in Pedley et al., Br. J. Cancer, 70: 1126-1130 (1994).

Many inert polymers, including but not limited to PEG, are suitable for use in pharmaceuticals. See, e.g., Davis et al., Biomedical Polymers: Polymeric Materials and Pharmaceuticals for Biomedical Use, pp.441-451 (1980). In some embodiments of the invention, a non-proteinaceous polymer is used. The nonproteinaceous polymer is typicially a hydrophilic synthetic polymer, i.e., a polymer not otherwise found in nature. However, polymers which exist in nature and are produced by recombinant or in vitro methods are also useful, as are polymers which are isolated from native sources. Hydrophilic polyvinyl polymers are disclosed herein, e.g. polyvinylalcohol and polyvinylpyrrolidone. Particularly useful are polyalkylene ethers such as polyethylene glycol (PEG); polyoxyalkylenes such as polyoxyethylene, polyoxypropylene, and block copolymers of polyoxyethylene and polyoxypropylene (Pluronics); polymethacrylates; carbomers; branched or unbranched polysaccharides which comprise the saccharide monomers D-mannose, D- and L-galactose, fucose, fructose, D-xylose, L-arabinose, D-glucuronic acid, sialic acid, D-galacturonic acid, D-mannuronic acid (e.g. polymannuronic acid, or alginic acid), D-glucosamine, D-galactosamine, D-glucose and neuraminic acid including homopolysaccharides and heteropolysaccharides such as lactose, amylopectin, starch, hydroxyethyl starch, amylose, dextrane sulfate, dextran, dextrins, glycogen, or the polysaccharide subunit of acid mucopolysaccharides, e.g. hyaluronic acid; polymers of sugar alcohols such as polysorbitol and polymannitol; heparin or heparon.

The polymer prior to conjugation need not be, but preferably is, water soluble, but the final conjugate is preferably water-soluble. Preferably, the conjugate exhibits a water solubility of at least about 0.01 mg/ml, and more preferably at least about 0.1 mg/ml, and still more preferably at least about 1 mg/ml. In addition, the polymer should not be highly immunogenic in the conjugate form, nor should it possess viscosity that is incompatible with intravenous infusion, injection, or inhalation if the conjugate is intended to be administered by such routes.

The molecular weight of the polymer can range up to about 100,000 D, and preferably is at least about 500 D, or at least about 1,000 D, or at least about 5,000 D. The PEG or other polymer may have a molecular weight in the range of 5000 to 20,000 D. The molecular weight chosen can depend upon the effective size of the conjugate to be achieved, the nature (e.g. structure, such as linear or branched) of the polymer, and the degree of derivatization, i.e. the number of polymer molecules per peptide, and the polymer attachment site or sites on the peptide. Branched PEG's may be used to induce a large increase in effective size of the peptides. PEG or other polymer conjugates may be utilized to increase half-life, increase solubility, stabilize against proteolytic attack, and reduce immunogenicity.

Functionalized PEG polymers to modify the peptides of the invention are available from Nektar Technologies of San Carlos, CA (formerly Shearwater Polymers, Inc.). Such commercially available PEG derivatives include, but are not limited to, amino-PEG, PEG amino acid esters. PEG- N-hydroxysuccinamide chemistry (NHS), PEG-hydrazide, PEG-thiol, PEG-succinate, carboxymethylated PEG, PEG-propionic acid, PEG amino acids, PEG succinimidyl succinate, PEG succinimidyl propionate, succinimidyl ester of carboxymethylated PEG, succinimidyl carbonate of PEG, succinimidyl esters of amino acid PEGs, PEG-xycarbonylimidazole, PEG-nitrophenyl carbonate, PEG tresylate, PEG-glycidyl ether, PEG-aldehyde, PEG vinylsulfone, PEG-maleimide, PEG-orthopyridyl-disulfide, heterofunctional PEGs, PEG vinyl derivatives, PEG silanes, and PEG phospholides. The reaction conditions for coupling these PEG derivatives will vary depending on the protein, the desired degree of PEGylation, and the PEG derivative utilized. Some factors involved in the choice of PEG derivatives include: the desired point of attachment (such as lysine or cysteine R-groups), hydrolytic stability and reactivity of the derivatives, stability, toxicity and antigenicity of the linkage, suitability for analysis, etc. Specific instructions for the use of any particular derivative are available from the manufacturer.

### c. Characterization of conjugates.

The conjugates may be characteized by SDS-PAGE, gel filtration, NMR, tryptic mapping, liquid chromatrography-mass spectrophotometry, and in vitro biological assays. For example, the extent of PEG conjugation may be shown by SDS-PAGE and gel filtration, and then analyzed by NMR, which has a specific resonance peak for the methylene hydrogens of PEG. The number of PEG groups on each molecule can be calculated from the NMR spectrum or mass spectrometry. Polyacrylamide gel electrophoresis in 10% SDS is appropriately run in 10 mM Tris-HCI pH 8.0, 100 mM NaCl as elution buffer. To demonstrate which residue is PEGylated, tryptic mapping can be performed. Thus, PEGylated peptides are digested with trypsin at the protein/enzyme ratio of 100 to 1 in mg basis at 37°C for 4 hours in 100 mM sodium acetate, 10 mM Tris-HCI, 1 mM calcium chloride, pH 8.3, and acidified to pH<4 to stop digestion before separating on HPLC Nucleosil C-18 (4.6 mm.times.150 mm, 5.mu., 100A). The chromatogram is compared to that of non-PEGylated starting material. Each peak can then be analyzed by mass spectrometry to verify the size of the fragment in the peak. The fragment(s) that carried PEG groups are usually not retained on the HPLC column after injection and disappear from the chromatograph. Such disappearance from the chromatograph is an indication of PEGylation on that particular fragment that should contain at least one lysine residue. PEGylated peptides may then be assayed for ability to bind to the BLyS by conventional methods.

Conjugates may be purified by ion-exchange chromatography, (e.g, ion exchange HPLC. The chemistry of many of the electrophilically activated PEG's results in a reduction of amino group charge of the PEGylated product. Thus, high resolution ion exchange chromatography can be used to separate the free and conjugated proteins, and to resolve species with different levels of PEGylation. In fact, the resolution of different species (e.g. containing one or two PEG residues) is also possible due to the difference in the ionic properties of the unreacted amino acids. Species with difference levels of PEGylation are resolved according to the methods described in WO 96/34015 (International Application No. PCT/US96/05550 published Oct. 31, 1996). Heterologous species of the conjugates are purified from one another in the same fashion.

PEG-N-hydroxysuccinamide (NHS) may react with a primary amine (e.g. lysines and the N-terminus). In some embodiments, PEG-NHS react with a C-terminal lysine (K) of the polypeptide. The lysine residue may be added to the C-terminus of the 17-mer polypeptide, while in other embodiments, X₁₇ is substituted with lysine. The polymer may react with the N-terminus. The conjugate may be generated by utilizing the derivatization and purification methods described in the Examples below.

Disclosed herein are any of the above-described conjugates formed by its component parts, i.e. one or more peptide(s) covalently attached to one or more polymer molecule(s), without any extraneous matter in the covalent molecular structure of the conjugate.

### Production of antibodies

The methods and articles of manufacture disclosed herein use, or incorporate, an antibody which binds to CD20. Accordingly, methods for generating such antibodies will be described here and in the Examples.

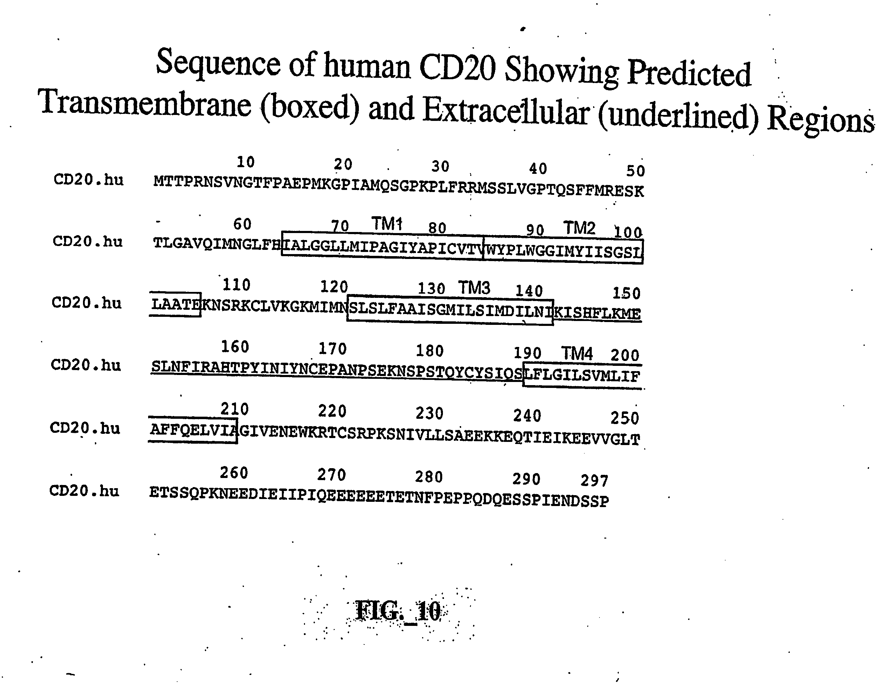

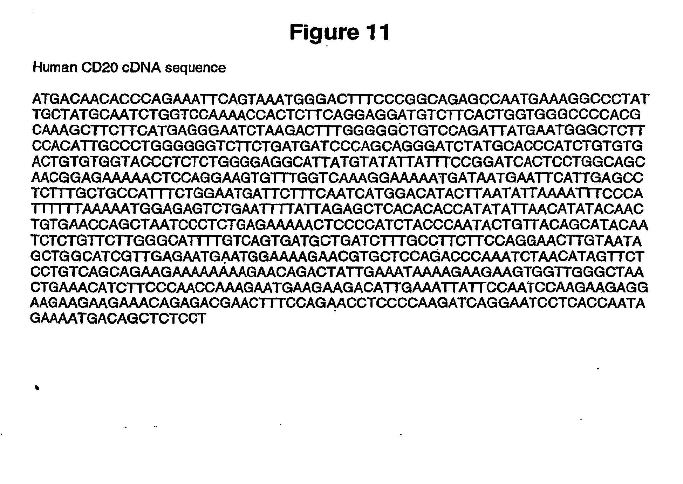

The CD20 to be used for production of, or screening for, antibodies may be, *e.g*., a soluble form of the antigen or a portion thereof, containing the desired epitope. The sequence of human CD20 is known, see Figure 10 and Figure 11. Cloning and the sequences for human CD20 are described in at least the following references: Stamenkovic I., Seed B., J. Exp. Med. 167, 1975-1980, 1988. Analysis of two cDNA clones encoding the B lymphocyte antigen CD20 (B 1, Bp35), a type III integral membrane protein."; Tedder T.F., Streuli M., Schlossman S.F., Saito H., Proc. Natl. Acad. Sci. U.S.A. 85, 208-212, 1988. "Isolation and structure of a cDNA encoding the B1 (CD20) cell-surface antigen of human B lymphocytes"; Tedder T.F., Klejman G., Schlossman S.F., Saito H., J. Immunol. 142, 2560-2568, 1989. "Structure of the gene encoding the human B lymphocyte differentiation antigen CD20 (B1)"; Einfeld D.A., Brown J.P., Valentine M.A., Clark E.A., Ledbetter J.A., EMBO J. 7, 711-717, 1988.: "Molecular cloning of the human B cell CD20 receptor predicts a hydrophobic protein with multiple transmembrane domains. "Peptide fragments of the extracellular domain (ECD) can be used as immunogens. Based on these known sequences and domain delineations, one of skill in the art can express the CD20 polypeptide and fragments thereof for use to produce antibodies.

To generate antibodies to human CD20, the extracellular domain amino acid residues 142-188 and peptide fragments of 6 of greater residues in length can be used as immunogens to raise antibodies in rodents including mice, hamsters, and rats, in rabbit, goat, or other suitable animal. Soluble CD20 polypeptide or immunogenic fragments thereof can be expressed is suitable host cells such as bacteria or eukaryotic cells. Human and murine detergent-solubilized full-length CD20 may be produced in E. coli (see Examples below) and used to immunize and screen for hybridomas producing anti-CD20 antibodies.

Alternatively, or additionally, B cells or cell lines expressing CD20 at their cell surface can be used to generate, and/or screen for, antibodies. One such cell line is the human lymphoblastoid cell line SB (ATCC accession no. ATCC CCL 120, from ATCC, Rockville, Md). The antibodies are generated to human CD20 for treatment of humans. Other forms of CD20 useful for generating antibodies will be apparent to those skilled in the art.

Phage display methodology can also be used to produce CD20 binding antibody.

The antibodies that bind CD20 may be chimeric, humanized, or human. Such antibodies and methods of generating them are described in more detail below.

### (i) Polyclonal antibodies

Polyclonal antibodies are preferably raised in animals by multiple subcutaneous (sc) or intraperitoneal (ip) injections of the relevant antigen and an adjuvant. It may be useful to conjugate the relevant antigen to a protein that is immunogenic in the species to be immunized, *e.g*., keyhole limpet hemocyanin, serum albumin, bovine thyroglobulin, or soybean trypsin inhibitor using a bifunctional or derivatizing agent, for example, maleimidobenzoyl sulfosuccinimide ester (conjugation through cysteine residues), N-hydroxysuccinimide (through lysine residues), glutaraldehyde, succinic anhydride, SOCl₂, or R¹N=C=NR, where R and R¹ are different alkyl groups.

Animals are immunized against the antigen, immunogenic conjugates, or derivatives by combining, *e.g*., 100 µg or 5 µg of the protein or conjugate (for rabbits or mice, respectively) with 3 volumes of Freund's complete adjuvant and injecting the solution intradermally at multiple sites. One month later the animals are boosted with 1/5 to 1/10 the original amount of peptide or conjugate in Freund's complete adjuvant by subcutaneous injection at multiple sites. Seven to 14 days later the animals are bled and the serum is assayed for antibody titer. Animals are boosted until the titer plateaus. Preferably, the animal is boosted with the conjugate of the same antigen, but conjugated to a different protein and/or through a different cross-linking reagent. Conjugates also can be made in recombinant cell culture as protein fusions. Also, aggregating agents such as alum are suitably used to enhance the immune response.

### (ii) Monoclonal antibodies

Monoclonal antibodies are obtained from a population of substantially homogeneous antibodies, *i.e*., the individual antibodies comprising the population are identical except for possible naturally occurring mutations that may be present in minor amounts. Thus, the modifier "monoclonal" indicates the character of the antibody as not being a mixture of discrete antibodies.

For example, the monoclonal antibodies may be made using the hybridoma method first described by Kohler et al., Nature, 256:495 (1975), or may be made by recombinant DNA methods (U.S. Patent No. 4,816,567).

In the hybridoma method, a mouse or other appropriate host animal, such as a hamster, is immunized as hereinabove described to elicit lymphocytes that produce or are capable of producing antibodies that will specifically bind to the protein used for immunization. Alternatively, lymphocytes may be immunized *in vitro.* Lymphocytes then are fused with myeloma cells using a suitable fusing agent, such as polyethylene glycol, to form a hybridoma cell (Goding, Monoclonal Antibodies: Principles and Practice, pp.59-103 (Academic Press, 1986)).

The hybridoma cells thus prepared are seeded and grown in a suitable culture medium that preferably contains one or more substances that inhibit the growth or survival of the unfused, parental myeloma cells. For example, if the parental myeloma cells lack the enzyme hypoxanthine guanine phosphoribosyl transferase (HGPRT or HPRT), the culture medium for the hybridomas typically will include hypoxanthine, aminopterin, and thymidine (HAT medium), which substances prevent the growth of HGPRT-deficient cells.

Preferred myeloma cells are those that fuse efficiently, support stable high-level production of antibody by the selected antibody-producing cells, and are sensitive to a medium such as HAT medium. Among these, preferred myeloma cell lines are murine myeloma lines, such as those derived from MOPC-21 and MPC-11 mouse tumors available from the Salk Institute Cell Distribution Center, San Diego, California USA, and SP-2 or X63-Ag8-653 cells available from the American Type Culture Collection, Rockville, Maryland USA. Human myeloma and mouse-human heteromyeloma cell lines also have been described for the production of human monoclonal antibodies (Kozbor, J. Immunol., 133:3001 (1984); Brodeur et al, Monoclonal Antibody Production Techniques and Applications, pp. 51-63 (Marcel Dekker, Inc., New York, 1987)).

Culture medium in which hybridoma cells are growing is assayed for production of monoclonal antibodies directed against the antigen. Preferably, the binding specificity of monoclonal antibodies produced by hybridoma cells is determined by immunoprecipitation or by an *in vitro* binding assay, such as radioimmunoassay (RIA) or enzyme-linked immunoabsorbent assay (ELISA).

The binding affinity of the monoclonal antibody can, for example, be determined by the Scatchard analysis of Munson et al., Anal. Biochem., 107:220 (1980).

After hybridoma cells are identified that produce antibodies of the desired specificity, affinity, and/or activity, the clones may be subcloned by limiting dilution procedures and grown by standard methods (Goding, Monoclonal Antibodies: Principles and Practice, pp.59-103 (Academic Press, 1986)). Suitable culture media for this purpose include, for example, D-MEM or RPMI-1640 medium. In addition, the hybridoma cells may be grown *in vivo* as ascites tumors in an animal.

The monoclonal antibodies secreted by the subclones are suitably separated from the culture medium, ascites fluid, or serum by conventional immunoglobulin purification procedures such as, for example, protein A-Sepharose, hydroxylapatite chromatography, gel electrophoresis, dialysis, or affinity chromatography.

DNA encoding the monoclonal antibodies is readily isolated and sequenced using conventional procedures (*e.g*., by using oligonucleotide probes that are capable of binding specifically to genes encoding the heavy and light chains of murine antibodies). The hybridoma cells serve as a preferred source of such DNA. Once isolated, the DNA may be placed into expression vectors, which are then transfected into host cells such as *E*. *coli* cells, simian COS cells, Chinese Hamster Ovary (CHO) cells, or myeloma cells that do not otherwise produce immunoglobulin protein, to obtain the synthesis of monoclonal antibodies in the recombinant host cells: Review articles on recombinant expression in bacteria of DNA encoding the antibody include Skerra et al., Curr. Opinion in Immunol., 5:256-262 (1993) and Plückthun, Immunol. Revs., 130:151-188 (1992).

Antibodies or antibody fragments can be isolated from antibody phage libraries generated using the techniques described in McCafferty et al., Nature, 348:552-554 (1990). Clackson et al., Nature, 352:624-628 (1991) and Marks et al., J. Mol. Biol., 222:581-597 (1991) describe the isolation of murine and human antibodies, respectively, using phage libraries. Subsequent publications describe the production of high affinity (nM range) human antibodies by chain shuffling (Marks et al., Bio/Technology, 10:779-783 (1992)), as well as combinatorial infection and *in vivo* recombination as a strategy for constructing very large phage libraries (Waterhouse et al., Nuc. Acids. Res., 21:2265-2266 (1993)). Thus, these techniques are viable alternatives to traditional monoclonal antibody hybridoma techniques for isolation of monoclonal antibodies.

The DNA also may be modified, for example, by substituting the coding sequence for human heavy- and light-chain constant domains in place of the homologous murine sequences (U.S. Patent No. 4,816,567; Morrison, et al., Proc. Natl Acad. Sci. USA, 81:6851 (1984)), or by covalently joining to the immunoglobulin coding sequence all or part of the coding sequence for a non-immunoglobulin polypeptide.

Typically such non-immunoglobulin polypeptides are substituted for the constant domains of an antibody, or they are substituted for the variable domains of one antigen-combining site of an antibody to create a chimeric bivalent antibody comprising one antigen-combining site having specificity for an antigen and another antigen-combining site having specificity for a different antigen.

### (iii) Humanized antibodies

Methods for humanizing non-human antibodies have been described in the art. Preferably, a humanized antibody has one or more amino acid residues introduced into it from a source which is non-human. These non-human amino acid residues are often referred to as "import" residues, which are typically taken from an "import" variable domain. Humanization can be essentially performed following the method of Winter and co-workers (Jones et al., Nature, 321:522-525 (1986); Riechmann et al., Nature, 332:323-327 (1988); Verhoeyen et al., Science, 239:1534-1536 (1988)), by substituting hypervariable region sequences for the corresponding sequences of a human antibody. Accordingly, such "humanized" antibodies are chimeric antibodies (U.S. Patent No. 4,816,567) wherein substantially less than an intact human variable domain has been substituted by the corresponding sequence from a non-human species. In practice, humanized antibodies are typically human antibodies in which some hypervariable region residues and possibly some FR residues are substituted by residues from analogous sites in rodent antibodies.

The choice of human variable domains, both light and heavy, to be used in making the humanized antibodies is very important to reduce antigenicity. According to the so-called "best-fit" method, the sequence of the variable domain of a rodent antibody is screened against the entire library of known human variable-domain sequences. The human sequence which is closest to that of the rodent is then accepted as the human framework region (FR) for the humanized antibody (Sims et al., J. Immunol., 151:2296 (1993); Chothia et al., J. Mol. Biol., 196:901 (1987)). Another method uses a particular framework region derived from the consensus sequence of all human antibodies of a particular subgroup of light or heavy chains. The same framework may be used for several different humanized antibodies (Carter et al., Proc. Natl. Acad. Sci. USA, 89:4285 (1992); Presta et al., J. Immunol., 151:2623 (1993)).

It is further important that antibodies be humanized with retention of high affinity for the antigen and other favorable biological properties. To achieve this goal, according to a preferred method, humanized antibodies are prepared by a process of analysis of the parental sequences and various conceptual humanized products using three-dimensional models of the parental and humanized sequences. , Three-dimensional immunoglobulin models are commonly available and are familiar to those skilled in the art. Computer programs are available which illustrate and display probable three-dimensional conformational structures of selected candidate immunoglobulin sequences. Inspection of these displays permits analysis of the likely role of the residues in the functioning of the candidate immunoglobulin sequence, *i.e*., the analysis of residues that influence the ability of the candidate immunoglobulin to bind its antigen. In this way, FR residues can be selected and combined from the recipient and import sequences so that the desired antibody characteristic, such as increased affinity for the target antigen(s), is achieved. In general, the hypervariable region residues are directly and most substantially involved in influencing antigen binding.

### (iv) Human antibodies

As an alternative to humanization, human antibodies can be generated. For example, it is now possible to produce transgenic animals (*e.g*., mice) that are capable, upon immunization, of producing a full repertoire of human antibodies in the absence of endogenous immunoglobulin production. For example, it has been described that the homozygous deletion of the antibody heavy-chain joining region (J_{H}) gene in chimeric and germ-line mutant mice results in complete inhibition of endogenous antibody production. Transfer of the human germ-line immunoglobulin gene array in such germ-line mutant mice will result in the production of human antibodies upon antigen challenge. See, *e.g*., Jakobovits et al., Proc. Natl. Acad. Sci. USA, 90:2551 (1993); Jakobovits et al., Nature, 362:255-258 (1993); Bruggermann et al., Year in Immuno., 7:33 (1993); and US Patent Nos. 5,591,669, 5,589,369 and 5,545,807.

Alternatively, phage display technology (McCafferty et al., Nature 348:552-553 (1990)) can be used to produce human antibodies and antibody fragments *in vitro*, from immunoglobulin variable (V) domain gene repertoires from unimmunized donors. According to this technique, antibody V domain genes are cloned in-frame into either a major or minor coat protein gene of a filamentous bacteriophage, such as M13 or fd, and displayed as functional antibody fragments on the surface of the phage particle. Because the filamentous particle contains a single-stranded DNA copy of the phage genome, selections based on the functional properties of the antibody also result in selection of the gene encoding the antibody exhibiting those properties. Thus, the phage mimics some of the properties of the B cell. Phage display can be performed in a variety of formats; for their review see, *e.g*., Johnson, Kevin S. and Chiswell, David J., Current Opinion in Structural Biology 3:564-571 (1993). Several sources of V-gene segments can be used for phage display. Clackson et al., Nature, 352:624-628 (1991) isolated a diverse array of anti-oxazolone antibodies from a small random combinatorial library of V genes derived from the spleens of immunized mice. A repertoire of V genes from unimmunized human donors can be constructed and antibodies to a diverse array of antigens (including self-antigens) can be isolated essentially following the techniques described by Marks et al., J. Mol. Biol. 222:581-597 (1991), or Griffith et al., EMBO J. 12:725-734 (1993). See, also, US Patent Nos. 5,565;332 and 5,573,905.

Human antibodies may also be generated by *in vitro* activated B cells (see US Patents 5,567,610 and 5,229,275).

### (v) Antibody fragments

Various techniques have been developed for the production of antibody fragments. Traditionally, these fragments were derived via proteolytic digestion of intact antibodies (see, *e.g*., Morimoto et al., Journal of Biochemical and Biophysical Methods 24:107-117 (1992) and Brennan et al., Science, 229:81 (1985)). However, these fragments can now be produced directly by recombinant host cells. For example, the antibody fragments can be isolated from the antibody phage libraries discussed above. Alternatively, Fab'-SH fragments can be directly recovered from *E*. *coli* and chemically coupled to form F(ab')₂ fragments (Carter et al., Bio/Technology 10:163-167 (1992)). According to another approach, F(ab')₂ fragments can be isolated directly from recombinant host cell culture. Other techniques for the production of antibody fragments will be apparent to the skilled practitioner. In other embodiments, the antibody of choice is a single chain Fv fragment (scFv). See WO 93/16185; US Patent No. 5,571,894; and US Patent No. 5,587,458. The antibody fragment may also be a "linear antibody", *e.g*., as described in US Patent 5,641,870 for example. Such linear antibody fragments may be monospecific or bispecific.

### (vi) Bispecific antibodies

Bispecific antibodies are antibodies that have binding specificities for at least two different epitopes. Exemplary bispecific antibodies may bind to two different epitopes of the B cell surface marker. Other such antibodies may bind a first B cell marker and further bind a second B cell surface marker. Alternatively, an anti-B cell marker binding arm may be combined with an arm which binds to a triggering molecule on a leukocyte such as a T-cell receptor molecule (*e.g*. CD2 or CD3), or Fc receptors for IgG (FcγR), such as FcγRI (CD64), FcγRII (CD32) and FcγRIII (CD16) so as to focus cellular defense mechanisms to the B cell. Bispecific antibodies may also be used to localize cytotoxic agents to the B cell. These antibodies possess a B cell marker-binding arm and an arm which binds the cytotoxic agent (*e.g.* saporin, anti-interferon-, vinca alkaloid, ricin A chain, methotrexate or radioactive isotope hapten). Bispecific antibodies can be prepared as full length antibodies or antibody fragments (*e.g*. F(ab')₂ bispecific antibodies).

Methods for making bispecific antibodies are known in the art. Traditional production of full length bispecific antibodies is based on the coexpression of two immunoglobulin heavy chain-light chain pairs, where the two chains have different specificities (Millstein et al., Nature, 305:537-539 (1983)). Because of the random assortment of immunoglobulin heavy and light chains, these hybridomas (quadromas) produce a potential mixture of 10 different antibody molecules, of which only one has the correct bispecific structure. Purification of the correct molecule, which is usually done by affinity chromatography steps, is rather cumbersome, and the product yields are low. Similar procedures are disclosed in WO 93/08829, and in Traunecker et al., EMBO J., 10:3655-3659 (1991).

According to a different approach, antibody variable domains with the desired binding specificities (antibody-antigen combining sites) are fused to immunoglobulin constant domain sequences. The fusion preferably is with an immunoglobulin heavy chain constant domain, comprising at least part of the hinge, CH2, and CH3 regions. It is preferred to have the first heavy-chain constant region (CH1) containing the site necessary for light chain binding, present in at least one of the fusions. DNAs encoding the immunoglobulin heavy chain fusions and, if desired, the immunoglobulin light chain, are inserted into separate expression vectors, and are co-transfected into a suitable host organism. This provides for great flexibility in adjusting the mutual proportions of the three polypeptide fragments in embodiments when unequal ratios of the three polypeptide chains used in the construction provide the optimum yields. It is, however, possible to insert the coding sequences for two or all three polypeptide chains in one expression vector when the expression of at least two polypeptide chains in equal ratios results in high yields or when the ratios are of no particular significance.

In a preferred embodiment of this approach, the bispecific antibodies are composed of a hybrid immunoglobulin heavy chain with a first binding specificity in one arm, and a hybrid immunoglobulin heavy chain-light chain pair (providing a second binding specificity) in the other arm. It was found that this asymmetric structure facilitates the separation of the desired bispecific compound from unwanted immunoglobulin chain combinations, as the presence of an immunoglobulin light chain in only one half of the bispecific molecule provides for a facile way of separation. This approach is disclosed in WO 94/04690. For further details of generating bispecific antibodies see, for example, Suresh et al., Methods in Enzymology, 121:210 (1986). According to another approach described in US Patent No. 5,731,168, the interface between a pair of antibody molecules can be engineered to maximize the percentage of heterodimers which are recovered from recombinant cell culture. The preferred interface comprises at least a part of the C_{H}3 domain of an antibody constant domain. In this method, one or more small amino acid side chains from the interface of the first antibody molecule are replaced with larger side chains (*e.g*. tyrosine or tryptophan). Compensatory "cavities" of identical or similar size to the large side chain(s) are created on the interface of the second antibody molecule by replacing large amino acid side chains with smaller ones (*e.g*. alanine or threonine). This provides a mechanism for increasing the yield of the heterodimer over other unwanted end-products such as homodimers.

Bispecific antibodies include cross-linked or "heteroconjugate" antibodies. For example, one of the antibodies in the heteroconjugate can be coupled to avidin, the other to biotin. Such antibodies have, for example, been proposed to target immune system cells to unwanted cells (US Patent No. 4,676,980), and for treatment of HIV infection (WO 91/00360, WO 92/200373, and EP 03089). Heteroconjugate antibodies may be made using any convenient cross-linking methods. Suitable cross-linking agents are well known in the art, and are disclosed in US Patent No. 4,676,980, along with a number of cross-linking techniques.

Techniques for generating bispecific antibodies from antibody fragments have also been described in the literature. For example, bispecific antibodies can be prepared using chemical linkage. Brennan et al., Science, 229: 81 (1985) describe a procedure wherein intact antibodies are proteolytically cleaved to generate F(ab')2 fragments. These fragments are reduced in the presence of the dithiol complexing agent sodium arsenite to stabilize vicinal dithiols and prevent intermolecular disulfide formation. The Fab' fragments generated are then converted to thionitrobenzoate (TNB) derivatives. One of the Fab'-TNB derivatives is then reconverted to the Fab'-thiol by reduction with mercaptoethylamine and is mixed with an equimolar amount of the other Fab'-TNB derivative to form the bispecific antibody. The bispecific antibodies produced can be used as agents for the selective immobilization of enzymes.

Recent progress has facilitated the direct recovery of Fab'-SH fragments from *E*. *coli,* which can be chemically coupled to form bispecific antibodies. Shalaby et al., J. Exp. Med., 175: 217-225 (1992) describe the production of a fully humanized bispecific antibody F(ab')2 molecule. Each Fab' fragment was separately secreted from E. *coli* and subjected to directed chemical coupling *in vitro* to form the bispecific antibody. The bispecific antibody thus formed was able to bind to cells overexpressing the ErbB2 receptor and normal human T cells, as well as trigger the lytic activity of human cytotoxic lymphocytes against human breast tumor targets.

Various techniques for making and isolating bispecific antibody fragments directly from recombinant cell culture have also been described. For example, bispecific antibodies have been produced using leucine zippers. Kostelny et al., J. Immunol., 148(5):1547-1553 (1992). The leucine zipper peptides from the Fos and Jun proteins were linked to the Fab' portions of two different antibodies by gene fusion. The antibody homodimers were reduced at the hinge region to form monomers and then re-oxidized to form the antibody heterodimers. This method can also be utilized for the production of antibody homodimers. The "diabody" technology described by Hollinger et al., Proc. Natl. Acad. Sci. USA, 90:6444-6448 (1993) has provided an alternative mechanism for making bispecific antibody fragments. The fragments comprise a heavy-chain variable domain (V_{H}) connected to a light-chain variable domain (V_{L}) by a linker which is too short to allow pairing between the two domains on the same chain. Accordingly, the V_{H} and V_{L} domains of one fragment are forced to pair with the complementary V_{L} and V_{H} domains of another fragment, thereby forming two antigen-binding sites. Another strategy for making bispecific antibody fragments by the use of single-chain Fv (sFv) dimers has also been reported. See Gruber et al., J. Immunol., 152:5368 (1994).

Antibodies with more than two valencies are contemplated. For example, trispecific antibodies can be prepared. Tutt et al. J. Immunol. 147: 60 (1991).

Amino acid sequence modification(s) of protein or peptide antagonists and antibodies described herein are contemplated. For example, it may be desirable to improve the binding affinity and/or other biological properties of the CD20 binding antibody or antagonist. Amino acid sequence variants of the antagonist are prepared by introducing appropriate nucleotide changes into the antagonist nucleic acid, or by peptide synthesis. Such modifications include, for example, deletions from, and/or insertions into and/or substitutions of, residues within the amino acid sequences of the antagonist. Any combination of deletion, insertion, and substitution is made to arrive at the final construct, provided that the final construct possesses the desired characteristics. The amino acid changes also may alter post-translational processes of the antagonist, such as changing the number or position of glycosylation sites.

A useful method for identification of certain residues or regions of the antagonist that are preferred locations for mutagenesis is called "alanine scanning mutagenesis" as described by Cunningham and Wells Science, 244:1081-1085 (1989). Here, a residue or group of target residues are identified (*e.g*., charged residues such as arg, asp, his, lys, and glu) and replaced by a neutral or negatively charged amino acid (most preferably alanine or polyalanine) to affect the interaction of the amino acids with antigen. Those amino acid locations demonstrating functional sensitivity to the substitutions then are refined by introducing further or other variants at, or for,-the sites of substitution. Thus, while the site for introducing an amino acid sequence variation is predetermined, the nature of the mutation *per se* need not be predetermined. For example, to analyze the performance of a mutation at a given site ala scanning or random mutagenesis is conducted at the target codon or region and the expressed antagonist variants are screened for the desired activity.

Amino acid sequence insertions include amino- and/or carboxyl-terminal fusions ranging in length from one residue to polypeptides containing a hundred or more residues, as well as intrasequence insertions of single or multiple amino acid residues. Examples of terminal insertions include an antagonist with an N-terminal methionyl residue or the antagonist fused to a cytotoxic polypeptide. Other insertional variants of the antagonist molecule include the fusion to the N- or C-terminus of the antagonist of an enzyme, or a polypeptide which increases the serum half-life of the antagonist.

Another type of variant is an amino acid substitution variant. These variants have at least one amino acid residue in the antagonist molecule replaced by different residue. The sites of greatest interest for substitutional mutagenesis of antibody antagonists include the hypervariable regions, but FR alterations are also contemplated. Conservative substitutions are shown in Table 1 under the heading of "preferred substitutions". If such substitutions result in a change in biological activity, then more substantial changes, denominated "exemplary substitutions" in Table 1, or as further described below in reference to amino acid classes, may be introduced and the products screened.

**Table 1**

| **Original Residue** | **Exemplary Substitutions** | **Preferred Substitutions** |
|---|---|---|
| Ala (A) | Val; Leu; Ile | Val |
| Arg (R) | Lys; Gln; Asn | Lys |
| Asn (N) | Gln; His; Asp, Lys; Arg | Gln |
| Asp (D) | Glu; Asn | Glu |
| Cys (C) | Ser; Ala | Ser |
| Gln (Q) | Asn; Glu | Asn |
| Glu (E) | Asp; Gln | Asp |
| Gly (G) | Ala | Ala |
| His (H) | Asn; Gln; Lys; Arg | Arg |
| Ile (I) | Leu; Val; Met; Ala; Phe; Norleucine | Leu |
| Leu (L) | Norleucine; Ile; Val; Met; Ala; Phe | Ile |
| Lys (K) | Arg; Gln; Asn | Arg |
| Met (M) | Leu; Phe; Ile | Leu |
| Phe (F) | Trp; Leu; Val; Ile; Ala; Tyr | Tyr |
| Pro (P) | Ala | Ala |
| Ser (S) | Thr | Thr |
| Thr (T) | Val; Ser | Ser |
| Trp (W) | Tyr; Phe | Tyr |
| Tyr (Y) | Trp; Phe; Thr; Ser | Phe |
| Val (V) | Ile; Leu; Met; Phe; Ala; Norleucine | Leu |

Substantial modifications in the biological properties of the antagonist are accomplished by selecting substitutions that differ significantly in their effect on maintaining (a) the structure of the polypeptide backbone in the area of the substitution, for example, as a sheet or helical conformation, (b) the charge or hydrophobicity of the molecule at the target site, or (c) the bulk of the side chain. Naturally occurring residues are divided into groups based on common side-chain properties:
(1) hydrophobic: norleucine, met, ala, val, leu, ile;
(2) neutral hydrophilic: cys, ser, thr;
(3) acidic: asp, glu;
(4) basic: asn, gln, his, lys, arg;
(5) residues that influence chain orientation: gly, pro; and
(6) aromatic: trp, tyr, phe.
Non-conservative substitutions will entail exchanging a member of one of these classes for another class.

Any cysteine residue not involved in maintaining the proper conformation of the antagonist also may be substituted, generally with serine, to improve the oxidative stability of the molecule and prevent aberrant crosslinking. Conversely, cysteine bond(s) may be added to the antagonist to improve its stability (particularly where the antagonist is an antibody fragment such as an Fv fragment).

A particularly preferred type of substitutional variant involves substituting one or more hypervariable region residues of a parent antibody. Generally, the resulting variant(s) selected for further development will have improved biological properties relative to the parent antibody from which they are generated. A convenient way for generating such substitutional variants is affinity maturation using phage display. Briefly, several hypervariable region sites (*e.g*. 6-7 sites) are mutated to generate all possible amino substitutions at each site. The antibody variants thus generated are displayed in a monovalent fashion from filamentous phage particles as fusions to the gene III product of M13 packaged within each particle. The phage-displayed variants are then screened for their biological activity (*e.g*. binding affinity) as herein disclosed. In order to identify candidate hypervariable region sites for modification, alanine scanning mutagenesis can be performed to identify hypervariable region residues contributing significantly to antigen binding. Alternatively, or in additionally, it may be beneficial to analyze a crystal structure of the antigen-antibody complex to identify contact points between the antibody and antigen. Such contact residues and neighboring residues are candidates for substitution according to the techniques elaborated herein. Once such variants are generated, the panel of variants is subjected to screening as described herein and antibodies with superior properties in one or more relevant assays may be selected for further development.

Another type of amino acid variant of the antagonist alters the original glycosylation pattern of the antagonist. By altering is meant deleting one or more carbohydrate moieties found in the antagonist, and/or adding one or more glycosylation sites that are not present in the antagonist.
Glycosylation of polypeptides is typically either N-linked or O-linked. N-linked refers to the attachment of the carbohydrate moiety to the side chain of an asparagine residue. The tripeptide sequences asparagine-X-serine and asparagine-X-threonine, where X is any amino acid except proline, are the recognition sequences for enzymatic attachment of the carbohydrate moiety to the asparagine side chain. Thus, the presence of either of these tripeptide sequences in a polypeptide creates a potential glycosylation site. O-linked glycosylation refers to the attachment of one of the sugars N-aceylgalactosamine, galactose, or xylose to a hydroxyamino acid, most commonly serine or threonine, although 5-hydroxyproline or 5-hydroxylysine may also be used.

Addition of glycosylation sites to the antagonist is conveniently accomplished by altering the amino acid sequence such that it contains one or more of the above-described tripeptide sequences (for N-linked glycosylation sites). The alteration may also be made by the addition of, or substitution by, one or more serine or threonine residues to the sequence of the original antagonist (for O-linked glycosylation sites).
Nucleic acid molecules encoding amino acid sequence variants of the antagonist are prepared by a variety of methods known in the art. These methods include, but are not limited to, isolation from a natural source (in the case of naturally occurring amino acid sequence variants) or preparation by oligonucleotide-mediated (or site-directed) mutagenesis, PCR mutagenesis, and cassette mutagenesis of an earlier prepared variant or a non-variant version of the antagonist.

It may be desirable to modify the antagonist of the invention with respect to effector function, *e.g*. so as to enhance antigen-dependent cell-mediated cyotoxicity (ADCC) and/or complement dependent cytotoxicity (CDC) of the antagonist. This may be achieved by introducing one or more amino acid substitutions in an Fc region of an antibody antagonist. Alternatively or additionally, cysteine residue(s) may be introduced in the Fc region, thereby allowing interchain disulfide bond formation in this region. The homodimeric antibody thus generated may have improved internalization capability and/or increased complement-mediated cell killing and antibody-dependent cellular cytotoxicity (ADCC). See Caron et al., J. Exp Med. 176:1191-1195 (1992) and Shopes, B. J. Immunol. 148:2918-2922 (1992). Homodimeric antibodies with enhanced anti-tumor activity may also be prepared using heterobifunctional cross-linkers as described in Wolff et al. Cancer Research 53:2560-2565 (1993). Alternatively, an antibody can be engineered which has dual Fc regions and may thereby have enhanced complement lysis and ADCC capabilities. See Stevenson et al. Anti-Cancer Drug Design 3:219-230 (1989).

To increase the serum half life of the antagonist, one may incorporate a salvage receptor binding epitope into the antagonist (especially an antibody fragment) as described in US Patent 5,739,277, for example. As used herein, the term "salvage receptor binding epitope" refers to an epitope of the Fc region of an IgG molecule (*e.g*., IgG₁, IgG₂, IgG₃, or IgG₄) that is responsible for increasing the *in vivo* serum half-life of the IgG molecule.

### Assays

Peripheral B-cell concentrations are determined by a FACS method that count CD3-/CD40+ cells. The percent of CD3-CD40+ B cells of total lymphocytes in samples can be obtained by the following gating strategy. The lymphocyte population is marked on the forward scatter/ side scatter scattergram to define Region 1 (R1). Using events in R1, fluorescence intensity dot plots are displayed for CD40 and CD3 markers. Fluorescently labeled isotype controls are used to determine respective cutoff points for CD40 and CD3 positivity.

### FACS analysis

Half million cells are washed and resuspended in 100 µl of FACS buffer, which is phosphate buffered saline with 1% BSA, containing 5 µl of staining or control antibody. All the staining antibodies, including isotype controls, are obtained from PharMingen, San Diego, CA. Human CD20 expression is assessed by staining with Rituxan® along with FITC-conjugated anti-human IgG1 secondary antibody. FACS analysis is conducted using FACScan and Cell Quest (Becton Dickinson Immunocytometry Systems, San Jose, CA). All the lymphocytes are defined in the forward and side light scatterings, while all the B lymphocytes are defined with the expression of B220 on the cell surface.

B cell depletion and recovery are assessed by analyzing peripheral B cell counts and analysis of hCD20+ B cells by FACS in the spleen, lymph node and bone marrow on a daily basis for the first week after injection and thereafter on a weekly basis. Serum levels of the injected 2H7 variant antibody are monitored.

### Pharmaceutical Formulations

Therapeutic formulations of the CD20-binding antibodies used in accordance with the present invention are prepared for storage by mixing an antibody having the desired degree of purity with optional pharmaceutically acceptable carriers, excipients or stabilizers (Remington's Pharmaceutical Sciences 16th edition, Osol, A. Ed. (1980)), in the form of lyophilized formulations or aqueous solutions. Acceptable carriers, excipients, or stabilizers are nontoxic to recipients at the dosages and concentrations employed, and include buffers such as phosphate, citrate, and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as olyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (*e.g*. Zn-protein complexes); and/or non-ionic surfactants such as TWEEN™, PLURONICS™ or polyethylene glycol (PEG).

Exemplary anti-CD20 antibody formulations are described in WO98/56418. Another formulation is a liquid multidose formulation comprising the anti-CD20 antibody at 40 mg/mL, 25 mM acetate, 150 mM trehalose, 0.9% benzyl alcohol, 0.02% polysorbate 20 at pH 5.0 that has a minimum shelf life of two years storage at 2-8°C. Another anti-CD20 formulation of interest comprises 10mg/mL antibody in 9.0 mg/mL sodium chloride, 7.35 mg/mL sodium citrate dihydrate, 0.7mg/mL polysorbate 80, and Sterile Water for Injection, pH 6.5. Yet another aqueous pharmaceutical formulation comprises 10-30 mM sodium acetate from about pH 4.8 to about pH 5.5, preferably at pH5.5, polysorbate as a surfactant in a an amount of about 0.01-0.1% v/v, trehalose at an amount of about 2-10% w/v, and benzyl alcohol as a preservative (U.S. 6,171,586). Lyophilized formulations adapted for subcutaneous administration are described in WO97/04801. Such lyophilized formulations may be reconstituted with a suitable diluent to a high protein concentration and the reconstituted formulation may be administered subcutaneously to the mammal to be treated herein.

One formulation for the humanized 2H7 variants is antibody at 12-14 mg/mL in 10 mM histidine, 6% sucrose, 0.02% polysorbate 20, pH 5.8. In a specific embodiment, 2H7 variants and in particular 2H7.v16 is formulated at 20mg/mL antibody in 10mM histidine sulfate, 60mg/ml sucrose., 0.2 mg/ml polysorbate 20, and Sterile Water for Injection, at pH5.8.

The formulation herein may also contain more than one active compound as necessary for the particular indication being treated, preferably those with complementary activities that do not adversely affect each other. For example, it may be desirable to further provide a cytotoxic agent, chemotherapeutic agent, cytokine or immunosuppressive agent (*e.g*. one which acts on T cells, such as cyclosporin or an antibody that binds T cells, *e.g*. one which binds LFA-1). The effective amount of such other agents depends on the amount of antibody present in the formulation, the type of disease or disorder or treatment, and other factors discussed above. These are generally used in the same dosages and with administration routes as described herein or about from 1 to 99% of the heretofore employed dosages.

The active ingredients may also be entrapped in microcapsules prepared, for example, by coacervation techniques or by interfacial polymerization, for example, hydroxymethylcellulose or gelatin-microcapsules and poly-(methylmethacylate) microcapsules, respectively, in colloidal drug delivery systems (for example, liposomes, albumin microspheres, microemulsions, nano-particles and nanocapsules) or in macroemulsions. Such techniques are disclosed in Remington's Pharmaceutical Sciences 16th edition, Osol, A. Ed. (1980).

Sustained-release preparations may be prepared. Suitable examples of sustained-release preparations include semi-permeable matrices of solid hydrophobic polymers containing the antagonist, which matrices are in the form of shaped articles, *e.g*. films, or microcapsules. Examples of sustained-release matrices include polyesters, hydrogels (for example, poly(2-hydroxyethyl-methacrylate), or poly(vinylalcohol)), polylactides (U.S. Pat. No. 3,773,919), copolymers of L-glutamic acid and ethyl-L-glutamate, non-degradable ethylene-vinyl acetate, degradable lactic acid-glycolic acid copolymers such as the LUPRON DEPOT™ (injectable microspheres composed of lactic acid-glycolic acid copolymer and leuprolide acetate), and poly-D-(-)-3-hydroxybutyric acid.

The formulations to be used for *in vivo* administration must be sterile. This is readily accomplished by filtration through sterile filtration membranes.

### Disease Treatment

### Diseases

The CD20 binding antibodies and BLyS antagonists disclosed herein are useful to treat B cell malignancies and B-cell regulated autoimmune disorders.

B-cell regulated autoimmune diseases include arthritis (rheumatoid arthritis, juvenile rheumatoid arthritis, osteoarthritis, psoriatic arthritis), psoriasis, dermatitis including atopic dermatitis; chronic autoimmune urticaria, polymyositis/dermatomyositis, toxic epidermal necrolysis, systemic scleroderma and sclerosis, responses associated with inflammatory bowel disease (IBD) (Crohn's disease, ulcerative colitis), respiratory distress syndrome, adult respiratory distress syndrome (ARDS), meningitis, allergic rhinitis, encephalitis, uveitis, colitis, glomerulonephritis, allergic conditions, eczema, asthma, conditions involving infiltration of T cells and chronic inflammatory responses, atherosclerosis, autoimmune myocarditis, leukocyte adhesion deficiency, systemic lupus erythematosus (SLE), lupus (including nephritis, non-renal, discoid, alopecia), juvenile onset diabetes, multiple sclerosis, allergic encephalomyelitis, immune responses associated with acute and delayed hypersensitivity mediated by cytokines and T-lymphocytes, tuberculosis, sarcoidosis, granulomatosis including Wegener's granulomatosis, agranulocytosis, vasculitis (including ANCA), aplastic anemia, Coombs positive anemia, Diamond Blackfan anemia, immune hemolytic anemia including autoimmune hemolytic anemia (AIHA), pernicious anemia, pure red cell aplasia (PRCA), Factor VIII deficiency, hemophilia A, autoimmune neutropenia, pancytopenia, leukopenia, diseases involving leukocyte diapedesis, CNS inflammatory disorders, multiple organ injury syndrome, myasthenia gravis, antigen-antibody complex mediated diseases, anti-glomerular basement membrane disease, anti-phospholipid antibody syndrome, allergic neuritis, Bechet disease, Castleman's syndrome, Goodpasture's Syndrome, Lambert-Eaton Myasthenic Syndrome, Reynaud's syndrome, Sjorgen's syndrome, Stevens-Johnson syndrome, solid organ transplant rejection (including pretreatment for high panel reactive antibody titers, IgA deposit in tissues, etc), graft versus host disease (GVHD), pemphigoid bullous, pemphigus (all including vulgaris, foliaceus), autoimmune polyendocrinopathies, Reiter's disease, stiff-man syndrome, giant cell arteritis, immune complex nephritis, IgA nephropathy, IgM polyneuropathies or IgM mediated neuropathy, idiopathic thrombocytopenic purpura (ITP), thrombotic throbocytopenic purpura (TTP), autoimmune thrombocytopenia, autoimmune disease of the testis and ovary including autoimune orchitis and oophoritis, primary hypothyroidism; autoimmune endocrine diseases including autoimmune thyroiditis, chronic thyroiditis (Hashimoto's Thyroiditis), subacute thyroiditis, idiopathic hypothyroidism, Addison's disease, Grave's disease, autoimmune polyglandular syndromes (or polyglandular endocrinopathy syndromes), Type I diabetes also referred to as insulin-dependent diabetes mellitus (IDDM) and Sheehan's syndrome; autoimmune hepatitis, Lymphoid interstitial pneumonitis (HIV), bronchiolitis obliterans (non-transplant) vs NSIP, Guillain-Barre' Syndrome, Large Vessel Vasculitis (including Polymyalgia Rheumatica and Giant Cell (Takayasu's) Arteritis), Medium Vessel Vasculitis (including Kawasaki's Disease and Polyarteritis Nodosa), ankylosing spondylitis, Berger's Disease (IgA nephropathy), Rapidly Progressive Glomerulonephritis, Primary biliary cirrhosis, Celiac sprue (gluten enteropathy), Cryoglobulinemia, ALS, coronary artery disease.

The B cell neoplasms include CD20-positive Hodgkin's disease including lymphocyte predominant Hodgkin's disease (LPHD); non-Hodgkin's lymphoma (NHL); follicular center cell (FCC) lymphomas; acute lymphocytic leukemia (ALL); chronic lymphocytic leukemia (CLL); Hairy cell leukemia. The non-Hodgkins lymphoma include low grade/follicular non-Hodgkin's lymphoma (NHL), small lymphocytic (SL) NHL, intermediate grade/follicular NHL, intermediate grade diffuse NHL, high grade immunoblastic NHL, high grade lymphoblastic NHL, high grade small non-cleaved cell NHL, bulky disease NHL, plasmacytoid lymphocytic lymphoma, mantle cell lymphoma, AIDS- related lymphoma and Waldenstrom's macroglobulinemia. Treatment of relapses of these cancers are also contemplated. LPHD is a type of Hodgkin's disease that tends to relapse frequently despite radiation or chemotherapy treatment and is characterized by CD20-positive malignant cells. CLL is one of four major types of leukemia. A cancer of mature B-cells called lymphocytes, CLL is manifested by progressive accumulation of cells in blood, bone marrow and lymphatic tissues. Indolent lymphoma is a slow-growing, incurable disease in which the average patient survives between six and 10 years following numerous periods of remission and relapse.

BLyS antagonists and CD20 binding antibodies may be used to treat non-Hodgkin's lymphoma (NHL), lymphocyte predominant Hodgkin's disease (LPHD), chronic lymphocytic leukemia (CLL), small lymphocytic lymphoma (SLL) which is a type of non-Hodgkin's lymphoma (NHL), rheumatoid arthritis and juvenile rheumatoid arthritis, systemic lupus erythematosus (SLE) including lupus nephritis, Wegener's disease, inflammatory bowel disease, idiopathic thrombocytopenic purpura (ITP), thrombotic throbocytopenic purpura (TTP), autoimmune thrombocytopenia, multiple sclerosis, psoriasis, IgA nephropathy, IgM polyneuropathies, myasthenia gravis, vasculitis, diabetes mellitus, Reynaud's syndrome, Sjorgen's syndrome and glomerulonephritis.

The desired level of B cell depletion will depend on the disease. For the treatment of a CD20 positive cancer, it may be desirable to maximize the depletion of the B cells which are the target of the anti-CD20 antibodies of the invention. Thus, for the treatment of a CD20 positive B cell neoplasm, it is desirable that the B cell depletion be sufficient to at least prevent progression of the disease which can be assessed by the physician of skill in the art, e.g., by monitoring tumor growth (size), proliferation of the cancerous cell type, metastasis, other signs and symptoms of the particular cancer. Preferably, the B cell depletion is sufficient to prevent progression of disease for at least 2 months, more preferably 3 months, even more preferably 4 months, more preferably 5 months, even more preferably 6 or more months. In even more preferred embodiments, the B cell depletion is sufficient to increase the time in remission by at least 6 months, more preferably 9 months, more preferably one year, more preferably 2 years, more preferably 3 years, even more preferably 5 or more years. In a most preferred embodiment, the B cell depletion is sufficient to cure the disease. In preferred embodiments, the B cell depletion in a cancer patient is at least about 75% and more preferably, 80%, 85%, 90%, 95%, 99% and even 100% of the baseline level before treatment.

For treatment of an autoimmune disease, it may be desirable to modulate the extent of B cell depletion depending on the disease and/or the severity of the condition in the individual patient, by adjusting the dosage of CD20 binding antibody. Thus, B cell depletion can but does not have to be complete. Or, total B cell depletion may be desired in initial treatment but in subsequent treatments, the dosage may be adjusted to achieve only partial depletion. In one.embodiment, the B cell depletion is at least 20%, i.e., 80% or less of CD20 positive B cells remain as compared to the baseline level before treatment. In other embodiments, B cell depletion is 25%, 30%, 40%, 50%, 60%, 70% or greater. Preferably, the B cell depletion is sufficient to halt progression of the disease, more preferably to alleviate the signs and symptoms of the particular disease under treatment, even more preferably to cure the disease.

Publications concerning therapy with Rituximab include: Perotta and Abuel "Response of chronic relapsing ITP of 10 years duration to Rituximab" Abstract # 3360 Blood 10(1)(part 1-2): p. 88B (1998); Stashi et al. "Rituximab chimeric anti-CD20 monoclonal antibody treatment for adults with chronic idopathic thrombocytopenic purpura" Blood 98(4):952-957 (2001); Matthews, R. "Medical Heretics" New Scientist (7 April, 2001); Leandro et al. "Clinical outcome in 22 patients with rheumatoid arthritis treated with B lymphocyte depletion" Ann Rheum Dis 61:833-888 (2002); Leandro et al. "Lymphocyte depletion in rheumatoid arthritis: early evidence for safety, efficacy and dose response. Arthritis and Rheumatism 44(9): S370 (2001); Leandro et al. "An open study of B lymphocyte depletion in systemic lupus erythematosus", Arthritis & Rheumatism 46(1):2673-2677 (2002); Edwards and Cambridge "Sustained improvement in rheumatoid arthritis following a protocol designed to deplete B lymphocytes" Rheumatology 40:205-211 (2001); Edwards et al. "B-lymphocyte depletion therapy in rheumatoid arthritis and other autoimmune disorders" Biochem. Soc. Trans. 30(4):824-828 (2002); Edwards et al. "Efficacy and safety of Rituximab, a B-cell targeted chimeric monoclonal antibody: A randomized, placebo controlled trial in patients with rheumatoid arthritis. Arthritis and Rheumatism 46(9): S 197 (2002); Levine and Pestronk "IgM antibody-related polyneuropathies: B-cell depletion chemotherapy using Rituximab" Neurology 52: 1701-1704 (1999); DeVita et al. "Efficacy of selective B cell blockade in the treatment of rheumatoid arthritis" Arthritis & Rheum 46:2029-2033 (2002); Hidashida et al. "Treatment of DMARD-Refractory rheumatoid arthritis with rituximab." Presented at the Annual Scientific Meeting of the American College of Rheumatology; Oct 24-29; New Orleans, LA 2002; Tuscano, J. "Successful treatment of Infliximab-refractory rheumatoid arthritis with rituximab" Presented at the Annual Scientific Meeting of the American College of Rheumatology; Oct 24-29; New Orleans, LA 2002.

For therapeutic applications, the anti-CD20 antibody and BLyS antagonist compositions disclosed herein can be used in combination therapy with, e.g., chemotherapeutic agents, hormones, antiangiogens, radiolabelled compounds, or with surgery, cryotherapy, and/or radiotherapy. The preceding treatment methods can be administered in conjunction with other forms of conventional therapy, either consecutively with, pre- or post-conventional therapy. The anti-CD20 antibody and BLyS antagonist will be administered with a therapeutically effective dose of the chemotherapeutic agent. The anti-CD20 antibody and BLyS antagonist are administered in conjunction with chemotherapy to enhance the activity and efficacy of the chemotherapeutic agent. The Physicians' Desk Reference (PDR) discloses dosages of chemotherapeutic agents that have been used in the treatment of various cancers. The dosing regimen and dosages of these aforementioned chemotherapeutic drugs that are therapeutically effective will depend on the particular cancer being treated, the extent of the disease and other factors familiar to the physician of skill in the art and can be determined by the physician.

A patient is alleviated or successfully treated of a B cell neoplasm or a B cell regulated autoimmune diseases if there is a measurable improvement in the symptoms or other applicable criteria after administration of the compositions of the invention compared to before treatment. The effect of treatment may be apparent within 3-10 weeks after administration of the compositions disclosed herein. The applicable criteria for each disease will be well known to the physician of skill in the appropriate art. For example, the physician can monitor the treated patient for clinical, or serologic evidence of disease such as serologic markers of disease, complete blood count including B cell count, and serum immunoglobulin levels. Serum levels of IgG and IgM are reduced in BR3-Fc treated mice. It is expected that human patients responding to BR3-Fc or anti-CD20 antibody treatment or both would likewise show a reduction in serum IgG and IgM levels. The patient may show observable and/or measurable reduction in or absence of one or more of the following: reduction in the number of cancer cells or absence of the cancer cells; reduction in the tumor size; inhibition (*i.e*., slow to some extent and preferably stop) of cancer cell infiltration into organs; inhibition (*i.e*., slow to some extent and preferably stop) of tumor metastasis; inhibition, to some extent, of tumor growth; and/or relief to some extent, one or more of the symptoms associated with the specific cancer; reduced morbidity and mortality, and improvement in quality of life issues. Preferably, after administration of the compositions disclosed herein, the improvement is at least 20 % over the baseline for a particular symptom or criterion taken before treatment by the methods of the invention, more preferably, 25-30%, even more preferably 30-35%, most preferably 40% and above.

The parameters for assessing efficacy or success of treatment of the neoplasm will be known to the physician of skill in the appropriate disease. Generally, the physician of skill will look for reduction in the signs and symptoms of the specific disease. Parameters can include median time to disease progression, time in remission and stable disease. For B cell neoplasms, measurable criteria may include, e.g., time to disease progression, an increase in duration of overall and/or progression-free survival. In the case of leukemia, a bone marrow biopsy can be conducted to determine the degree of remission. Complete remission can be defined as the leukemia cells making up less than 5 percent of all cells found in a patient's bone marrow 30 days following treatment.

The following references describe lymphomas and CLL, their diagnoses, treatment and standard medical procedures for measuring treatment efficacy. Canellos GP, Lister, TA, Sklar JL: The Lymphomas. W.B.Saunders Company, Philadelphia, 1998; van Besien K and Cabanillas, F: Clinical Manifestations, Staging and Treatment of Non-Hodgkin's Lymphoma, Chap. 70, pp 1293-1338, in: Hematology , Basic Principles and Practice, 3rd ed. Hoffman et al. (editors). Churchill Livingstone, Philadelphia, 2000; and Rai, K and Patel, D:Chronic Lymphocytic Leukemia, Chap. 72, pp 1350-1362, in: Hematology, Basic Principles and Practice, 3rd ed. Hoffman et al. (editors). Churchill Livingstone, Philadelphia, 2000.

The parameters for assessing efficacy or success of treatment of an autoimmune or autoimmune related disease will be known to the physician of skill in the appropriate disease. Generally, the physician of skill will look for reduction in the signs and symptoms of the specific disease. The following are by way of examples.

Rheumatoid arthritis (RA) is an autoimmune disorder of unknown etiology. Most RA patients suffer a chronic course of disease that, even with therapy, may result in progressive joint destruction, deformity, disability and even premature death. The goals of RA therapy are to prevent or control joint damage; prevent loss of function and decrease pain. Initial therapy of RA usually involves administration of one or more of the following drugs: nonsteroidal anti-inflammatory drugs (NSAIDs), glucocorticoid (via joint injection), and low-dose prednisone. See "Guidelines for the management of rheumatoid arthritis" Arthritis & Rheumatism 46(2): 328-346 (February, 2002). The majority of patients with newly diagnosed RA are started with disease-modifying antirheumatic drug (DMARD) therapy within 3 months of diagnosis. DMARDs commonly used in RA are hydroxycloroquine, sulfasalazine, methotrexate, leflunomide, etanercept, infliximab (plus oral and subcutaneous methrotrexate), azathioprine, D-penicillamine, Gold (oral), Gold (intramuscular), minocycline, cyclosporine, Staphylococcal protein A immunoadsorption.

Because the body produces tumor necrosis factor alpha (TNFα) during RA, TNFα inhibitors have used for therapy of that disease. Etanercept (ENBREL®) is an injectable drug approved in the US for therapy of active RA. Etanercept binds to TNFα and serves to remove most TNFα from joints and blood, thereby preventing TNFα from promoting inflammation and other symptoms of rheumatoid arthritis. Etanercept is an "immunoadhesin" fusion protein consisting of the extracellular ligand binding portion of the human 75 kD (p75) tumor necrosis factor receptor (TNFR) linked to the Fc portion of a human IgG1. Infliximab, sold under the trade name REMICADE®, is an immune-suppressing drug prescribed to treat RA and Crohn's disease. Infliximab is a chimeric monoclonal antibody that binds to TNFα and reduces inflammation in the body by targeting and binding to TNFα which produces inflammation.

Adalimumab (HUMIRA™, Abbott Laboratories), previously known as D2E7, is a human monoclonal antibody that binds to TNFα and is approved for reducing the signs and symptoms and inhibiting the progression of structural damage in adults with moderately to severely active RA who have had insufficient response to one or more traditional disease modifying DMARDs.

Treatment of rheumatoid arthritis by administering an anti-CD20 antibody and a BLyS antagonist can be preformed in conjunction with therapy with one or more of the aforementioned drugs for RA.

For rheumatoid arthritis, for example, measurements for progress in treatment may include the number of swollen and tender joints and the length of morning stiffness. Patients may be examined for how much the joint in the hands and feet have eroded by using X-rays and a scoring system known as the Sharp score. Another scoring system is based on the American College of Rheumatology criteria for assessing response to therapies.

One method of evaluating treatment efficacy in RA is based on American College of Rheumatology (ACR) criteria, which measures the percentage of improvement in tender and swollen joints, among other things. The RA patient can be scored at for example, ACR 20 (20 percent improvement) compared with no antibody treatment (e.g,, baseline before treatment) or treatment with placebo. Other ways of evaluating the efficacy of antibody treatment include X-ray scoring such as the Sharp X-ray score used to score structural damage such as bone erosion and joint space narrowing. Patients can also be evaluated for the prevention of or improvement in disability based on Health Assessment Questionnaire [HAQ] score, AIMS score, SF-36 at time periods during or after treatment. The ACR 20 criteria may include 20% improvement in both tender (painful) joint count and swollen joint count plus a 20% improvement in at least 3 of 5 additional measures:
1. patient's pain assessment by visual analog scale (VAS),
2. patient's global assessment of disease activity (VAS),
3. physician's global assessment of disease activity (VAS),
4. patient's self-assessed disability measured by the Health Assessment Questionnaire, and
5. acute phase reactants, CRP or ESR.
The ACR 50 and 70 are defined analogously. Preferably, the patient is administered an amount of a CD20 binding antibody of the invention effective to achieve at least a score of ACR 20, preferably at least ACR 30, more preferably at least ACR50, even more preferably at least ACR70, most preferably at least ACR 75 and higher.

Psoriatic arthritis has unique and distinct radiographic features. For psoriatic arthritis, joint erosion and joint space narrowing can be evaluated by the Sharp score as well. The humanized CD20 binding antibodies disclosed herein can be used to prevent the joint damage as well as reduce disease signs and symptoms of the disorder.

Disclosed herein is a method of treating Lupus or SLE by administering to the patient suffering from SLE, a therapeutically effective amount of a humanized CD20 binding antibody of the invention. SLEDAI scores provide a numerical quantitation of disease activity. The SLEDAI is a weighted index of 24 clinical and laboratory parameters known to correlate with disease activity, with a numerical range of 0-103. see Bryan Gescuk & John Davis, "Novel therapeutic agent for systemic lupus erythematosus" in Current Opinion in Rheumatology 2002, 14:515-521. Antibodies to double-stranded DNA are believed to cause renal flares and other manifestations of lupus. Patients undergoing antibody treatment can be monitored for time to renal flare, which is defined as a significant, reproducible increase in serum creatinine, urine protein or blood in the urine. Alternatively or in addition, patients can be monitored for levels of antinuclear antibodies and antibodies to double-stranded DNA. Treatments for SLE include high-dose corticosteroids and/or cyclophosphamide (HDCC).

Spondyloarthropathies are a group of disorders of the joints, including ankylosing spondylitis, psoriatic arthritis and Crohn's disease. Treatment success can be determined by validated patient and physician global assessment measuring tools.

For systemic lupus erythematosus, patients can be monitored for levels of antinuclear antibodies and antibodies to double-stranded DNA.

Various medications are used to treat psoriasis; treatment differs directly in relation to disease severity. Patients with a more mild form of psoriasis typically utilize topical treatments, such as topical steroids, anthralin, calcipotriene, clobetasol, and tazarotene, to manage the disease while patients with moderate and severe psoriasis are more likely to employ systemic (methotrexate, retinoids, cyclosporine, PUVA and UVB) therapies. Tars are also used. These therapies have a combination of safety concerns, time consuming regimens, or inconvenient processes of treatment. Furthermore, some require expensive equipment and dedicated space in the office setting. Systemic medications can produce serious side effects, including hypertension, hyperlipidemia, bone marrow suppression, liver disease, kidney disease and gastrointestinal upset. Also, the use of phototherapy can increase the incidence of skin cancers. In addition to the inconvenience and discomfort associated with the use of topical therapies, phototherapy and systemic treatments require cycling patients on and off therapy and monitoring lifetime exposure due to their side effects.

Treatment efficacy for psoriasis is assessed by monitoring changes in clinical signs and symptoms of the disease including Physician's Global Assessment (PGA) changes and Psoriasis Area and Severity Index (PASI) scores, Psoriasis Symptom Assessment (PSA), compared with the baseline condition. The patient can be measured periodically throughout treatment on the Visual analog scale used to indicate the degree of itching experienced at specific time points.

### Dosing

Depending on the indication to be treated and factors relevant to the dosing that a physician of skill in the field would be familiar with, the BLyS antagonists and CD20 binding antibodies disclosed herein will be administered at a dosage that is efficacious for the treatment of that indication while minimizing toxicity and side effects. For the treatment of patients suffering from B-cell neoplasm such as non-Hodgkins lymphoma, anti-CD20 antibodies disclosed herein will be administered to a human patient at a dosage range of 1mg/kg to 20mg/kg body weight, preferably at 2.5mg/kg to 10mg/kg. The anti-CD20 antibody may be administered at a dosage of 10mg/kg or 375mg/m². For treating NHL, one dosing regimen would be to administer 375mg/m² of anti-CD20 antibody every other week for 2-4 doses, or one dose of the antibody composition in the first week of treatment, followed by a 2 week interval, then a second dose of the same amount of antibody is administered. Generally, NHL patients receive such treatment once during a year but upon recurrence of the lymphoma, such treatment can be repeated. In the treatment of NHL, the anti-CD20 antibody plus BLyS antagonist therapy can be combined with chemotherapy such as with CHOP. For the treatment of B cell neoplasms such as CLL or SLL, patients may receive four weekly doses of Rituxan at 375 mg/m² after or before administration with BR3-Fc.with relapsed CLL. For CLL, treatment with the anti-CD20 antibody and BLyS antagonists can be combined with chemotherapy, for example, with fludarabine and cytoxan.

For treating rheumatoid arthritis, Rituxan^{™} which is a chimeric antibody may be administered at 500mg per dose every other week for a total of 2 doses. A humanized anti-CD20 antibody, e.g., hu2H7v.16 or any other variant of hu 2H7 as disclosed herein, can be administered at less than 500mg per dose such as at between about 200-500mg per dose, between about 250mg-450mg, or 300-400mg per dose, for 2-4 doses every other week or every 3rd week.

BR3-Fc can be administered at a dosage range of 0.5mg/kg to 10mg/kg, preferably 1mg/kg to 5mg/kg, more preferably, 1.5mg/kg to 2.5mg/kg. BR3-Fc may be administered at 5mg/kg every other day from day 1 to day 12 of treatment. Also contemplated is dosing at about 2-5mg/kg every 2-3 days for a total of 2-5 doses.

The treatment methods disclosed herein comprise a combination of concurrently and sequentially administering the anti-CD20 antibody and the BLyS antagonist (both referred to herein as the drugs). In sequential administration, the drugs can be administered in either order, i.e., BLyS antagonist first followed by anti-CD20 antibody. The patient is treated with one drug and monitored for efficacy before treatment with the one drug. For example, if the BLyS antagonist produces a partial response, treatment can be followed with the anti-CD20 antibody to achieve a full response, and vice versa. The BR3-Fc which is an immunoadhesin, has a shorter half compared to a full length anti-CD20 antibody. For the treatment of autoimmune diseases such as rheumatoid arthritis, if the anti-CD20 antibody is Rituxan and the BLyS antagonist is-BR3-Fc, the patient in need thereof may receive BR3-Fc prior to treatment with Rituxan. Alternatively, the patient can be initially administered both drugs and subsequent dosing can be with only one or the other drug.

To condition the patient to tolerate the drugs and/or to reduce the occurrence of adverse effects such as infusion-related symptoms which arise from the initial and subsequent administrations of the therapeutic compound, the mammal in need thereof can be administered a first or initial conditioning dose of one or both drugs and then administered at least a second therapeutically effective dose of one or both drugs wherein the second and any subsequent doses are higher than the first dose. The first dose serves to condition the mammal to tolerate the higher second therapeutic dose. In this way, the mammal is able to tolerate higher doses of the therapeutic compound than could be administered initially. A "conditioning dose" is a dose which attenuates or reduces the frequency or the severity of first dose adverse side effects associated with administration of a therapeutic compound. The conditioning dose may be a therapeutic dose, a sub-therapeutic dose, a symptomatic dose or a sub-symptomatic dose. A therapeutic dose is a dose which exhibits a therapeutic effect on the patient and a sub-therapeutic dose is a dose which dose not exhibit a therapeutic effect on the patient treated. A symptomatic dose is a dose which induces at least one adverse effect on administration and a sub-symptomatic dose is a dose which does not induce an adverse effect. Some adverse effects are fever, headache, nausea, vomiting, breathing difficulties, myalgia, and chills.

### Route of administration

The BLyS antagonists and the anti-CD20 antibodies may be administered to a human patient in accord with known methods, such as by intravenous administration, *e.g*., as a bolus or by continuous infusion over a period of time, by subcutaneous, intramuscular, intraperitoneal, intracerobrospinal, intra-articular, intrasynovial, intrathecal, or inhalation routes. The anti-CD20 antibody will generally be administered by intravenous or subcutaneous administration. The drugs can be administered by the same or different route.

### Articles of Manufacture and Kits

Disclosed herein is an article of manufacture comprising a BLyS antagonist and an anti-CD20 antibody useful for the treatment of a B cell based malignancy or a B-cell regulated autoimmune disorder disclosed above. In a specific embodiment, the article of manufacture contains the BR3-Fc of SEQ ID 2, and the hu2H7v. 16 antibody, for the treatment of non-Hodgkin's lymphoma.

The article of manufacture comprises at least one container and a label or package insert on or associated with the container. Suitable containers include, for example, bottles, vials, syringes, etc. The containers may be formed from a variety of materials such as glass or plastic. The container holds a composition which is effective for treating the condition and may have a sterile access port (for example the container may be an intravenous solution bag or a vial having a stopper pierceable by a hypodermic injection needle). At least one active agent in the composition is a CD20 binding antibody such as Rituxan^{™} or hu2H7v.16, and the other active agent is a BLyS antagonist such as BR3-Fc. The label or package insert indicates that the composition is used for treating the particular condition, e.g., non-Hodgkin's lymphoma or rheumatoid arthritis. The label or package insert will further comprise instructions for administering the composition to the patient. Additionally, the article of manufacture may further comprise a second container comprising a pharmaceutically-acceptable buffer, such as bacteriostatic water for injection (BWFI), phosphate-buffered saline, Ringer's solution and dextrose solution. It may further include other materials desirable from a commercial and user standpoint, including other buffers, diluents, filters, needles, and syringes.

Kits are also disclosed herein that are useful for various purposes, e.g., for B-cell killing assays. As with the article of manufacture, the kit comprises a container and a label or package insert on or associated with the container. The container holds a composition comprising at least one anti-CD20 antibody and one BLyS antagonist disclosed herein. Additional containers may be included that contain, e.g., diluents and buffers, control antibodies. The label or package insert may provide a description of the composition as well as instructions for the intended in vitro or diagnostic use.

### Experimental Examples

### Example 1

### Development of monoclonal antibodies to CD20

Six Balb/c mice, three Lewis rats (Charles River Laboratories, Hollister, CA) and three Armenian hamsters (Cytogen Research and Development, Inc., Boston, MA) were hyperimmunized with adenovirus-infected human 293 cells transiently expressing murine CD20 (Genentech, Inc., South San Francisco, CA), in phosphate buffered saline (PBS). Pre-fusion boosts, consisting of murine cells expressing endogenous levels of murine CD20 and purified, purified recombinant mCD20 expressed in *E. coli* (Genentech, Inc., South San Francisco, CA), were administered three days prior to fusion. B-cells from all animals were fused with mouse myeloma cells (X63.Ag8.653; American Type Culture Collection, Manassas, VA) using a modified protocol analogous to one previously described (Kohler and Milstein, 1975; Hongo et al., 1995). After 10-14 days, the supernatants from the mouse and hamster fusions were harvested and screened for anti-murine CD20 and anti-human CD20 antibody production by direct enzyme-linked immunosorbent assay (ELISA). The mCD20 ELISA screen identified 59 positive hybridomas from the hamster fusion (2 crossreactive with hCD20) and 1 positive hybridoma from the mouse fusion (crossreactive with hCD20). Positive clones, showing the highest immunobinding after subcloning by limiting dilution, are either injected into Pristane-primed mice (Freund and Blair, 1982) for *in vivo* production of Mab or cultured in vitro. The ascites fluids and/or supernatants are pooled and purified by affinity chromatography (Pharmacia fast protein liquid chromatography [FPLC]; Pharmacia, Uppsala, Sweden) as previously described (Hongo et al., 1995) or using a modified version of the protocol previously described. The purified antibody preparations are sterile filtered (0.2-µm pore size; Nalgene, Rochester NY) and stored at 4°C in PBS.

### Direct ELISA for the evaluation of immune sera

Microtiter plates (NUNC) were coated with 100 µl/well of murine or human CD20 (1 µg/ml; Genentech, Inc., South San Francisco, CA) in 0.05 M carbonate buffer, pH 9.6. Coated plates were washed three times with ELISA wash buffer (PBS/0.05% Tween 20) and blocked for at least 1 hr with PBS containing 0.5% bovine serum albumin and 0.05% Tween 20 (PBS/BSA/T20). Blocking buffer was then removed and 100 µl of diluted samples and controls were added and incubated for 1 hr at ambient temperature. The plates were then washed and horseradish peroxidase conjugated species specific anti-IgG conjugate (Sigma, St. Louis, MO or ICN Cappel, Durham, NC) was added (100 µl/well) and incubated for 1 hr at ambient temperature. The plates were washed and incubated with tetramethylbenzidine substrate (BioFX Laboratories, Owings Mills, MD) for 5-10 minutes followed by the addition of Stop Solution (100 µl/well; BioFX Laboratories). The plates were then read using an automated plate reader (EL808, BioTek Instruments, Inc., Winooski, VT).

### References:

Hongo, J.S., Mora-Worms, M., Lucas, C. and Fendly, B.M.: Development and characterization of murine monoclonal antibodies to the latency-associated peptide of transforming growth factor B1. Hybridoma 1995; 14:253-260.
Kohler, G. and Milstein, C.: Continuous cultures of fused cells secreting antibody of predefined specificity. Nature 1975; 256: 495-497.
Freund YR and Blair PB: Depression of natural killer activity and mitogen responsiveness in mice treated with pristane. J Immunol 1982; 129:2826-2830.

### Example 2

### Humanization of 2H7 anti-CD20 murine monoclonal antibody

Humanization of the murine anti-human CD20 antibody, 2H7 (also referred to herein as m2H7, m for murine), was carried out in a series of site-directed mutagenesis steps. The CDR residues of 2H7 were identified by comparing the amino acid sequence of the murine 2H7 variable domains (disclosed in U.S. 5,846,818) with the sequences of known antibodies (Kabat et al., Sequences of proteins of immunological interest, Ed. 5. Public Health Service, National Institutes of Health, Bethesda, MD (1991)). The CDR regions for the light and heavy chains were defined based on sequence hypervariability (Kabat et al., *supra*) and are shown in Fig. 12 and Fig. 13, respectively. Using synthetic oligonucleotides (Table 2), site-directed mutagenesis (Kunkel, Proc. Natl. Acad. Sci. 82:488-492 (1985)) was used to introduce all six of the murine 2H7 CDR regions into a complete human Fab framework corresponding to a consensus sequence V_{κ}I,V_{H}III (V_{L} kappa subgroup I, V_{H} subgroup III) contained on plasmid pVX4. The phagemid pVX4 was used for mutagenesis as well as for expression of F(ab)s in E. coli. Based on the phagemid pb0720, a derivative of pB0475 (Cunningham et al., Science 243: 1330-1336 (1989)), pVX4 contains a DNA fragment encoding a humanized consensus κ-subgroup I light chain (V_{L}κI-C_{L}) and a humanized consensus subgroup III heavy chain (V_{H}III-C_{H}1) anti-IFN-α (interferon α) antibody. pVX4 also has an alkaline phosphatase promotor and Shine-Dalgarno sequence both derived from another previously described pUC119-based plasmid, pAK2 (Carter et al., Proc. Natl. Acad. Sci. USA 89: 4285 (1992)). A unique Spel restriction site was introduced between the DNA encoding for the F(ab) light and heavy chains. The first 23 amino acids in both anti-IFN-α heavy and light chains are the *StII* secretion signal sequence (Chang et al., Gene 55: 189-196 (1987)).

To construct the CDR-swap version of 2H7 (2H7.v2), site-directed mutagenesis was performed on a deoxyuridine-containing template of pVX4; all six CDRs of anti-IFN-α were changed to the murine 2H7 CDRs. The resulting molecule is referred to as humanized 2H7 version 2 (2H7.v2), or the "CDR-swap version" of 2H7; it has the m2H7 CDR residues with the consensus human FR residues shown in Figures 12 and 13. Humanized 2H7.v2 was used for further humanization.

Table 2 shows the oligonucleotide sequence used to create each of the murine 2H7 (m2H7) CDRs in the H and L chain. For example, the CDR-H1 oligonucleotide was used to recreate the m2H7 H chain CDR1. CDR-H1, CDR-H2 and CDR-H3 refers to the H chain CDR1, CDR2 and CDR3, respectively; similarly, CDR-L1, CDR-L2 and CDR-L3 refers to each of the L chain CDRs. The substitutions in CDR-H2 were done in two steps with two oligonucleotides, CDR-H2A and CDR-H2B.

**Table 2. Oligonucleotide sequences used for construction of the CDR-swap of murine 2H7 CDRs into a human framework in pVX4. Residues changed by each oligonucleotide are underlined.**

| Substitution | Oligonucleotide sequence |
|---|---|
| CDR-H1 | C TAC ACC TTC ACG AGC TAT AAC ATG CAC TGG GTC CG (SEQ ID NO.) |
| CDR-H2A | G ATT AAT CCT GAC AAC GGC GAC ACG AGC TAT AAC CAG AAG TTC AAG GGC CG (SEQ ID NO._) |
| CDR-H2B | GAA TGG GTT GCA GCG ATC TAT CCT GGC AAC GGC GAC AC (SEQ ID NO._) |
| CDR-H3 | AT TAT TGT GCT CGA GTG GTC TAC TAT AGC AAC AGC TAC TGG TAC TTC GAC GTC TGG GGT CAA GGA (SEQ ID NO._) |
| CDR-L1 | C TGC ACA GCC AGC TCT TCT GTC AGC TAT ATG CAT TG (SEQ ID NO._) |
| CDR-L2 | AA CTA CTG ATT TAC GCT CCA TCG AAC CTC GCG TCT GGA GTC C (SEQ ID NO._) |
| CDR-L3 | TAT TAC TGT CAA CAG TGG AGC TTC AAT CCG CCC ACA TTT GGA CAG (SEQ ID NO._) |

Based on a sequence comparison of the murine 2H7 framework residues with the human V_{κ}I,V_{H}III consensus framework (Figures 12 and 13) and previously humanized antibodies (Carter et al., Proc. Natl. Acad. Sci. USA 89:4285-4289 (1992)), several framework mutations were introduced into the 2H7.v2 Fab construct by site-directed mutagenesis. These mutations result in a change of certain human consensus framework residues to those found in the murine 2H7 framework, at sites that might affect CDR conformations or antigen contacts. Version 3 contained V_{H}(R71V, N73K), version 4 contained V_{H}(R71V), version 5 contained V_{H}(R71V, N73K) and V_{L}(L46P), and version 6 contained V_{H}(R71V, N73K) and V_{L}(L46P, L47W).

Humanized and chimeric Fab versions of m2H7 antibody were expressed in E. coli and purified as follows. Plasmids were transformed into E. coli strain XL-1 Blue (Stratagene, San Diego, CA) for preparation of double-and single-stranded DNA. For each variant, both light and heavy chains were completely sequenced using the dideoxynucleotide method (Sequenase, U.S. Biochemical Corp.). Plasmids were transformed into E. coli strain 16C9, a derivative of MM294, plated onto LB plates containing 5 µg/ml carbenicillin, and a single colony selected for protein expression. The single colony was grown in 5 ml LB-100 µg/ml carbenicillin for 5-8 h at 37° C. The 5 ml culture was added to 500 ml AP5-100 µg/ml carbenicillin and allowed to grow for 16 h in a 4 L baffled shake flask at 37°C. AP5 media consists of: 1.5g glucose, 11.0 Hycase SF, 0.6g yeast extract (certified), 0.19g anhydrous MgSO₄, 1.07g NH₄Cl, 3.73g KCl, 1.2g NaCl, 120 ml 1 M triethanolamine, pH 7.4, to 1 L water and then sterile filtered through 0.1 µm Sealkeen filter.

Cells were harvested by centrifugation in a 1 L centrifuge bottle (Nalgene) at 3000xg and the supernatant removed. After freezing for 1 h, the pellet was resuspended in 25 ml cold 10 mM MES-10 mM EDTA, pH 5.0 (buffer A). 250 µl of 0.1M PMSF (Sigma) was added to inhibit proteolysis and 3.5 ml of stock 10 mg/ml hen egg white lysozyme (Sigma) was added to aid lysis of the bacterial cell wall. After gentle shaking on ice for 1 h, the sample was centrifuged at 40,000xg for 15 min. The supernatant was brought to 50 ml with buffer A and loaded onto a 2 ml DEAE column equilibrated with buffer A. The flow-through was then applied to a protein G-Sepharose CL-4B (Pharmacia) column (0.5 ml bed volume) equilibrated with buffer A. The column was washed with 10 ml buffer A and eluted with 3 ml 0.3 M glycine, pH 3.0, into 1.25 ml 1 M Tris, pH 8.0. The F(ab) was then buffer exchanged into PBS using a Centricon-30 (Amicon) and concentrated to a final volume of 0.5 ml. SDS-PAGE gels of all F(ab)s were run to ascertain purity and the molecular weight of each variant was verified by electrospray mass spectrometry.

Plasmids for expression of full-length IgG's were constructed by subcloning the V_{L} and V_{H} domains of chimeric Fab as well as humanized Fab of hu2H7 antibodies into previously described pRK vectors for mammalian cell expression (Gorman et al., DNA Prot. Eng. Tech. 2:3-10 (1990)). Briefly, each Fab construct was digested with *EcoR*V and *Blp*I to excise a V_{L} fragment, which was cloned into the *EcoRV*/*Blp*I sites of plasmid pDR1 for expression of the complete light chain (V_{L}-C_{L} domains). Additionally, each Fab construct was digested with *Pvu*II and *Apa*I to excise a V_{H} fragment, which was cloned into the *Pvu*II/*Apa*I sites of plasmid pDR2 for expression of the complete heavy chain (VH-CH₁-CH₂-CH₃ domains). For each IgG variant, transient transfections were performed by cotransfecting a light-chain expressing plasmid and a heavy-chain expressing plasmid into an adenovirus-transformed human embryonic kidney cell line, 293 (Graham et al., J. Gen. Virol., 36:59-74, (1977)). Briefly, 293 cells were split on the day prior to transfection, and plated in serum-containing medium. On the following day, double-stranded DNA prepared as a calcium phosphate precipitate was added, followed by pAdVAntage™ DNA (Promega, Madison, WI), and cells were incubated overnight at 37°C. Cells were cultured in serum-free medium and harvested after 4 days. Antibodies were purified from culture supernatants using protein A-Sepharose CL-4B, then buffer exchanged into 10 mM sodium succinate, 140 mM NaCl, pH 6.0, and concentrated using a Centricon-10 (Amicon). Protein concentrations were determined by quantitative amino acid analysis.

### Example 3

This example describes generation of human CD20 BAC transgenic (Tg) mice and experiments to study the effects of anti-CD20 antibody or BLyS antagonist alone in the hCD20+ mice.

Human CD20 transgenic mice were generated from human CD20 BAC DNA (Invitrogen, Carlsbad, CA). Mice were screened based on the FACS analysis of human CD20 expression. As can be seen from the FACS plots in Figure 14, mice hemizygous (Tg+/-) and homozygous (Tg+/+) for the transgene express human CD20 on their B220+ B cells. Figure 15 shows the expression of various cell surface markers (CD43, IgM, IgD) during B cell differentiation and maturation. In the Tg+ mice, hCD20 is expressed on pre-B and immature B cells and mostly on mature B cells. The Tg+ mice were screened for human CD20 expression in the B cells of the bone marrow, spleen, mesenteric LN and Peyer's patches; the results are shown in Figures 16-19. Gating the cells on B220 and CD43 allows delineation into the various populations of B cells. Tg+ mice were then treated with anti-CD20 mAb (1mg total = 50mg/kg, equivalent to 3.5mg for a 70kg man) to see the effects on the B cells as outlined in the schematic in Figure 20. FACS analyses were done on peripheral blood, spleen, lymph node, bone marrow, and Peyer's Patches. Serum levels of anti-CD20 mAb were monitored. In mice, B cell depletion occurs within 3-4 days of treatment with anti-CD20 antibody. Not to be bound by any theory, B cell death appears to be mediated by ADCC or apoptosis or both. Treatment of Tg+ mice with anti-hCD20 mAb (m2H7) alone results in depletion of B cells in peripheral blood, mature peripheral lymph node B cells, T2 and follicular B cells in the spleen (see Figures 21-24). However, it was observed that certain B cell subsets are resistant to killing by anti-CD20 antibody despite very high, likely saturating levels of antibody on the cell surface. These resistant B cells are the marginal zone B cells in the spleen (Fig. 23), and the germinal center B cells in both the Peyer's patches (Fig. 25) and spleen (Fig. 27). In one experiment (Fig. 27), mice were injected with a first dose of anti-CD20 mAb at 100ug on day 1, followed by a second, 100ug dose on day 3 (it is likely that a single dose at 50ug was sufficient to saturate the B cells); T2/follicular B cells were depleted but the germinal center B cells from the Peyer's patches were shown to be bound with anti-CD20 mAb but were resistant to killing.

The recovery of B cells following anti-CD20 antibody treatment was followed. Mice were administered antibody at day 1. Figure 26 shows that at day 6 post antibody treatment, B cells in the peripheral blood were not detectable. At week 6, upon clearance of the antibody, hCD20+ cells begin to be detected and by week 14, B cells appeared to have recovered to normal levels. Recovery stems from precursor B cells which do not express CD20 developing into CD20+ mature B cells.

Figure 27 shows FACS plots demonstrating resistance of splenic germinal center B cells to short-term (single injection) anti-CD20 mAb treatment. Mice were unimmunized or immunized with sheep red blood cells (SRBC) by intraperitoneal injection at day 1 to induce germinal centers in the spleen. The germinal centers appear by day 7. At day 8, one group of mice was treated with the m2H7 mAb to human CD20. The control set of mice was treated with mIgG2a isotype control antibody. Spleen cells from the mice were analyzed at day 12. PNA (peanut agglutinin) stains for germinal center. No detectable germinal center cells were seen in the spleens of mice not immunized with SRBC whereas the spleens of immunized mice show 0.3% PNA staining cells. While T2/Follicular B cells are depleted with anti-CD20 antibody treatment, marginal center B cells in the spleen are resistant to the antibody.

Next, it was determined whether upon B cell depletion, the mice were able to develop T independent immune response. Mice were treated with m2H7 or isotpype control antibody mIgG2a at day 0. At days 3-7, B cell depletion has occurred. At day 7, the mice were injected i.v. with *Streptococcus pneumoniae* IV to induce a response to the polysaccharide. A T cell independent response was mounted on day 11. The results shown in Figure 28 demonstrated that treatment with anti-CD20 (2H7 or Rituxan) did not affect the B cell response from the marginal zone and germinal centers of the spleen, i.e., the non-depleted MZ and B1 B cells confer protection to T-independent antigens. This data demonstrates that some aspects of humoral immunity-specifically T-independent B cell responses (in this case) are preserved despite treatment with anti-CD20 mAb.

### Example 4

This example demonstrates the synergy between anti-CD20 mAb and BLys antagonist treatments for B cell modulation/depletion.

BAFF/BLyS/TALL-1 (member of the TNF superfamily) plays an important role in the survival and maturation of immature T2, FO and MZ B cells and enhances competitive survival of autoreactive B cells (S. Mandala et al., Science 296, 346-9 (2002); F. Mackay, P. Schneider, P. Rennert, J. Browning, Annu Rev Immunol 21, 231-64 (2003); P. A. Moore et al., Science 285, 260-3 (1999)). Overexpression of a soluble form of BAFF/BLyS/TALL-1 in mice results in B cell hyperplasia, hypergammaglobulinemia and autoimmune lupus-like syndrome (S. A. Marsters et al., Curr Biol 10, 785-8 (2000)). Conversely, treatment of lupus-prone mice with a BAFFR/BR3-Fc fusion protein, which neutralizes BAFF/BLyS, results in improved autoimmune serologies, renal pathology and mortality (R. Lesley et al., Immunity 20, 441-53 (2004)).

### Materials and methods:

In the Experimental Examples, the BR3-Fc or BAFFR/BR3-Fc used is hBR3-Fc of SEQ ID. NO. 2. For the experiment shown in Fig. 29, FVB mice expressing a bacterial artificial chromosome encoding human CD20 (designated as hCD20⁺ mice) were treated with intraperitoneal injections of anti-CD20 mAb (single injection of 100 micrograms on Day 9), BR3-Fc (100 micrograms every other day from Days 1 through 12), or the combination of anti-CD20 mAb and BR3-Fc. Each group consisted of 4 mice. Two days following the last injection, the mice were sacrificed and analyzed for hCD20⁺ B cells. FACS analysis of spleen, blood, lymph node and Peyer's Patches were analyzed for B cell markers (CD21⁺CD23⁺).

For the experiment shown in Fig. 30, hCD20 Tg+ mice were treated with control IgG₂ₐ, BAFFR/BR3-Fc (100 µg/mouse IP daily for 12 days), anti-hCD20 mAb (100 µg/mouse IP on day 9) or the combination of BAFFR/BR3-Fc and anti-hCD20 mAb (same dosing as single treatment groups). B220⁺ splenocytes were isolated on day 13 and stained for CD21 and CD23. N=5 mice/group. Figure 30 shows the synergistic effects on B cell depletion of the combination of anti-hCD20 mAb and BR3-Fc in the human CD20 Tg+ mice. Figure 31 shows quantitation of depletion of B220+ total spleen B cells, marginal zone (MZ) and follicular (FO) B cells from hCD20 Tg+ mice. The mice were treated with single doses of 0.1 mg control IgG₂ₐ, BAFF/BR3-Fc or anti-hCD20 mAb. Splenocytes were analyzed on day 4. N=5 mice/group.

### Results:

These results are shown in Fig. 29, Fig. 30, and Fig. 31.
1. Anti-CD20 mAb therapy depletes >99% of mature circulating B cells in the blood and lymph nodes.
2. BR3-Fc decreases mature circulating B cells in the blood and lymph nodes.
3. Anti-CD20 mAb therapy depletes T2 and follicular B cells, but not marginal zone B cells in the spleen.
4. BR3-Fc decreases T2/follicular and marginal zone B cells in the spleen.
5. The combination of anti-CD20 mAb and BR3-Fc synergizes to deplete all populations of B cells in the spleen.

Treatment of hCD20⁺ mice with BAFFR/BR3-Fc for -2 weeks resulted in a marked-decrease in MZ and T2/FO B cells (Fig. 30, panel 3). Combined treatment of BAFFRBR3-Fc and anti-hCD20 mAb, surprisingly, resulted in the depletion of all splenic B cell subsets (Fig. 30, panel 4). To further explore the potential synergy of BAFF neutralization and anti-hCD20 mAb, the extent of B cell loss four days following treatment with single doses of anti-hCD20 mAb and BAFFR/BR3-Fc was quantified. While treatment with single doses of anti-hCD20 mAb or BAFFR/BR3-Fc resulted in ∼40-50% loss of MZ B cells and ∼33-70% loss of FO B cells, the combination anti-hCD20 mAb and BAFFR/BR3-Fc resulted in >90% loss of MZ and FO B cells (Fig. 31). Hence, survival factors also play an important role in determining susceptibility to anti-hCD20 mAb mediated B cell depletion.

### Example 5

### Production of BlyS antagonists

### BLyS₈₂₋₂₈₅ production.

A DNA fragment encoding human BAFF (residues 82-285) was cloned into the pET15b (Novagen) expression vector, creating a fusion with an N-terminal His-tag followed by a thrombin cleavage site. E. coli BL21(DE3) (Novagen) cultures were grown to mid-log phase at 37°C in LB medium with 50 mg/L carbenicillin and then cooled to 16 °C prior to induction with 1.0 mM IPTG. Cells were harvested by centrifugation after 12 h of further growth and stored at -80°C. The cell pellet was resuspended in 50 mM Tris, pH 8.0, and 500 mM NaCl and sonicated on ice. After centrifugation, the supernatant was loaded onto a Ni-NTA agarose column (Qiagen). The column was washed with 50 mM Tris, pH 8.0, 500 mM NaCl, and 20 mM imidazole and then eluted with a step gradient in the same buffer with 250 mM imidazole. BAFF-containing fractions were pooled, thrombin was added, and the sample was dialyzed overnight against 20 mM Tris, pH 8.0, and 5 mM CaCl₂ at 4°C. The protein was further purified on a monoQ (Pharmacia) column and finally on an S-200 size exclusion column in 20 mM Tris, 150 mM NaCl, and 5 mM MgCl₂. The resulting BLyS protein was used as described below.

### BR3 extracellular domain production

The extracellular domain of human BR3 (residues 1 to 61) was subcloned into the pET32a expression vector (Novagen), creating a fusion with an N-terminal thioredoxin (TRX)-His-tag followed by an enterokinase protease site. E. coli BL21(DE3) cells (Novagen) were grown at 30°C and protein expression induced with IPTG. TRX-BR3 was purified over a Ni-NTA column (Qiagen), eluted with an imidazole gradient, and cleaved with enterokinase (Novagen). BR3 was then purified over an S-Sepharose column, refolded overnight in PBS, pH 7.8, in the presence of 3 mM oxidized and 1 mM reduced glutathione, dialyzed against PBS, repurified over a MonoS column, concentrated, and dialyzed into PBS.

### Peptide synthesis

MiniBR3 was synthesized as a C-terminal amide on a Pioneer peptide synthesizer (PE Biosystems) using standard Fmoc chemistry. The side chain thiols of cysteines 19 and 32 were protected as trifluoroacetic acid (TFA)-stable acetamidomethyl (Acm) derivatives. Peptides were cleaved from the resin by treatment with 5% triisopropyl silane in TFA for 1.5-4 hr at room temperature. After removal of TFA by rotary evaporation, peptides were precipitated by addition of ethyl ether, then purified by reversed-phase HPLC (acetonitrile/H₂O/0.1% TFA). Peptide identity was confirmed by electrospray mass spectrometry. After lyophilization, the oxidized peptide was purified by HPLC. HPLC fractions containing reduced miniBR3 were adjusted to a pH of - 9 with NH₄OH; the disulfide between cysteines 24 and 35 was then formed by addition of a small excess of K₃Fe(CN)₆, and the oxidized peptide purified by HPLC. Acm groups were removed (with concomitant formation of the second disulfide) by treatment of the HPLC eluate with a small excess of I₂ over - 4 h. The progress of the oxidation was monitored by analytical HPLC, and the final product was again purified by HPLC. MiniBR3 was amino-terminally biotinylated on the resin by reaction with a 10-fold molar excess of sulfo-NHS-biotin (Pierce Chemical, Co.). The biotinylated miniBR3 was then cleaved from the resin and purified as described above for the unbiotinylated miniBR3.

The following peptides ECFDLLVRHWVACGLLR (BLyS0027) (SEQ ID NO:9), ECFDLLVRHWVPCGLLR (BLyS0048) (SEQ ID NO:6) and ECFDLLVRAWVPCSVLK (BLyS0051) (SEQ ID NO:5) were synthesized generally as follows. Peptides were synthesized on a Rainin Symphony peptide synthesizer system using Rink amide resin and a threefold excess of 9-fluorenylmethoxycarbonyl (Fmoc) protected amino acid activated with 2-(1H-Benzotriazone-1-yl)-1,1,3,3-tetramethyluronium hexafluorophosphate (HBTU) in the presence of a fivefold excess of diisopropylethylamine (DIPEA). Amino acids were coupled twice at each position before deprotecting with a 20% solution of piperidine in dimethylformamide (DMF) and moving to the next residue. Washes between coupling steps were performed using dimethylacetamide (DMA). Following coupling of the final amino acid onto the peptide and its deprotection with 20% piperidine in DMF, the peptides were acylated at their amino terminus using 3 equivalents of acetic anhydride and 5 equivalents of DIPEA in DMA. Alternatively, the amino terminus was modified through acylation with 5-carboxyfluorescein, with (+)-biotin, or through reaction with another fluorophore or reporter molecule. The peptide was then cleaved from the resin through treatment with a solution of 95% trifluoroacetic acid (TFA) containing 2.5% water and 2.5% triisopropylsilane for 90 minutes. The volatiles were removed under reduced pressure, diethyl ether was added and the solids filtered off. The resulting precipitate was washed again with diethyl ether and the combined organics discarded. The washed solids were then washed successively with acetic acid, a 1:1 mixture of acetic acid and acetonitrile, a 1:1:1 mixture of acetic acid, acetonitrile and water, an 1:1:8 mixture of acetic acid, acetonitrile and water and finally with water. The combined washes were lyophilized and the resulting crude peptides purified using C18 reverse phase high performance liquid chromatography using a 30 minute 10% to 70% gradient of acetonitrile in water with 0.1% trifluoroacetic acid in each solvent at a flow rate of 15 milliliters per minute. Fractions containing the desired peptide were oxidized through addition of a saturated solution of iodine in acetic acid until the solution remained colored. This solution was then lyophilized. Finally, the lyophilized crude oxidized peptide was purified a second time under identical conditions and the fractions containing the desired peptide lyophilized. Some of the peptides were synthesized under identical conditions except that the synthesis was performed on a PerSeptive Pioneer automated synthesizer using a fourfold excess of amino acid, coupling only once per residue.

### Example 6

### Phage Display of 17mers

*Library construction.* A phagemid encoding the STII secretion signal sequence ("STII ss"), a linker (GGGSGGG, SEQ ID NO:__), and a sequence encoding the C-terminal residues of minor protein III of M13 phage (e.g., residues 267-421) (hereinafter, "cP3") was used as a template for library , construction. Two libraries were constructed using Kunkel mutagenesis techniques and oligonucleotides that introduced a fragment corresponding to residues 23-39 of human BR3 with a C32W mutation also known as "17-mer C32W", and additionally encoded mutations within the 17-mer C32W region. Specifically, library 1 encoded replacement codons at residues numbered 31, 34 and 36-39 (replacement codon: NNS = any codon), and library 2 encoded replacement codons residues 27, 30, 31, 34 and 36-39 (replacement codon: VNC = encodes amino acids L, P, H, R, I, T, N, S, V, A, D and G). In the replacement codons: N is 25% A, 25% C, 25% G, 25% T ; S is 50% G/50% C; V is 33% G/33% A/33% C; and C is 100% C. Library 1 encoded 1.1 × 10⁹ members and Library 2 encoded 4.3 x 10⁸ members.

*Library Sorting.* The phage were subject to four rounds of selection. In general, the phage input per round was 10¹⁴ phage for the 1^{st} round (solid phase sorting) and 3 x 10¹² phage for additional rounds (solution phase sorting).

*Phage Selection.* The first round of selection was a solid phase sorting method. Maxisorp immunoplates (96-well) were coated with BLyS₈₂₋₂₈₅ prepared as described above (100µl at 2µg/ml in 50mM carbonate buffer (pH 9.6)) overnight at 4°C. The wells were then blocked for one hour with 0.2% (w/v) BSA in phosphate-buffered saline (PBS) and washed 3-5 times with PBS, 0.05% Tween20. Phage particles ((100µl/well in ELISA buffer (PBS/0.5%BSA /0.05% Tween20)) were added to the wells. After two hours, the wells were washed several times with PBS, 0.05% Tween20. The phage bound to the wells were eluted with 0.1N HCl for 10 min at RT. The eluted phage were neutralized by adding 1/20 volume 2M Tris pH 11.0.

To titer the phage, log phase XL-1 (OD 600nm∼0.3) was infected with eluted phage at 37°C for 30 minutes. Next, the infected cells were serially diluted in 10 fold increments in 2YT. 10µl aliquots of the infected cells were plated per carbenicillin plate. ∼10⁸ phage from each library were obtained from the first round of selection.

To propagate the phage, eluted phage was used to infect log phase XL-1 (OD 600nm∼0.3) at 37°C for 30 minutes. Helper phage, KO7, and carbenicillin were added to the infection at a final concentration of 1 x 10¹⁰ pfu/ml KO7 and 50ug/ml carbenicillin at 37°C for another 30 minutes. The culture was grown in 2YT media with carbenicillin 50ug/ml and 25ug/ml kanamycin to final volumes of 25ml at 37°C overnight.

The phage were purified by spinning down the cells at 10000 rpm for 10 minutes. The supernatant was collected. 20% PEG/2.5M NaCl was added at 1/5 of the supernatant volume, mixed and allowed to sit at room temperature for 5 minutes. The phage were spun down into a pellet at 10000 rpm for 10 minutes. The supernatant was discarded and the phage pellet spun again for 5 minutes at 5000 rpm. The pellets were resuspended in 0.7ml PBS and spun down at 13000 rpm for 10 minutes to clear debris. The OD of the resupended phage pellet was read at 268nm.

The second to fourth rounds of selection utilized solution sorting methods. For the second round, Maxisorp Nunc 96-well plates were coated with 5ug/ml neutravidin (Pierce) at 4°C overnight. Next, the plate was blocked with 200 µl/ml Superblock (Pierce) in PBS for 30 min at room temperature. Tween20 was added to each well for a final concentration of 0.2% (v/w) and blocked for another 30 minutes at room temperature. The amplified, purified phage from the first round of selection were incubated with 50nM biotinylated BLyS (final concentration) in 150ul buffer containing Superblock 0.5% and 0.1% Tween20 for 1 h at room temperature. The mixtures were then diluted 5-10X with PBS/0.05% Tween and applied at 100µl/well to the neutravidin coated plate. The plate was gently shaken for five minutes at room temperature to allow phage bound to biotinylated BLyS to be captured in the wells. The wells were then washed with PBS/0.05% Tween20 several times. Bound phage were eluted with 0.1N HCl for 10 min, neutralized, tittered, propagated and purified as described above. -3 x 10⁶ phage from each library were obtained from the second round of selection.

The third round of selection was similar to the second round, except a concentration of 2nM biotinylated BLyS was incubated with the phage prior to dilution and addition to each well. Bound phage were eluted with 0.1N HCl for 10 min, neutralized, titered and propagated as described above. ∼10⁴ phage from each library were obtained from the third round of selection.

Phage from the third round of selection were next subjected to two different selection methods in the fourth round. Method 4a was similar to the second and third rounds of selection except that the phage was incubated in the presence of 0.5nM biotinylated BLyS for lh at room temperature. The mixture was then incubated for an addtional 15 minutes at room temperature in the presence of 1000 fold excess (500nM) of unbiotinylated BLyS prior to dilution and addition to the coated wells Method 4b was also similar to the second and third rounds of selection except that 0.2nM BLyS was incubated with the phage before dilution and addition to each well. Bound phage from each round four selection were eluted with 0.1N HCl for 10 min, neutralized, titered and propagated as described above. ∼10³ phage were obtained for each library from each of the fourth rounds (4a and 4b) of selection.

*Clone Analysis.* After the fourth round of selection, individual clones were grown in a 96-well format in 400 µL of 2YT medium supplemented with carbenicillin and KO7 helper phage. Supernatants from these cultures were used in phage ELISAs. For phage ELISAs, Nunc Maxisorp 96-well plates were coated overnight at 4 °C with 100 µl of a 2 µg/ml solution of BLyS in carbonate buffer, pH 9.6. The plate was washed with PBS and blocked with 0.5% BSA in PBS for two hours. Phage supernatant was diluted 1:4 in ELISA binding buffer (PBS, 0.5%BSA, 0.05% Tween20) in the absence or presence of 50nM BLyS and incubated for 1h at RT. 100 ul of the diluted phage supernatants were then transferred to the coated plates and allowed to shake gently to capture phage for 20 minutes. The plates were then washed with PBS/0.05% Tween20 several times. 100 µl per well of HRP-conjugated anti-M13 antibody in PBS/0.05% Tween20 (1:5000) was then transferred to the plates and incubated for 20 min. After washing with PBS/0.05%Tween followed by PBS, the plate was incubated 5 min with 100 µl PBS substrate solution containing 0.8 mg/ml OPD (Sigma) and 0.01% H₂O₂. The reaction was quenched with 100 µl/well 1M H₃PO₄ and the plate read at 490 nm. The clones tested were then sequenced as previously described (Weiss, G. A., Watanabe, C. K., Zhong, A., Goddard, A., and Sidhu, S. S. (2000) Proc. Natl. Acad. Sci. U.S.A. 97, 8950-8954). Sequences of acceptable quality were translated and aligned. The amino acid sequences of the 17mers are shown in FIG.32.

Fourteen clones were further analyzed in a BLyS binding assay to determine their IC50 value. Clones 2 and 7 had a high number of siblings (clones with an identical sequence) in the fourth round. According to the phage ELISA assay, clones 13, 19, 22, 26, 32, 39 and 44 were greatly inhibited from binding to the plate by 50nM BLyS (FIG.11). The binding of clones 35, 45, 68, 82 and 90 was also greatly inhibited in the phage ELISA assay (FIG.11). Phage supernatants from these 14 clones were used to infect log phase XL-1 which were propagated and purified as described above.

To normalize for display and phage yield and determine the appropriate dilution of phage for IC50 measurement, serial dilutions of purified phage from each clone were incubated in ELISA binding buffer (PBS, 0.5%BSA, 0.05% Tween20) for 1 h at room temperature. 100 µl of each dilution were transferred to BLyS coated plates and allowed to shake gently to capture phage for 20 minutes as described above. Bound phage was detected by HRP-conjugated anti-M13 antibody, followed by OPD/H₂O₂ substrate reaction, quenched and read at 490nm as described above. By this process, the dilution of each clone that yielded -1 O.D. at 490nm was determined and used in the IC50 assay.

To determine the IC50 value of each of the 14 clones, Nunc Maxisorp 96-well plates were coated overnight at 4 °C with 100 µl of a 2 µg/ml solution of BLyS in carbonate buffer, pH 9.6, and washed and blocked as described above. A dilution of amplified, purified phage for each of the 14 clones was incubated in the presence of a concentration series of BLyS ranging from 0.003-1000 nM in 130ul ELISA binding buffer (PBS, 0.5%BSA, 0.05% Tween20) for 1 h at room temperature. 100 µl of each of these concentration series were transferred to BLyS coated plates and captured, washed, detected with HRP-conjugated anti-M13 antibody and processed as described above. IC50 values were determined by a four-parameter fit of the ELISA signal for each of the 14 clones. The IC50 values ranged from 0.4 (clone 44) to 11 nM (clone 22).

*Competitive Displacement ELISA.* The following 17-mers, Ac-ECFDLLVRHWVACGLLR-NH₂ (SEQ ID NO:_) ("BLyS0027"), Ac-ECFDLLVRHWVPCGLLR-NH₂ (SEQ ID NO:_) ("BLyS0048"), Ac-ECFDLLVRAWVPCSVLK-NH₂ (SEQ ID NO:_) ("BLyS0051") were synthesized as described above. Nunc Maxisorp 96-well plates were coated overnight at 4°C with 100 µl of a 2 µg/ml solution of BLyS in carbonate buffer, pH 9.6. The plate was washed with PBS and blocked with 1% skim milk in PBS. Serial dilutions of the BR3 ECD (residues 1-61) and the above 17-mer peptides were prepared in PBS10.05% Tween 20 containing 3 ng/ml biotinylated miniBR3. After washing with PBS/0.05% Tween, 100 µl/well of each dilution was transferred and incubated for 1 hour at room temperature. The plate was washed with PBS/0.05%Tween and incubated 15 min with 100 µl/well of 0.1 U/ml Streptavidin-POD (Boehringer Mannheim) in PBS/0.05%Tween. After washing with PBS/0.05%Tween followed by PBS, the plate was incubated 5 min with 100 µl PBS substrate solution containing 0.8 mg/ml OPD (Sigma) and 0.01% H₂O₂. The reaction was quenched with 100 µl/well 1M H₃PO₄ and the plate read at 490 nm. IC₅₀ values were determined by a four-parameter fit of the competitive displacement ELISA signal. The concentrations of initial stock solutions of miniBR3 and BR3 extracellular domain were determined by quantitative amino acid analysis.

The IC50 values were determined for BR3 ECD, BLyS0027, BLyS0048 and BLyS0051 using this assay. The 17-mer peptides all had greater affinity for BLyS than the 62-mer BR3 ECD.

### Example 4

### Peptide-PEG conjugates

### BLyS₈₂₋₂₈₅ production and Peptide synthesis. As described in Example 5 above.

*Conjugation of Polymers to Peptides*. PEGylated 17-mer peptides were generated by using linear PEGs modified with N-hydroxysuccinimide chemistry (NHS) to react with primary amines (lysines and N-terminus). All PEG-NHS (PEG-SPA) reagents were purchased from Nektar Therapeutics, San Carlos, CA and stored under nitrogen at-70°C. The peptide was dissolved at 1 mg/mL in phosphate-buffer saline (PBS). To 0.4 mL aliquots of the peptide solution was added solid 2KPEG-SPA, 5KPEG-SPA, or 20KPEG-SPA. Enough solid was added to obtain a 3:1 molar ratio of PEG-SPA to peptide. These solutions were incubated at room temperature for 1 hour and then the progress of the reaction was analyzed by reverse phase analytical HPLC on a 50 µL portion of the solution. The PEG addition and incubation was repeated 2 times until all of the peptide had been modified. The PEGylated peptides were tested for BlyS binding without further purification. The ratio of PEG:peptide in the purified conjugated product is approximately 1:1.

*Competitive Displacement ELISA*. A 17-mer, Ac-ECFDLLVRHWVPCGLLR-NH2 (SEQ ID NO:_) ("blys0048") was synthesized as described above. ECFDLLVRHWVPCGLL K (blys0095) (SEQ ID NO:_) was synthesized and coupled to each of 2K, 5K and 20K PEG-NHS as described above. Nunc Maxisorp 96-well plates were coated overnight at 4°C with 100 µl of a 2 µg/ml solution of BLyS in carbonate buffer, pH 9.6. The plate was washed with PBS and blocked with 1% skim milk in PBS. Serial dilutions of mini-BR3 (SEQ. ID.) and the above 17-mer peptide and PEG-peptide conjugate were prepared in PBS/0.05% Tween 20 containing 3 ng/ml biotinylated miniBR3. After washing with PBS/0.05% Tween, 100 µl/well of each dilution was transferred and incubated for 1 hour at room temperature. The plate was washed with PBS/0.05%Tween and incubated 15 min with 100 µl/well of 0.1 U/ml Streptavidin-POD (Boehringer Mannheim) in PBS/0.05%Tween. After washing with PBS/0.05%Tween followed by PBS, the plate was incubated 5 min with 100 µl PBS substrate solution containing 0.8 mg/ml OPD (Sigma) and 0.01% H₂O₂. The reaction was quenched with 100 µl/well 1M H₃PO₄ and the plate read at 490 nm. IC₅₀ values were determined by a four-parameter fit of the competitive displacement ELISA signal. The equation is: y = ml + (m2-m1)/(1+m0/m4)^m3, where ml is the asorbance at infinite competitor concentration, m2 is the absorbance for no added competitor, m3 is the slope of the curve near the midpoint, m4 is the IC50 value and m0 is the concentration of competitor, peptide in this case. The concentration of biotinylated miniBR3 was about 10 pM. The concentration of initial stock solution of miniBR3 was determined by quantitative amino acid analysis.

### Results

The four-parameter fit of the competitive displacement ELISA signals provided IC50 values for: blys0095 of 19nM, blys0048 of 14nM and blys0095-2kPEG conjugate of 43nM, and blys0095-5kPEG conjugate of 51nM using this assay. Similarly, the fit of the competitive displacement ELISA signals for a separate experiment provided IC50 values for blys0095-20kPEG conjugate of 99nM and blys0048 of 15nM.

The 17-mer peptide-PEG conjugates (2k, 5k and 20k) demonstrated binding ability for BLyS. The conjugation of PEG to blys0095 did not significantly reduce its binding affinity as compared to similar unconjugated peptides.

### Conclusion

The experiments herein demonstrated surprising results in that the combination of anti-CD20 mAb and BR3-Fc resulted in great synergy in depletion of all subsets of B cells.

## Claims

1. A CD20 binding antibody and a BLyS antagonist as a combination for use in a method of alleviating a B-cell regulated autoimmune disorder, the method comprising administering to a patient suffering from the disorder a therapeutically effective amount of the CD20 binding antibody and of the BLyS antagonist.

2. The CD20 binding antibody and BLyS antagonist as a combination for use according to claim 1, wherein the BLyS antagonist is an antibody, wherein said antibody is an anti-BLyS antibody or an anti-BR3 antibody; a BR3 immunoadhesin, such as a BR3 immunoadhesin comprising the extracellular domain of BR3; or a polypeptide having the sequence of SEQ ID NO:5, SEQ ID NO:6, SEQ ID NO:7, SEQ ID NO:8, SEQ ID NO:9, or SEQ ID NO:10.

3. The CD20 binding antibody and BLyS antagonist for use according to claim 2, wherein the BR3 immunoadhesin is BR3-Fc of SEQ ID NO:2.

4. The CD20 binding antibody and BLyS antagonist for use according to any one of the preceding claims, wherein the BLyS antagonist partially or fully blocks BR3 interaction with a BLyS polypeptide.

5. The CD20 binding antibody and BLyS antagonist for use according to any one of the preceding claims, wherein the CD20 binding antibody is a humanised antibody.

6. The CD20 binding antibody and BLyS antagonist for use according to claim 5, wherein the CD20 binding antibody is a chimeric antibodycomprising the variable regions from a murine antibody fused to the constant regions of a human antibody.

7. The CD20 binding antibody and BLyS antagonist for use according to claim 6, wherein the chimeric antibody is rituximab or hu2H7v.16 having the light and heavy chain sequences of SEQ ID NO.15 and SEQ ID NO.16, respectively.

8. The CD20 binding antibody and BLyS antagonist for use according to claim 1, wherein in the method the CD20 binding antibody and BLyS antagonist are administered concurrently.

9. The CD20 binding antibody and BLyS antagonist for use according to claim 1, wherein in the method the CD20 binding antibody and BLyS antagonist are administered sequentially.

10. The CD20 binding antibody and BLyS antagonist for use according to claim 1, wherein in the method the BLyS antagonist is administered before the CD20 binding antibody.

11. The CD20 binding antibody and BLyS antagonist for use according to claim 1, wherein the autoimmune disorder is selected from the group consisting of systemic lupus erythematosus (SLE), lupus, rheumatoid arthritis, juvenile rheumatoid arthritis, Wegener's disease, inflammatory bowel disease, idiopathic thrombocytopenic purpura (ITP), thrombotic throbocytopenic purpura (TTP), autoimmune thrombocytopenia, multiple sclerosis, psoriasis, IgA nephropathy, IgM polyneuropathies, myasthenia gravis, vasculitis, diabetes mellitus, Reynaud's syndrome, Sjorgen's syndrome and glomerulonephritis.

12. The CD20 binding antibody and BLyS-antagonist for use according to claim 3, wherein in the method the BR3-Fc is administered at a dosage of about 2-5 mg/kg and rituximab is administered at a dosage of about 2.5-10 mg/kg.

13. The CD20 binding antibody and BLyS antagonist for use according to claim 1, wherein administration of the BLyS antagonist and the CD20 binding antibody produces a synergistic effect to deplete the B cells.

14. The CD20 binding antibody and BLyS antagonist for use according to claim 11, wherein in the method the BLyS antagonist and the CD20 binding antibody are administered in conjunction with therapy using a drug selected from nonsteroidal anti-inflammatory drugs (NSAIDs), glucocorticoid, prednisone, and disease-modifying antirheumatic drug (DMARD).

15. The CD20 binding antibody and BLyS antagonist for use according to claim 1, wherein marginal zone or germinal center B cells in the patient are depleted.

## Patentansprüche

1. CD20 bindender Antikörper und BLyS-Antagonist als Kombination zur Verwendung in einem Verfahren zur Linderung einer B-Zell-regulierten Autoimmunstörung, wobei das Verfahren die Verabreichung einer therapeutisch wirksamen Menge des CD20 bindenden Antikörpers und des BLyS-Antagonisten an einen Patienten umfasst, der an der Störung leidet.

2. CD20 bindender Antikörper und BLyS-Antagonist als Kombination zur Verwendung nach Anspruch 1, wobei der BLyS-Antagonist ein Antikörper ist, wobei der Antikörper ein Anti-BLyS-Antikörper oder ein Anti-BR3-Antikörper; ein BR3-lmmunadhäsin, wie z.B. ein BR3-lmmunadhäsin, das eine extrazelluläre Domäne von BR3 umfasst; oder ein Polypeptid mit der Sequenz der Seq.-ID Nr. 5, Seq.-ID Nr. 6, Seq.-ID Nr. 7, Seq.-ID Nr. 8, Seq.-ID Nr. 9 oder Seq.-ID Nr. 10 ist.

3. CD20 bindender Antikörper und BLyS-Antagonist zur Verwendung nach Anspruch 2, wobei das BR3-Immunadhäsin BR3-Fc der Seq.-ID Nr. 2 ist.

4. CD20 bindender Antikörper und BLyS-Antagonist zur Verwendung nach einem der vorangegangenen Ansprüche, wobei der BLyS-Antagonist BR3-Wechselwirkung mit einem BLyS-Polypeptid teilweise oder vollständig blockiert.

5. CD20 bindender Antikörper und BLyS-Antagonist zur Verwendung nach einem der vorangegangenen Ansprüche, wobei der CD20 bindende Antikörper ein humanisierter Antikörper ist.

6. CD20 bindender Antikörper und BLyS-Antagonist zur Verwendung nach Anspruch 5, wobei der CD20 bindende Antikörper ein chimärer Antikörper ist, der die variablen Regionen von einem Mausantikörper an die konstanten Regionen eines menschlichen Antikörpers fusioniert umfasst.

7. CD20 bindender Antikörper und BLyS-Antagonist zur Verwendung nach Anspruch 6, wobei der chimäre Antikörper Rituximab oder hu2H7v.16 mit der Leicht- und Schwerkettensequenz der Seq.-ID Nr. 15 bzw. Seq.-ID Nr. 16 ist.

8. CD20 bindender Antikörper und BLyS-Antagonist zur Verwendung nach Anspruch 1, wobei bei dem Verfahren der CD20 bindende Antikörper und der BLyS-Antagonist gleichzeitig verabreicht werden.

9. CD20 bindender Antikörper und BLyS-Antagonist zur Verwendung nach Anspruch 1, wobei bei dem Verfahren der CD20 bindende Antikörper und der BLyS-Antagonist nacheinander verabreicht werden.

10. CD20 bindender Antikörper und BLyS-Antagonist zur Verwendung nach Anspruch 1, wobei bei dem Verfahren der BLyS-Antagonist vor dem CD20 bindenden Antikörper verabreicht wird.

11. CD20 bindender Antikörper und BLyS-Antagonist zur Verwendung nach Anspruch 1, wobei die Autoimmunstörung aus der aus systemischem Lupus erythematodes (SLE), Lupus, rheumatoider Arthritis, juveniler rheumatoider Arthritis, Wegener-Krankheit, entzündlicher Darmerkrankung, idiopathischer thrombozytopenischer Purpura (ITP), thrombozytischer thrombozytopenischer Purpura (TTP), Autoimmunthrombozytopenie, multipler Sklerose, Psoriasis, IgA-Nephropathie, IgM-Polyneuropathien, Myasthenia gravis, Vaskulitis, Diabetes mellitus, Reynaud-Syndrom, Sjögren-Syndrom und Glomerulonephritis bestehenden Gruppe ausgewählt ist.

12. CD20 bindender Antikörper und BLyS-Antagonist zur Verwendung nach Anspruch 3, wobei bei dem Verfahren das BF3-Fc in einer Dosis von etwa 2-5 mg/kg verabreicht wird und Rituximab in einer Dosis von etwa 2,5-10 mg/kg verabreicht wird.

13. CD20 bindender Antikörper und BLyS-Antagonist zur Verwendung nach Anspruch 1, wobei die Verabreichung des BLyS-Antagonisten und des CD20 bindenden Antikörpers eine synergistische Wirkung zur Abreicherung der B-Zellen erzeugt.

14. CD20 bindender Antikörper und BLyS-Antagonist zur Verwendung nach Anspruch 11, wobei bei dem Verfahren der BLyS-Antagonist und der CD20 bindende Antikörper gemeinsam mit einer Therapie unter Verwendung eines Arzneimittels, das aus nichtsteroidalen Antiphlogistika (NSAIDs), Glucocorticoid, Prednison und langfristig wirksamen Antirheumatika (DMARD) ausgewählt ist, verabreicht werden.

15. CD20 bindender Antikörper und BLyS-Antagonist zur Verwendung nach Anspruch 1, wobei die Randzonen- oder Keimzentrum-B-Zellen im Patienten abgereichert werden.

## Revendications

1. Anticorps de liaison à CD20 et antagoniste de BLyS sous forme d'une association pour une utilisation dans un procédé de soulagement d'un trouble autoimmun régulé par les lymphocytes B, le procédé comprenant l'administration à un patient souffrant de ce trouble d'une quantité thérapeutiquement efficace de l'anticorps de liaison à CD20 et de l'antagoniste de BLyS.

2. Anticorps de liaison à CD20 et antagoniste de BLyS sous forme d'une association pour une utilisation suivant la revendication 1, l'antagoniste de BLyS étant un anticorps, ledit anticorps étant un anticorps anti-BLyS ou un anticorps anti-BR3 ; une immunoadhésine de BR3, telle qu'une immunoadhésine de BR3 comprenant le domaine extracellulaire de BR3 ; ou un polypeptide ayant la séquence de la SEQ ID N° 5, de la SEQ ID N° 6, de la SEQ ID N° 7, de la SEQ ID N° 8, de la SEQ ID N° 9 ou de la SEQ ID N° 10.

3. Anticorps de liaison à CD20 et antagoniste de BLyS pour une utilisation suivant la revendication 2, l'immunoadhésine de BR3 étant BR3-Fc de la SEQ ID N° 2.

4. Anticorps de liaison à CD20 et antagoniste de BLyS pour une utilisation suivant l'une quelconque des revendications précédentes, l'antagoniste de BLyS bloquant partiellement ou totalement l'interaction de BR3 avec un polypeptide BlyS.

5. Anticorps de liaison à CD20 et antagoniste de BLyS pour une utilisation suivant l'une quelconque des revendications précédentes, l'anticorps de liaison à CD20 étant un anticorps humanisé.

6. Anticorps de liaison à CD20 et antagoniste de BLyS pour une utilisation suivant la revendication 5, l'anticorps de liaison à CD20 étant un anticorps chimère comprenant les régions variables d'un anticorps murin fusionnées aux régions constantes d'un anticorps humain.

7. Anticorps de liaison à CD20 et antagoniste de BLyS pour une utilisation suivant la revendication 6, l'anticorps chimère étant le rituximab ou le hu2H7v.16 comprenant les séquences de chaîne légère et chaîne lourde respectivement de la SEQ ID N° 15 et de la SEQ ID N° 16.

8. Anticorps de liaison à CD20 et antagoniste de BLyS pour une utilisation suivant la revendication 1, l'anticorps de liaison à CD20 et l'antagoniste de BLyS étant administrés conjointement dans le procédé.

9. Anticorps de liaison à CD20 et antagoniste de BLyS pour une utilisation suivant la revendication 1, l'anticorps de liaison à CD20 et l'antagoniste de BLyS étant administrés séquentiellement dans le procédé.

10. Anticorps de liaison à CD20 et antagoniste de BLyS pour une utilisation suivant la revendication 1, l'antagoniste de BLyS étant administré avant l'anticorps de liaison à CD20 dans le procédé.

11. Anticorps de liaison à CD20 et antagoniste de BLyS pour une utilisation suivant la revendication 1, le trouble autoimmun étant choisi dans le groupe consistant en le lupus érythémateux disséminé (SLE), le lupus, la polyarthrite rhumatoïde, la polyarthrite rhumatoïde juvénile, la maladie de Wegener, une maladie intestinale inflammatoire, le purpura thrombocytopénique idiopathique (ITP), le purpura thrombocytopénique thrombotique (TTP), la thrombocytopénie autoimmune, la sclérose en plaques, le psoriasis, la néphropathie à IgA, les polyneuropathies à IgM, la myasthénie grave, la vasculite, le diabète sucré, le syndrome de Raynaud, le syndrome de Sjögren et la glomérulonéphrite.

12. Anticorps de liaison à CD20 et antagoniste de BLyS pour une utilisation suivant la revendication 3, le BR3-Fc étant administré dans le procédé à une dose d'environ 2 à 5 mg/kg et le rituximab étant administré dans le procédé à une dose d'environ 2,5 à 10 mg/kg.

13. Anticorps de liaison à CD20 et antagoniste de BLyS pour une utilisation suivant la revendication 1, l'administration de l'antagoniste de BLyS et l'anticorps de liaison à CD20 produisant un effet synergique pour la déplétion des lymphocytes B.

14. Anticorps de liaison à CD20 et antagoniste de BLyS pour une utilisation suivant la revendication 11, l'antagoniste de BLyS et l'anticorps de liaison à CD20 étant administrés dans le procédé conjointement avec une thérapie utilisant un médicament choisi entre des médicaments anti-inflammatoires non stéréoïdiens (MAINS), un glucocorticoïde, la prednisone, et un médicament anti-rhumatismal modificateur de maladie (DMARD).

15. Anticorps de liaison à CD20 et antagoniste de BLyS pour une utilisation suivant la revendication 1, provoquant chez le patient une déplétion de lymphocytes B de la zone marginale ou du centre germinal.
